# EUROPEAN PATENT APPLICATION

(11) **EP 2 096 121 A1**
(43) Date of publication of application: **02.09.2009**
(21) Application number: 09290150.3
(22) Date of filing: 02.03.2009
(51) Int. Cl.: C07K 16/08, C07K 16/10

(54) **Antiviral peptides comprising lipid attachment signals and methods of use**

(30) Priority: 29.02.2008 EP 08290205; 23.04.2008 EP 08290396
(71) Applicant: Institut Pasteur Of Shanghai, Shanghai 200025 (CN)
(72) Inventor: Zhou, Paul, San Antonio, TX 78249 (US); Wang, Huiqiang, Shanghai 200025 (CN); Li, Ping, Shanghai 200025 (CN)
(74) Representative: Thomas, Dean

(57) **Abstract**

An anti-viral lipodated peptide comprising an anti-viral antibody or an active fragment thereof and a lipid attachment signal, wherein the anti-viral peptide is configured to present the antibody upon the exterior of a cellular membrane and thereby confer immunity to a cell against the virus against which the antibody was raised.

SEQUENCE LISTING

<110>  Institut Pasteur of Shanghai

<120>  ANTIVIRAL PEPTIDES COMPRISING LIPID ATTACHMENT SIGNALS AND
       METHODS OF USE

<130>  BLO/DDT.F226-160

<160>  21

<170>  PatentIn version 3.3

<210>  1
<211>  11
<212>  PRT
<213>  Homo sapiens

<400>  1

Asp Ser Phe Gly Gly His His Trp Ser Trp Ile
1               5                   10

<210>  2
<211>  106
<212>  PRT
<213>  Homo sapiens

<400>  2

Gly Gly Ala Ala Gly Thr Gly Gly Ala Ala Cys Cys Ala Cys Thr Thr
1               5                   10                  15

Cys Ala Gly Gly Thr Ala Cys Thr Ala Cys Cys Cys Gly Thr Cys Thr
            20                  25                  30

Thr Cys Thr Ala Thr Cys Thr Gly Gly Gly Cys Ala Cys Ala Cys Gly
        35                  40                  45

Thr Gly Thr Thr Thr Cys Ala Cys Gly Thr Thr Gly Ala Cys Ala Gly
    50                  55                  60

Gly Thr Thr Thr Gly Cys Thr Thr Gly Gly Gly Ala Cys Gly Cys Thr
65                  70                  75                  80

Ala Gly Thr Ala Ala Cys Cys Ala Thr Gly Gly Gly Cys Thr Thr Gly
                85                  90                  95

Cys Thr Gly Ala Cys Thr Thr Ala Gly Thr
            100                 105

<210>  3
<211>  124
<212>  DNA
<213>  Artificial

<220>
<223>  scFv/hinge/his-tag/DAF nucleotide

<400>  3

accatgaaac atctgtggtt cttcctcctc ctagtggcag ctcccagatg ggtcctgtcc 60

caggtgcagc tgcagcaatg gggcgcagga ctcttgaagc cttcggagac cctgtccctc 120

acct 124

<210> 4
<211> 123
<212> DNA
<213> Artificial

<220>
<223> 6his tag/C'DAF/stop nucleotide

<400> 4
ctggagatca aacgaacccc gctgggtgac accacccaca cctccggaca ccatcaccat 60

caccatcgaa gtggaaccac ttcaggtact acccgtcttc tatctgggca cacgtgtttc 120

acg 123

<210> 5
<211> 946
<212> DNA
<213> Artificial

<220>
<223> scFv/hinge/his-tag/DAF complete sequence

<400> 5
atgaaacatc tgtggttctt cctcctccta gtggcagctc ccagatgggt cctgtcccag 60

gtgcagctgc agcaatgggg cgcaggactc ttgaagcctt cggagaccct gtccctcacc 120

tgcgctgttt atggtgactc cttcggtggt caccactgga gctggatccg ccagccccct 180

gggaagggac tagagtggat cggcgaaata aattttcttg gcaacaccaa ttacaatccg 240

tccctcaaga gtcgagtcgc catatcaaag gacacgtcca aaaatcaatt ctccctgacc 300

ttgaattctg cgaccgccgc ggacacggca gtctacttct gtgcgcgacg ggaaactatg 360

gctagtcgaa agagggcttt tgacatctgg ggccagggga caatggtcac tgtctctgca 420

ggtggcggtg gctctggagg tggtgggagc ggtggcggcg gatctgccga gctcatgacc 480

cagtctcctc ccaccctgtc tgcatctgta ggagacagag tcaccatcac ctgccgggcc 540

agtcagagtc tcagtggctg ggtggcctgg tatcaacaaa aaccagggaa agcccctaaa 600

ctcctgatct ataaggcgtc taccttagaa agtggggtcc catcaaggtt cagcggcagt 660

ggatctggga cagtgttcgc actcaccatt agcagcctgc agcccgatga ctctgcgact 720

tactactgcc aacaacatag tgctttttcg tccacttttg gccaggggac caaactggag 780

atcaaacgaa ccccgctggg tgacaccacc cacacctccg gacaccatca ccatcaccat 840

ggaagtggaa ccacttcagg tactacccgt cttctatctg ggcacacgtg tttcacgttg 900

acaggtttgc ttgggacgct agtaaccatg ggcttgctga cttagt 946

<210> 6
<211> 7582
<212> DNA

<213>  Artificial

<220>
<223>  pRRL- scFv/hinge/his-tag/DAF vector

<400>  6
caggtggcac ttttcgggga aatgtgcgcg gaacccctat ttgtttattt ttctaaatac      60

attcaaatat gtatccgctc atgagacaat aaccctgata aatgcttcaa taatattgaa     120

aaaggaagag tatgagtatt caacatttcc gtgtcgccct tattcccttt tttgcggcat     180

tttgccttcc tgtttttgct cacccagaaa cgctggtgaa agtaaaagat gctgaagatc     240

agttgggtgc acgagtgggt tacatcgaac tggatctcaa cagcggtaag atccttgaga     300

gttttcgccc cgaagaacgt tttccaatga tgagcacttt taaagttctg ctatgtggcg     360

cggtattatc ccgtattgac gccgggcaag agcaactcgg tcgccgcata cactattctc     420

agaatgactt ggttgagtac tcaccagtca cagaaaagca tcttacggat ggcatgacag     480

taagagaatt atgcagtgct gccataacca tgagtgataa cactgcggcc aacttacttc     540

tgacaacgat cggaggaccg aaggagctaa ccgctttttt gcacaacatg ggggatcatg     600

taactcgcct tgatcgttgg gaaccggagc tgaatgaagc cataccaaac gacgagcgtg     660

acaccacgat gcctgtagca atggcaacaa cgttgcgcaa actattaact ggcgaactac     720

ttactctagc ttcccggcaa caattaatag actggatgga ggcggataaa gttgcaggac     780

cacttctgcg ctcggccctt ccggctggct ggtttattgc tgataaatct ggagccggtg     840

agcgtgggtc tcgcggtatc attgcagcac tggggccaga tggtaagccc tcccgtatcg     900

tagttatcta cacgacgggg agtcaggcaa ctatggatga acgaaataga cagatcgctg     960

agataggtgc ctcactgatt aagcattggt aactgtcaga ccaagtttac tcatatatac    1020

tttagattga tttaaaactt catttttaat ttaaaaggat ctaggtgaag atcctttttg    1080

ataatctcat gaccaaaatc ccttaacgtg agttttcgtt ccactgagcg tcagaccccg    1140

tagaaaagat caaaggatct tcttgagatc ctttttttct gcgcgtaatc tgctgcttgc    1200

aaacaaaaaa accaccgcta ccagcggtgg tttgtttgcc ggatcaagag ctaccaactc    1260

tttttccgaa ggtaactggc ttcagcagag cgcagatacc aaatactgtc cttctagtgt    1320

agccgtagtt aggccaccac ttcaagaact ctgtagcacc gcctacatac ctcgctctgc    1380

taatcctgtt accagtggct gctgccagtg gcgataagtc gtgtcttacc gggttggact    1440

caagacgata gttaccggat aaggcgcagc ggtcgggctg aacggggggt tcgtgcacac    1500

agcccagctt ggagcgaacg acctacaccg aactgagata cctacagcgt gagctatgag    1560

aaagcgccac gcttcccgaa gggagaaagg cggacaggta tccggtaagc ggcagggtcg    1620

gaacaggaga gcgcacgagg gagcttccag ggggaaacgc ctggtatctt tatagtcctg    1680

tcgggtttcg ccacctctga cttgagcgtc gatttttgtg atgctcgtca ggggggcgga    1740

gcctatggaa aaacgccagc aacgcggcct ttttacggtt cctggccttt tgctggcctt    1800

ttgctcacat gttctttcct gcgttatccc ctgattctgt ggataaccgt attaccgcct    1860

ttgagtgagc tgataccgct cgccgcagcc gaacgaccga gcgcagcgag tcagtgagcg 1920
aggaagcgga agagcgccca atacgcaaac cgcctctccc cgcgcgttgg ccgattcatt 1980
aatgcagctg gcacgacagg tttcccgact ggaaagcggg cagtgagcgc aacgcaatta 2040
atgtgagtta gctcactcat taggcacccc aggctttaca ctttatgctt ccggctcgta 2100
tgttgtgtgg aattgtgagc ggataacaat ttcacacagg aaacagctat gaccatgatt 2160
acgccaagcg cgcaattaac cctcactaaa gggaacaaaa gctggagctg caagcttaat 2220
gtagtcttat gcaatactct tgtagtcttg caacatggta acgatgagtt agcaacatgc 2280
cttacaagga gagaaaaagc accgtgcatg ccgattggtg gaagtaaggt ggtacgatcg 2340
tgccttatta ggaaggcaac agacgggtct gacatggatt ggacgaacca ctgaattgcc 2400
gcattgcaga gatattgtat ttaagtgcct agctcgatac aataaacggg tctctctggt 2460
tagaccagat ctgagcctgg gagctctctg gctaactagg gaacccactg cttaagcctc 2520
aataaagctt gccttgagtg cttcaagtag tgtgtgcccg tctgttgtgt gactctggta 2580
actagagatc cctcagaccc ttttagtcag tgtggaaaat ctctagcagt ggcgcccgaa 2640
cagggacctg aaagcgaaag ggaaaccaga gctctctcga cgcaggactc ggcttgctga 2700
agcgcgcacg gcaagaggcg aggggcggcg actggtgagt acgccaaaaa ttttgactag 2760
cggaggctag aaggagagag atgggtgcga gagcgtcagt attaagcggg ggagaattag 2820
atcgcgatgg gaaaaaattc ggttaaggcc agggggaaag aaaaaatata aattaaaaca 2880
tatagtatgg gcaagcaggg agctagaacg attcgcagtt aatcctggcc tgttagaaac 2940
atcagaaggc tgtagacaaa tactgggaca gctacaacca tcccttcaga caggatcaga 3000
agaacttaga tcattatata atacagtagc aaccctctat tgtgtgcatc aaaggataga 3060
gataaaagac accaaggaag ctttagacaa gatagaggaa gagcaaaaca aaagtaagac 3120
caccgcacag caagcggccg ctgatcttca gacctggagg aggagatatg agggacaatt 3180
ggagaagtga attatataaa tataaagtag taaaaattga accattagga gtagcaccca 3240
ccaaggcaaa gagaagagtg gtgcagagag aaaaaagagc agtgggaata ggagctttgt 3300
tccttgggtt cttgggagca gcaggaagca ctatgggcgc agcctcaatg acgctgacgg 3360
tacaggccag acaattattg tctggtatag tgcagcagca gaacaatttg ctgagggcta 3420
ttgaggcgca acagcatctg ttgcaactca cagtctgggg catcaagcag ctccaggcaa 3480
gaatcctggc tgtggaaaga tacctaaagg atcaacagct cctggggatt tggggttgct 3540
ctggaaaact catttccacc actgctgtgc cttggaatgc tagttggagt aataaatctc 3600
tggaacagat ttggaatcac acgacctgga tggagtggga cagagaaatt aacaattaca 3660
caagcttaat acactcctta attgaagaat cgcaaaacca gcaagaaaag aatgaacaag 3720
aattattgga attagataaa tgggcaagtt tgtggaattg gtttaacata acaaattggc 3780
tgtggtatat aaaattattc ataatgatag taggaggctt ggtaggttta agaatagttt 3840
ttgctgtact ttctatagtg aatagagtta ggcagggata ttcaccatta tcgtttcaga 3900

cccacctccc aaccccgagg ggacccgaca ggcccgaagg aatagaagaa gaaggtggag 3960
agagagacag agacagatcc attcgattag tgaacggatc tcgacggtat cggttaactt 4020
ttaaaagaaa aggggggatt ggggggtaca gtgcagggga aagaatagta gacataatag 4080
caacagacat acaaactaaa gaattacaaa aacaaattac aaaaattcaa aattttatcg 4140
atcacgagac tagcctcgag aagcttgata tcgaattccc acggggttgg ggttgcgcct 4200
tttccaaggc agccctgggt ttgcgcaggg acgcggctgc tctgggcgtg gttccgggaa 4260
acgcagcggc gccgaccctg ggtctcgcac attcttcacg tccgttcgca gcgtcacccg 4320
gatcttcgcc gctacccttg tgggcccccc ggcgacgctt cctgctccgc ccctaagtcg 4380
ggaaggttcc ttgcggttcg cggcgtgccg gacgttacaa acggaagccg cacgtctcac 4440
tagtaccctc gcagacggac agcgccaggg agcaatggca gcgcgccgac cgcgatgggc 4500
tgtggccaat agcggctgct cagcggggcg cgccgagagc agcggccggg aaggggcggt 4560
gcgggaggcg gggtgtgggg cggtagtgtg ggccctgttc ctgcccgcgc ggtgttccgc 4620
attctgcaag cctccggagc gcacgtcggc agtcggctcc ctcgttgacc gaatcaccga 4680
cctctctccc caggaccatg aaacatctgt ggttcttcct cctcctagtg gcagctccca 4740
gatgggtcct gtcccaggtg cagctgcagc aatggggcgc aggactcttg aagccttcgg 4800
agaccctgtc cctcacctgc gctgtttatg gtgactcctt cggtggtcac cactggagct 4860
ggatccgcca gccccctggg aagggactag agtggatcgg cgaaataaat tttcttggca 4920
acaccaatta caatccgtcc ctcaagagtc gagtcgccat atcaaaggac acgtccaaaa 4980
atcaattctc cctgaccttg aattctgcga ccgccgcgga cacggcagtc tacttctgtg 5040
cgcgacggga aactatggct agtcgaaaga gggcttttga catctggggc caggggacaa 5100
tggtcactgt ctctgcaggt ggcggtggct ctggaggtgg tgggagcggt ggcggcggat 5160
ctgccgagct catgacccag tctcctccca ccctgtctgc atctgtagga gacagagtca 5220
ccatcacctg ccgggccagt cagagtctca gtggctgggt ggcctggtat caacaaaaac 5280
cagggaaagc ccctaaactc ctgatctata aggcgtctac cttagaaagt ggggtcccat 5340
caaggttcag cggcagtgga tctgggacag tgttcgcact caccattagc agcctgcagc 5400
ccgatgactc tgcgacttac tactgccaac aacatagtgc tttttcgtcc acttttggcc 5460
aggggaccaa actggagatc aaacgaaccc cgctgggtga caccacccac acctccggac 5520
accatcacca tcaccatgga agtggaacca cttcaggtac tacccgtctt ctatctgggc 5580
acacgtgttt cacgttgaca ggtttgcttg ggacgctagt aaccatgggc ttgctgactt 5640
agtcgacaat caacctctgg attacaaaat ttgtgaaaga ttgactggta ttcttaacta 5700
tgtggctcct tttacgctat gtggatacgc tgctttaatg cctttgtatc atgctattgc 5760
ttcccgtatg gctttcattt tctcctcctt gtataaatcc tggttgctgt ctctttatga 5820
ggagttgtgg cccgttgtca ggcaacgtgg cgtggtgtgc actgtgtttg ctgacgcaac 5880
ccccactggt tggggcattg ccaccacctg tcagctcctt tccgggactt tcgctttccc 5940

cctccctatt gccacggcgg aactcatcgc cgcctgcctt gcccgctgct ggacaggggc 6000
tcggctgttg ggcactgaca attccgtggt gttgtcgggg aagctgacct cctttccatg 6060
gctgctcgcc tgtgttgcca cctggattct gcgcgggacg tccttctgct acgtcccttc 6120
ggccctcaat ccagcggacc ttccttcccg cggcctgctg ccggctctgc ggcctcttcc 6180
gcgtcttcgc cttcgccctc agacgagtcg gatctccctt tgggccgcct ccccgcctgg 6240
aattcgagct cggtaccttt aagaccaatg acttacaagg cagctgtaga tcttagccac 6300
tttttaaaag aaaagggggg actggaaggg ctaattcact cccaacgaag acaagatctg 6360
ctttttgctt gtactgggtc tctctggtta gaccagatct gagcctggga gctctctggc 6420
taactaggga acccactgct taagcctcaa gaaagcttgc cttgagtgct tcaagtagtg 6480
tgtgcccgtc tgttgtgtga ctctggtaac tagagatccc tcagaccctt ttagtcagtg 6540
tggaaaatct ctagcagtag tagttcatgt catcttatta ttcagtattt ataacttgca 6600
aagaaatgaa tatcagagag tgagaggaac ttgtttattg cagcttataa tggttacaaa 6660
taaagcaata gcatcacaaa tttcacaaat aaagcatttt tttcactgca ttctagttgt 6720
ggtttgtcca aactcatcaa tgtatcttat catgtctggc tctagctatc ccgcccctaa 6780
ctccgcccag ttccgcccat tctccgcccc atggctgact aatttttttt atttatgcag 6840
aggccgaggc cgcctcggcc tctgagctat tccagaagta gtgaggaggc ttttttggag 6900
gcctaggctt ttgcgtcgag acgtacccaa ttcgccctat agtgagtcgt attacgcgcg 6960
ctcactggcc gtcgttttac aacgtcgtga ctgggaaaac cctggcgtta cccaacttaa 7020
tcgccttgca gcacatcccc ctttcgccag ctggcgtaat agcgaagagg cccgcaccga 7080
tcgcccttcc caacagttgc gcagcctgaa tggcgaatgg cgcgacgcgc cctgtagcgg 7140
cgcattaagc gcggcgggtg tggtggttac gcgcagcgtg accgctacac ttgccagcgc 7200
cctagcgccc gctcctttcg ctttcttccc ttcctttctc gccacgttcg ccggctttcc 7260
ccgtcaagct ctaaatcggg ggctcccttt agggttccga tttagtgctt tacggcacct 7320
cgaccccaaa aaacttgatt agggtgatgg ttcacgtagt gggccatcgc cctgatagac 7380
ggtttttcgc cctttgacgt tggagtccac gttctttaat agtggactct tgttccaaac 7440
tggaacaaca ctcaacccta tctcggtcta ttcttttgat ttataaggga ttttgccgat 7500
ttcggcctat tggttaaaaa atgagctgat ttaacaaaaa tttaacgcga attttaacaa 7560
aatattaacg tttacaattt cc 7582

<210> 7
<211> 7883
<212> DNA
<213> Artificial

<220>
<223> pLNCX-TCR-m-scFv vector

<400> 7
tttgaaagac cccacccgta ggtggcaagc tagcttaagt aacgccactt tgcaaggcat 60

ggaaaaatac ataactgaga atagaaaagt tcagatcaag gtcaggaaca aagaaacagc 120
tgaataccaa acaggatatc tgtggtaagc ggttcctgcc ccggctcagg gccaagaaca 180
gatgagacag ctgagtgatg ggccaaacag gatatctgtg gtaagcagtt cctgccccgg 240
ctcggggcca agaacagatg gtccccagat gcggtccagc cctcagcagt ttctagtgaa 300
tcatcagatg tttccagggt gccccaagga cctgaaaatg accctgtacc ttatttgaac 360
taaccaatca gttcgcttct cgcttctgtt cgcgcgcttc cgctctccga gctcaataaa 420
agagcccaca acccctcact cggcgcgcca gtcttccgat agactgcgtc gcccgggtac 480
ccgtattccc aataaagcct cttgctgttt gcatccgaat cgtggtctcg ctgttccttg 540
ggagggtctc ctctgagtga ttgactaccc acgacggggg tctttcattt gggggctcgt 600
ccgggatttg gagacccctg cccagggacc accgacccac caccgggagg taagctggcc 660
agcaacttat ctgtgtctgt ccgattgtct agtgtctatg tttgatgtta tgcgcctgcg 720
tctgtactag ttagctaact agctctgtat ctggcggacc cgtggtggaa ctgacgagtt 780
ctgaacaccc ggccgcaacc ctgggagacg tcccagggac tttgggggcc gtttttgtgg 840
cccgacctga ggaagggagt cgatgtggaa tccgaccccg tcaggatatg tggttctggt 900
aggagacgag aacctaaaac agttcccgcc tccgtctgaa tttttgcttt cggtttggaa 960
ccgaagccgc gcgtcttgtc tgctgcagcg ctgcagcatc gttctgtgtt gtctctgtct 1020
gactgtgttt ctgtatttgt ctgaaaatta gggccagact gttaccactc ccttaagttt 1080
gaccttaggt cactggaaag atgtcgagcg gatcgctcac aaccagtcgg tagatgtcaa 1140
gaagagacgt tgggttacct tctgctctgc agaatggcca acctttaacg tcggatggcc 1200
gcgagacggc acctttaacc gagacctcat cacccaggtt aagatcaagg tcttttcacc 1260
tggcccgcat ggacacccag accaggtccc ctacatcgtg acctgggaag ccttggcttt 1320
tgacccccct ccctgggtca agccctttgt acaccctaag cctccgcctc ctcttcctcc 1380
atccgccccg tctctccccc ttgaacctcc tcgttcgacc ccgcctcgat cctcccttta 1440
tccagccctc actccttctc taggcgccgg aattccgatc tgatcaagag acaggatgag 1500
gatcgtttcg catgattgaa caagatggat tgcacgcagg ttctccggcc gcttgggtgg 1560
agaggctatt cggctatgac tgggcacaac agacaatcgg ctgctctgat gccgccgtgt 1620
tccggctgtc agcgcagggg cgcccggttc tttttgtcaa gaccgacctg tccggtgccc 1680
tgaatgaact gcaggacgag gcagcgcggc tatcgtggct ggccacgacg ggcgttcctt 1740
gcgcagctgt gctcgacgtt gtcactgaag cgggaaggga ctggctgcta ttgggcgaag 1800
tgccggggca ggatctcctg tcatctcacc ttgctcctgc cgagaaagta tccatcatgg 1860
ctgatgcaat gcggcggctg catacgcttg atccggctac ctgcccattc gaccaccaag 1920
cgaaacatcg catcgagcga gcacgtactc ggatggaagc cggtcttgtc gatcaggatg 1980
atctggacga agagcatcag gggctcgcgc cagccgaact gttcgccagg ctcaaggcgc 2040
gcatgcccga cggcgaggat ctcgtcgtga cccatggcga tgcctgcttg ccgaatatca 2100

tggtggaaaa tggccgcttt tctggattca tcgactgtgg ccggctgggt gtggcggacc 2160
gctatcagga catagcgttg gctacccgtg atattgctga agagcttggc ggcgaatggg 2220
ctgaccgctt cctcgtgctt tacggtatcg ccgctcccga ttcgcagcgc atcgccttct 2280
atcgccttct tgacgagttc ttctgagcgg gactctgggg ttcgaaatga ccgaccaagc 2340
gacgcccaac ctgccatcac gagatttcga ttccaccgcc gccttctatg aaaggttggg 2400
cttcggaatc gttttccggg acgccggctg gatgatcctc cagcgcgggg atctcatgct 2460
ggagttcttc gcccaccccg ggctcgatcc cctcgcgagt tggttcagct gctgcctgag 2520
gctggacgac ctcgcggagt tctaccggca gtgcaaatcc gtcggcatcc aggaaaccag 2580
cagcggctat ccgcgcatcc atgcccccga actgcaggag tggggaggca cgatggccgc 2640
tttggtcgag gcggatctat tctagcaagc cttaattatc caactaaatc agaccaggat 2700
taagagatac tcttaaataa acaaggagat agggtgttta ttttatggac aagtttcttt 2760
tgtaacttgt aactcccttg aaagtcagcc agagtatgtc tcaaaccaaa gtcaagatag 2820
tgagcaagaa gtgttgcact tatgaaggga ggtgagtgag caatgcatgt ggtttccaac 2880
cgttaatgct agagttatca ctttctgtta tcaagtggct tcagctatgc aaggaaacca 2940
aacaggggaa gttttctcaa gcaggttgaa agcaggttcc aagaaagccc tttgaaaaat 3000
gtctaatcct ttcaattggg atccggccat tagccatatt attcattggt tatatagcat 3060
aaatcaatat tggctattgg ccattgcata cgttgtatcc atatcataat atgtacattt 3120
atattggctc atgtccaaca ttaccgccat gttgacattg attattgact agttattaat 3180
agtaatcaat tacggggtca ttagttcata gcccatatat ggagttccgc gttacataac 3240
ttacggtaaa tggcccgcct ggctgaccgc ccaacgaccc ccgcccattg acgtcaataa 3300
tgacgtatgt tcccatagta acgccaatag ggactttcca ttgacgtcaa tgggtggagt 3360
atttacggta aactgcccac ttggcagtac atcaagtgta tcatatgcca agtacgcccc 3420
ctattgacgt caatgacggt aaatggcccg cctggcatta tgcccagtac atgaccttat 3480
gggactttcc tacttggcag tacatctacg tattagtcat cgctattacc atggtgatgc 3540
ggttttggca gtacatcaat gggcgtggat agcggtttga ctcacgggga tttccaagtc 3600
tccaccccat tgacgtcaat gggagtttgt tttggcacca aaatcaacgg gactttccaa 3660
aatgtcgtaa caactccgcc ccattgacgc aaatgggcgg taggcatgta cggtgggagg 3720
tctatataag cagagctcgt ttagtgaacc gtcagatcgc ctggagacgc catccacgct 3780
gttttgacct ccatagaaga caccgggacc gatccagcct ccgcggcccc aagcttacca 3840
tgaaacatct gtggttcttc ctcctcctag tggcagctcc cagatgggtc ctgtcccagg 3900
tgcagctgca gcaatggggc gcaggactct tgaagccttc ggagaccctg tccctcacct 3960
gcgctgttta tggtgactcc ttcggtggtc accactggag ctggatccgc cagccccctg 4020
ggaagggact agagtggatc ggcgaaataa attttcttgg caacaccaat tacaatccgt 4080
ccctcaagag tcgagtcgcc atatcaaagg acacgtccaa aaatcaattc tccctgacct 4140

tgaattctgc gaccgccgcg gacacggcag tctacttctg tgcgcgacgg gaaactatgg 4200
ctagtcgaaa gagggctttt gacatctggg gccaggggac aatggtcact gtctctgcag 4260
gtggcggtgg ctctggaggt ggtgggagcg gtggcggcgg atctgccgag ctcatgaccc 4320
agtctcctcc caccctgtct gcatctgtag gagacagagt caccatcacc tgccgggcca 4380
gtcagagtct cagtggctgg gtggcctggt atcaacaaaa accagggaaa gcccctaaac 4440
tcctgatcta taaggcgtct accttagaaa gtggggtccc atcaaggttc agcggcagtg 4500
gatctgggac agtgttcgca ctcaccatta gcagcctgca gcccgatgac tctgcgactt 4560
actactgcca acaacatagt gctttttcgt ccacttttgg ccaggggacc aaactggaga 4620
tcaaacgaac cccgctgggt gacaccaccc acacctccgg aatttggctt atagttggaa 4680
tttgtattgc attatttgct ctcccgtttg tcatttatgc tgcgtaaaag cttgttaaca 4740
tcgataaaat aaaagatttt atttagtctc cagaaaaagg ggggaatgaa agaccccacc 4800
tgtaggtttg gcaagctagc ttaagtaacg ccattttgca aggcatggaa aaatacataa 4860
ctgagaatag agaagttcag atcaaggtca ggaacagatg gaacagctga atatgggcca 4920
aacaggatat ctgtggtaag cagttcctgc cccggctcag ggccaagaac agatggaaca 4980
gctgaatatg ggccaaacag gatatctgtg gtaagcagtt cctgccccgg ctcagggcca 5040
agaacagatg gtccccagat gcggtccagc cctcagcagt ttctagagaa ccatcagatg 5100
tttccagggt gccccaagga cctgaaatga ccctgtgcct tatttgaact aaccaatcag 5160
ttcgcttctc gcttctgttc gcgcgcttct gctccccgag ctcaataaaa gagcccacaa 5220
cccctcactc ggggcgccag tcctccgatt gactgagtcg cccgggtacc cgtgtatcca 5280
ataaaccctc ttgcagttgc atccgacttg tggtctcgct gttccttggg agggtctcct 5340
ctgagtgatt gactacccgt cagcgggggt ctttcatttg ggggctcgtc cgggatcggg 5400
agacccctgc ccagggacca ccgacccacc accgggaggt aagctggctg cctcgcgcgt 5460
ttcggtgatg acggtgaaaa cctctgacac atgcagctcc cggagacggt cacagcttgt 5520
ctgtaagcgg atgccgggag cagacaagcc cgtcagggcg cgtcagcggg tgttggcggg 5580
tgtcggggcg cagccatgac ccagtcacgt agcgatagcg gagtgtatac tggcttaact 5640
atgcggcatc agagcagatt gtactgagag tgcaccatat gcggtgtgaa ataccgcaca 5700
gatgcgtaag gagaaaatac cgcatcaggc gctcttccgc ttcctcgctc actgactcgc 5760
tgcgctcggt cgttcggctg cggcgagcgg tatcagctca ctcaaaggcg gtaatacggt 5820
tatccacaga atcaggggat aacgcaggaa agaacatgtg agcaaaaggc cagcaaaagg 5880
ccaggaaccg taaaaaggcc gcgttgctgg cgtttttcca taggctccgc ccccctgacg 5940
agcatcacaa aaatcgacgc tcaagtcaga ggtggcgaaa cccgacagga ctataaagat 6000
accaggcgtt tccccctgga agctccctcg tgcgctctcc tgttccgacc ctgccgctta 6060
ccggatacct gtccgccttt ctcccttcgg gaagcgtggc gctttctcat agctcacgct 6120
gtaggtatct cagttcggtg taggtcgttc gctccaagct gggctgtgtg cacgaacccc 6180

ccgttcagcc cgaccgctgc gccttatccg gtaactatcg tcttgagtcc aacccggtaa 6240
gacacgactt atcgccactg gcagcagcca ctggtaacag gattagcaga gcgaggtatg 6300
taggcggtgc tacagagttc ttgaagtggt ggcctaacta cggctacact agaaggacag 6360
tatttggtat ctgcgctctg ctgaagccag ttaccttcgg aaaaagagtt ggtagctctt 6420
gatccggcaa acaaaccacc gctggtagcg gtggtttttt tgtttgcaag cagcagatta 6480
cgcgcagaaa aaaaggatct caagaagatc ctttgatctt ttctacgggg tctgacgctc 6540
agtggaacga aaactcacgt taagggattt tggtcatgag attatcaaaa aggatcttca 6600
cctagatcct tttaaattaa aaatgaagtt ttaaatcaat ctaaagtata tatgagtaaa 6660
cttggtctga cagttaccaa tgcttaatca gtgaggcacc tatctcagcg atctgtctat 6720
ttcgttcatc catagttgcc tgactccccg tcgtgtagat aactacgata cgggagggct 6780
taccatctgg ccccagtgct gcaatgatac cgcgagaccc acgctcaccg gctccagatt 6840
tatcagcaat aaaccagcca gccggaaggg ccgagcgcag aagtggtcct gcaactttat 6900
ccgcctccat ccagtctatt aattgttgcc gggaagctag agtaagtagt tcgccagtta 6960
atagtttgcg caacgttgtt gccattgctg caggcatcgt ggtgtcacgc tcgtcgtttg 7020
gtatggcttc attcagctcc ggttcccaac gatcaaggcg agttacatga tcccccatgt 7080
tgtgcaaaaa agcggttagc tccttcggtc ctccgatcgt tgtcagaagt aagttggccg 7140
cagtgttatc actcatggtt atggcagcac tgcataattc tcttactgtc atgccatccg 7200
taagatgctt ttctgtgact ggtgagtact caaccaagtc attctgagaa tagtgtatgc 7260
ggcgaccgag ttgctcttgc ccggcgtcaa cacgggataa taccgcgcca catagcagaa 7320
ctttaaaagt gctcatcatt ggaaaacgtt cttcggggcg aaaactctca aggatcttac 7380
cgctgttgag atccagttcg atgtaaccca ctcgtgcacc caactgatct tcagcatctt 7440
ttactttcac cagcgtttct gggtgagcaa aaacaggaag gcaaaatgcc gcaaaaaagg 7500
gaataagggc gacacggaaa tgttgaatac tcatactctt cctttttcaa tattattgaa 7560
gcatttatca gggttattgt ctcatgagcg gatacatatt tgaatgtatt tagaaaaata 7620
aacaaatagg ggttccgcgc acatttcccc gaaaagtgcc acctgacgtc taagaaacca 7680
ttattatcat gacattaacc tataaaaata ggcgtatcac gaggcccttt cgtcttcaag 7740
aattcatacc agatcaccga aaactgtcct ccaaatgtgt ccccctcaca ctcccaaatt 7800
cgcgggcttc tgcctcttag accactctac cctattcccc acactcaccg gagccaaagc 7860
cgcggccctt ccgtttcttt gct 7883

<210> 8
<211> 6642
<212> DNA
<213> Artificial

<220>
<223> pRRLsin-18.PPT.hPGK.Wpre vector

<400> 8

caggtggcac ttttcgggga aatgtgcgcg gaacccctat ttgtttattt ttctaaatac 60
attcaaatat gtatccgctc atgagacaat aaccctgata aatgcttcaa taatattgaa 120
aaaggaagag tatgagtatt caacatttcc gtgtcgccct tattcccttt tttgcggcat 180
tttgccttcc tgtttttgct cacccagaaa cgctggtgaa agtaaaagat gctgaagatc 240
agttgggtgc acgagtgggt tacatcgaac tggatctcaa cagcggtaag atccttgaga 300
gttttcgccc cgaagaacgt tttccaatga tgagcacttt taaagttctg ctatgtggcg 360
cggtattatc ccgtattgac gccgggcaag agcaactcgg tcgccgcata cactattctc 420
agaatgactt ggttgagtac tcaccagtca cagaaaagca tcttacggat ggcatgacag 480
taagagaatt atgcagtgct gccataacca tgagtgataa cactgcggcc aacttacttc 540
tgacaacgat cggaggaccg aaggagctaa ccgctttttt gcacaacatg ggggatcatg 600
taactcgcct tgatcgttgg gaaccggagc tgaatgaagc cataccaaac gacgagcgtg 660
acaccacgat gcctgtagca atggcaacaa cgttgcgcaa actattaact ggcgaactac 720
ttactctagc ttcccggcaa caattaatag actggatgga ggcggataaa gttgcaggac 780
cacttctgcg ctcggccctt ccggctggct ggtttattgc tgataaatct ggagccggtg 840
agcgtgggtc tcgcggtatc attgcagcac tggggccaga tggtaagccc tcccgtatcg 900
tagttatcta cacgacgggg agtcaggcaa ctatggatga acgaaataga cagatcgctg 960
agataggtgc ctcactgatt aagcattggt aactgtcaga ccaagtttac tcatatatac 1020
tttagattga tttaaaactt catttttaat ttaaaaggat ctaggtgaag atcctttttg 1080
ataatctcat gaccaaaatc ccttaacgtg agttttcgtt ccactgagcg tcagaccccg 1140
tagaaaagat caaaggatct tcttgagatc ctttttttct gcgcgtaatc tgctgcttgc 1200
aaacaaaaaa accaccgcta ccagcggtgg tttgtttgcc ggatcaagag ctaccaactc 1260
tttttccgaa ggtaactggc ttcagcagag cgcagatacc aaatactgtc cttctagtgt 1320
agccgtagtt aggccaccac ttcaagaact ctgtagcacc gcctacatac ctcgctctgc 1380
taatcctgtt accagtggct gctgccagtg gcgataagtc gtgtcttacc gggttggact 1440
caagacgata gttaccggat aaggcgcagc ggtcgggctg aacggggggt tcgtgcacac 1500
agcccagctt ggagcgaacg acctacaccg aactgagata cctacagcgt gagctatgag 1560
aaagcgccac gcttcccgaa gggagaaagg cggacaggta tccggtaagc ggcagggtcg 1620
gaacaggaga gcgcacgagg gagcttccag ggggaaacgc ctggtatctt tatagtcctg 1680
tcgggtttcg ccacctctga cttgagcgtc gatttttgtg atgctcgtca ggggggcgga 1740
gcctatggaa aaacgccagc aacgcggcct ttttacggtt cctggccttt tgctggcctt 1800
ttgctcacat gttctttcct gcgttatccc ctgattctgt ggataaccgt attaccgcct 1860
ttgagtgagc tgataccgct cgccgcagcc gaacgaccga gcgcagcgag tcagtgagcg 1920
aggaagcgga agagcgccca atacgcaaac cgcctctccc cgcgcgttgg ccgattcatt 1980
aatgcagctg gcacgacagg tttcccgact ggaaagcggg cagtgagcgc aacgcaatta 2040

atgtgagtta gctcactcat taggcacccc aggctttaca ctttatgctt ccggctcgta 2100
tgttgtgtgg aattgtgagc ggataacaat ttcacacagg aaacagctat gaccatgatt 2160
acgccaagcg cgcaattaac cctcactaaa gggaacaaaa gctggagctg caagcttaat 2220
gtagtcttat gcaatactct tgtagtcttg caacatggta acgatgagtt agcaacatgc 2280
cttacaagga gagaaaaagc accgtgcatg ccgattggtg gaagtaaggt ggtacgatcg 2340
tgccttatta ggaaggcaac agacgggtct gacatggatt ggacgaacca ctgaattgcc 2400
gcattgcaga gatattgtat ttaagtgcct agctcgatac aataaacggg tctctctggt 2460
tagaccagat ctgagcctgg gagctctctg gctaactagg gaacccactg cttaagcctc 2520
aataaagctt gccttgagtg cttcaagtag tgtgtgcccg tctgttgtgt gactctggta 2580
actagagatc cctcagaccc ttttagtcag tgtggaaaat ctctagcagt ggcgcccgaa 2640
cagggacctg aaagcgaaag ggaaaccaga gctctctcga cgcaggactc ggcttgctga 2700
agcgcgcacg gcaagaggcg aggggcggcg actggtgagt acgccaaaaa ttttgactag 2760
cggaggctag aaggagagag atgggtgcga gagcgtcagt attaagcggg ggagaattag 2820
atcgcgatgg gaaaaaattc ggttaaggcc agggggaaag aaaaaatata aattaaaaca 2880
tatagtatgg gcaagcaggg agctagaacg attcgcagtt aatcctggcc tgttagaaac 2940
atcagaaggc tgtagacaaa tactgggaca gctacaacca tcccttcaga caggatcaga 3000
agaacttaga tcattatata atacagtagc aaccctctat tgtgtgcatc aaaggataga 3060
gataaaagac accaaggaag ctttagacaa gatagaggaa gagcaaaaca aaagtaagac 3120
caccgcacag caagcggccg ctgatcttca gacctggagg aggagatatg agggacaatt 3180
ggagaagtga attatataaa tataaagtag taaaaattga accattagga gtagcaccca 3240
ccaaggcaaa gagaagagtg gtgcagagag aaaaaagagc agtgggaata ggagctttgt 3300
tccttgggtt cttgggagca gcaggaagca ctatgggcgc agcctcaatg acgctgacgg 3360
tacaggccag acaattattg tctggtatag tgcagcagca gaacaatttg ctgagggcta 3420
ttgaggcgca acagcatctg ttgcaactca cagtctgggg catcaagcag ctccaggcaa 3480
gaatcctggc tgtggaaaga tacctaaagg atcaacagct cctggggatt tggggttgct 3540
ctggaaaact catttccacc actgctgtgc cttggaatgc tagttggagt aataaatctc 3600
tggaacagat ttggaatcac acgacctgga tggagtggga cagagaaatt aacaattaca 3660
caagcttaat acactcctta attgaagaat cgcaaaacca gcaagaaaag aatgaacaag 3720
aattattgga attagataaa tgggcaagtt tgtggaattg gtttaacata acaaattggc 3780
tgtggtatat aaaattattc ataatgatag taggaggctt ggtaggttta agaatagttt 3840
ttgctgtact ttctatagtg aatagagtta ggcagggata ttcaccatta tcgtttcaga 3900
cccacctccc aaccccgagg ggacccgaca ggcccgaagg aatagaagaa gaaggtggag 3960
agagagacag agacagatcc attcgattag tgaacggatc tcgacggtat cggttaactt 4020
ttaaaagaaa aggggggatt ggggggtaca gtgcagggga aagaatagta gacataatag 4080

caacagacat acaaactaaa gaattacaaa aacaaattac aaaaattcaa aattttatcg 4140
atcacgagac tagcctcgag aagcttgata tcgaattccc acggggttgg ggttgcgcct 4200
tttccaaggc agccctgggt ttgcgcaggg acgcggctgc tctgggcgtg gttccgggaa 4260
acgcagcggc gccgaccctg ggtctcgcac attcttcacg tccgttcgca gcgtcacccg 4320
gatcttcgcc gctacccttg tgggcccccc ggcgacgctt cctgctccgc ccctaagtcg 4380
ggaaggttcc ttgcggttcg cggcgtgccg gacgttacaa acggaagccg cacgtctcac 4440
tagtaccctc gcagacggac agcgccaggg agcaatggca gcgcgccgac cgcgatgggc 4500
tgtggccaat agcggctgct cagcggggcg cgccgagagc agcggccggg aaggggcggt 4560
gcgggaggcg gggtgtgggg cggtagtgtg ggccctgttc ctgcccgcgc ggtgttccgc 4620
attctgcaag cctccggagc gcacgtcggc agtcggctcc ctcgttgacc gaatcaccga 4680
cctctctccc cagggggatc cgtcgacaat caacctctgg attacaaaat ttgtgaaaga 4740
ttgactggta ttcttaacta tgtggctcct tttacgctat gtggatacgc tgctttaatg 4800
cctttgtatc atgctattgc ttcccgtatg gctttcattt tctcctcctt gtataaatcc 4860
tggttgctgt ctctttatga ggagttgtgg cccgttgtca ggcaacgtgg cgtggtgtgc 4920
actgtgtttg ctgacgcaac ccccactggt tggggcattg ccaccacctg tcagctcctt 4980
tccgggactt tcgctttccc cctccctatt gccacggcgg aactcatcgc cgcctgcctt 5040
gcccgctgct ggacaggggc tcggctgttg ggcactgaca attccgtggt gttgtcgggg 5100
aagctgacct cctttccatg gctgctcgcc tgtgttgcca cctggattct gcgcgggacg 5160
tccttctgct acgtcccttc ggccctcaat ccagcggacc ttccttcccg cggcctgctg 5220
ccggctctgc ggcctcttcc gcgtcttcgc cttcgccctc agacgagtcg gatctccctt 5280
tgggccgcct ccccgcctgg aattcgagct cggtaccttt aagaccaatg acttacaagg 5340
cagctgtaga tcttagccac tttttaaaag aaaagggggg actggaaggg ctaattcact 5400
cccaacgaag acaagatctg ctttttgctt gtactgggtc tctctggtta gaccagatct 5460
gagcctggga gctctctggc taactaggga acccactgct taagcctcaa gaaagcttgc 5520
cttgagtgct tcaagtagtg tgtgcccgtc tgttgtgtga ctctggtaac tagagatccc 5580
tcagaccctt ttagtcagtg tggaaaatct ctagcagtag tagttcatgt catcttatta 5640
ttcagtattt ataacttgca aagaaatgaa tatcagagag tgagaggaac ttgtttattg 5700
cagcttataa tggttacaaa taaagcaata gcatcacaaa tttcacaaat aaagcatttt 5760
tttcactgca ttctagttgt ggtttgtcca aactcatcaa tgtatcttat catgtctggc 5820
tctagctatc ccgcccctaa ctccgcccag ttccgcccat tctccgcccc atggctgact 5880
aatttttttt atttatgcag aggccgaggc cgcctcggcc tctgagctat tccagaagta 5940
gtgaggaggc ttttttggag gcctaggctt ttgcgtcgag acgtacccaa ttcgccctat 6000
agtgagtcgt attacgcgcg ctcactggcc gtcgttttac aacgtcgtga ctgggaaaac 6060
cctggcgtta cccaacttaa tcgccttgca gcacatcccc ctttcgccag ctggcgtaat 6120

agcgaagagg cccgcaccga tcgcccttcc caacagttgc gcagcctgaa tggcgaatgg 6180

cgcgacgcgc cctgtagcgg cgcattaagc gcggcgggtg tggtggttac gcgcagcgtg 6240

accgctacac ttgccagcgc cctagcgccc gctcctttcg ctttcttccc ttcctttctc 6300

gccacgttcg ccggctttcc ccgtcaagct ctaaatcggg ggctcccttt agggttccga 6360

tttagtgctt tacggcacct cgaccccaaa aaacttgatt agggtgatgg ttcacgtagt 6420

gggccatcgc cctgatagac ggtttttcgc cctttgacgt tggagtccac gttctttaat 6480

agtggactct tgttccaaac tggaacaaca ctcaacccta tctcggtcta ttcttttgat 6540

ttataaggga ttttgccgat ttcggcctat tggttaaaaa atgagctgat ttaacaaaaa 6600

tttaacgcga attttaacaa aatattaacg tttacaattt cc 6642

<210> 9
<211> 7530
<212> DNA
<213> Artificial

<220>
<223> pRRL-m-scFv vector

<400> 9
caggtggcac ttttcgggga aatgtgcgcg gaacccctat ttgtttattt ttctaaatac 60

attcaaatat gtatccgctc atgagacaat aaccctgata aatgcttcaa taatattgaa 120

aaaggaagag tatgagtatt caacatttcc gtgtcgccct tattcccttt tttgcggcat 180

tttgccttcc tgtttttgct cacccagaaa cgctggtgaa agtaaaagat gctgaagatc 240

agttgggtgc acgagtgggt tacatcgaac tggatctcaa cagcggtaag atccttgaga 300

gttttcgccc cgaagaacgt tttccaatga tgagcacttt taaagttctg ctatgtggcg 360

cggtattatc ccgtattgac gccgggcaag agcaactcgg tcgccgcata cactattctc 420

agaatgactt ggttgagtac tcaccagtca cagaaaagca tcttacggat ggcatgacag 480

taagagaatt atgcagtgct gccataacca tgagtgataa cactgcggcc aacttacttc 540

tgacaacgat cggaggaccg aaggagctaa ccgctttttt gcacaacatg ggggatcatg 600

taactcgcct tgatcgttgg gaaccggagc tgaatgaagc cataccaaac gacgagcgtg 660

acaccacgat gcctgtagca atggcaacaa cgttgcgcaa actattaact ggcgaactac 720

ttactctagc ttcccggcaa caattaatag actggatgga ggcggataaa gttgcaggac 780

cacttctgcg ctcggccctt ccggctggct ggtttattgc tgataaatct ggagccggtg 840

agcgtgggtc tcgcggtatc attgcagcac tggggccaga tggtaagccc tcccgtatcg 900

tagttatcta cacgacgggg agtcaggcaa ctatggatga acgaaataga cagatcgctg 960

agataggtgc ctcactgatt aagcattggt aactgtcaga ccaagtttac tcatatatac 1020

tttagattga tttaaaactt catttttaat ttaaaaggat ctaggtgaag atcctttttg 1080

ataatctcat gaccaaaatc ccttaacgtg agttttcgtt ccactgagcg tcagaccccg 1140

tagaaaagat caaaggatct tcttgagatc ctttttttct gcgcgtaatc tgctgcttgc 1200

aaacaaaaaa accaccgcta ccagcggtgg tttgtttgcc ggatcaagag ctaccaactc 1260
tttttccgaa ggtaactggc ttcagcagag cgcagatacc aaatactgtc cttctagtgt 1320
agccgtagtt aggccaccac ttcaagaact ctgtagcacc gcctacatac ctcgctctgc 1380
taatcctgtt accagtggct gctgccagtg gcgataagtc gtgtcttacc gggttggact 1440
caagacgata gttaccggat aaggcgcagc ggtcgggctg aacggggggt tcgtgcacac 1500
agcccagctt ggagcgaacg acctacaccg aactgagata cctacagcgt gagctatgag 1560
aaagcgccac gcttcccgaa gggagaaagg cggacaggta tccggtaagc ggcagggtcg 1620
gaacaggaga gcgcacgagg gagcttccag ggggaaacgc ctggtatctt tatagtcctg 1680
tcgggtttcg ccacctctga cttgagcgtc gatttttgtg atgctcgtca ggggggcgga 1740
gcctatggaa aaacgccagc aacgcggcct ttttacggtt cctggccttt tgctggcctt 1800
ttgctcacat gttctttcct gcgttatccc ctgattctgt ggataaccgt attaccgcct 1860
ttgagtgagc tgataccgct cgccgcagcc gaacgaccga gcgcagcgag tcagtgagcg 1920
aggaagcgga agagcgccca atacgcaaac cgcctctccc cgcgcgttgg ccgattcatt 1980
aatgcagctg gcacgacagg tttcccgact ggaaagcggg cagtgagcgc aacgcaatta 2040
atgtgagtta gctcactcat taggcacccc aggctttaca ctttatgctt ccggctcgta 2100
tgttgtgtgg aattgtgagc ggataacaat ttcacacagg aaacagctat gaccatgatt 2160
acgccaagcg cgcaattaac cctcactaaa gggaacaaaa gctggagctg caagcttaat 2220
gtagtcttat gcaatactct tgtagtcttg caacatggta acgatgagtt agcaacatgc 2280
cttacaagga gagaaaaagc accgtgcatg ccgattggtg gaagtaaggt ggtacgatcg 2340
tgccttatta ggaaggcaac agacgggtct gacatggatt ggacgaacca ctgaattgcc 2400
gcattgcaga gatattgtat ttaagtgcct agctcgatac aataaacggg tctctctggt 2460
tagaccagat ctgagcctgg gagctctctg gctaactagg gaacccactg cttaagcctc 2520
aataaagctt gccttgagtg cttcaagtag tgtgtgcccg tctgttgtgt gactctggta 2580
actagagatc cctcagaccc ttttagtcag tgtggaaaat ctctagcagt ggcgcccgaa 2640
cagggacctg aaagcgaaag ggaaaccaga gctctctcga cgcaggactc ggcttgctga 2700
agcgcgcacg gcaagaggcg aggggcggcg actggtgagt acgccaaaaa ttttgactag 2760
cggaggctag aaggagagag atgggtgcga gagcgtcagt attaagcggg ggagaattag 2820
atcgcgatgg gaaaaaattc ggttaaggcc agggggaaag aaaaaatata aattaaaaca 2880
tatagtatgg gcaagcaggg agctagaacg attcgcagtt aatcctggcc tgttagaaac 2940
atcagaaggc tgtagacaaa tactgggaca gctacaacca tcccttcaga caggatcaga 3000
agaacttaga tcattatata atacagtagc aaccctctat tgtgtgcatc aaaggataga 3060
gataaaagac accaaggaag ctttagacaa gatagaggaa gagcaaaaca aaagtaagac 3120
caccgcacag caagcggccg ctgatcttca gacctggagg aggagatatg agggacaatt 3180
ggagaagtga attatataaa tataaagtag taaaaattga accattagga gtagcaccca 3240

ccaaggcaaa gagaagagtg gtgcagagag aaaaaagagc agtgggaata ggagctttgt 3300
tccttgggtt cttgggagca gcaggaagca ctatgggcgc agcctcaatg acgctgacgg 3360
tacaggccag acaattattg tctggtatag tgcagcagca gaacaatttg ctgagggcta 3420
ttgaggcgca acagcatctg ttgcaactca cagtctgggg catcaagcag ctccaggcaa 3480
gaatcctggc tgtggaaaga tacctaaagg atcaacagct cctggggatt tggggttgct 3540
ctggaaaact catttccacc actgctgtgc cttggaatgc tagttggagt aataaatctc 3600
tggaacagat ttggaatcac acgacctgga tggagtggga cagagaaatt aacaattaca 3660
caagcttaat acactcctta attgaagaat cgcaaaacca gcaagaaaag aatgaacaag 3720
aattattgga attagataaa tgggcaagtt tgtggaattg gtttaacata acaaattggc 3780
tgtggtatat aaaattattc ataatgatag taggaggctt ggtaggttta agaatagttt 3840
ttgctgtact ttctatagtg aatagagtta ggcagggata ttcaccatta tcgtttcaga 3900
cccacctccc aaccccgagg ggacccgaca ggcccgaagg aatagaagaa gaaggtggag 3960
agagagacag agacagatcc attcgattag tgaacggatc tcgacggtat cggttaactt 4020
ttaaaagaaa aggggggatt ggggggtaca gtgcagggga aagaatagta gacataatag 4080
caacagacat acaaactaaa gaattacaaa aacaaattac aaaaattcaa aattttatcg 4140
atcacgagac tagcctcgag aagcttgata tcgaattccc acggggttgg ggttgcgcct 4200
tttccaaggc agccctgggt ttgcgcaggg acgcggctgc tctgggcgtg gttccgggaa 4260
acgcagcggc gccgaccctg ggtctcgcac attcttcacg tccgttcgca gcgtcacccg 4320
gatcttcgcc gctacccttg tgggcccccc ggcgacgctt cctgctccgc ccctaagtcg 4380
ggaaggttcc ttgcggttcg cggcgtgccg gacgttacaa acggaagccg cacgtctcac 4440
tagtaccctc gcagacggac agcgccaggg agcaatggca gcgcgccgac cgcgatgggc 4500
tgtggccaat agcggctgct cagcggggcg cgccgagagc agcggccggg aaggggcggt 4560
gcgggaggcg gggtgtgggg cggtagtgtg ggccctgttc ctgcccgcgc ggtgttccgc 4620
attctgcaag cctccggagc gcacgtcggc agtcggctcc ctcgttgacc gaatcaccga 4680
cctctctccc cagggggatc catgaaacat ctgtggttct tcctcctcct agtggcagct 4740
cccagatggg tcctgtccca ggtgcagctg cagcaatggg gcgcaggact cttgaagcct 4800
tcggagaccc tgtccctcac ctgcgctgtt tatggtgact ccttcggtgg tcaccactgg 4860
agctggatcc gccagccccc tgggaaggga ctagagtgga tcggcgaaat aaattttctt 4920
ggcaacacca attacaatcc gtccctcaag agtcgagtcg ccatatcaaa ggacacgtcc 4980
aaaaatcaat tctccctgac cttgaattct gcgaccgccg cggacacggc agtctacttc 5040
tgtgcgcgac gggaaactat ggctagtcga aagagggctt ttgacatctg gggccagggg 5100
acaatggtca ctgtctctgc aggtggcggt ggctctggag gtggtgggag cggtggcggc 5160
ggatctgccg agctcatgac ccagtctcct cccaccctgt ctgcatctgt aggagacaga 5220
gtcaccatca cctgccgggc cagtcagagt ctcagtggct gggtggcctg gtatcaacaa 5280

aaaccaggga aagcccctaa actcctgatc tataaggcgt ctaccttaga aagtggggtc 5340
ccatcaaggt tcagcggcag tggatctggg acagtgttcg cactcaccat tagcagcctg 5400
cagcccgatg actctgcgac ttactactgc caacaacata gtgctttttc gtccactttt 5460
ggccagggga ccaaactgga gatcaaacga accccgctgg gtgacaccac ccacacctcc 5520
ggaatttggc ttatagttgg aatttgtatt gcattatttg ctctcccgtt tgtcatttat 5580
gctgcgtaag tcgacaatca acctctggat tacaaaattt gtgaaagatt gactggtatt 5640
cttaactatg tggctccttt tacgctatgt ggatacgctg ctttaatgcc tttgtatcat 5700
gctattgctt cccgtatggc tttcattttc tcctccttgt ataaatcctg gttgctgtct 5760
ctttatgagg agttgtggcc cgttgtcagg caacgtggcg tggtgtgcac tgtgtttgct 5820
gacgcaaccc ccactggttg gggcattgcc accacctgtc agctcctttc cgggactttc 5880
gctttccccc tccctattgc cacggcggaa ctcatcgccg cctgccttgc ccgctgctgg 5940
acaggggctc ggctgttggg cactgacaat tccgtggtgt tgtcggggaa gctgacctcc 6000
tttccatggc tgctcgcctg tgttgccacc tggattctgc gcgggacgtc cttctgctac 6060
gtcccttcgg ccctcaatcc agcggacctt ccttcccgcg gcctgctgcc ggctctgcgg 6120
cctcttccgc gtcttcgcct tcgccctcag acgagtcgga tctccctttg ggccgcctcc 6180
ccgcctggaa ttcgagctcg gtacctttaa gaccaatgac ttacaaggca gctgtagatc 6240
ttagccactt tttaaaagaa aaggggggac tggaagggct aattcactcc caacgaagac 6300
aagatctgct ttttgcttgt actgggtctc tctggttaga ccagatctga gcctgggagc 6360
tctctggcta actagggaac ccactgctta agcctcaaga aagcttgcct tgagtgcttc 6420
aagtagtgtg tgcccgtctg ttgtgtgact ctggtaacta gagatccctc agaccctttt 6480
agtcagtgtg gaaaatctct agcagtagta gttcatgtca tcttattatt cagtatttat 6540
aacttgcaaa gaaatgaata tcagagagtg agaggaactt gtttattgca gcttataatg 6600
gttacaaata aagcaatagc atcacaaatt tcacaaataa agcatttttt tcactgcatt 6660
ctagttgtgg tttgtccaaa ctcatcaatg tatcttatca tgtctggctc tagctatccc 6720
gcccctaact ccgcccagtt ccgcccattc tccgccccat ggctgactaa ttttttttat 6780
ttatgcagag gccgaggccg cctcggcctc tgagctattc cagaagtagt gaggaggctt 6840
ttttggaggc ctaggctttt gcgtcgagac gtacccaatt cgccctatag tgagtcgtat 6900
tacgcgcgct cactggccgt cgttttacaa cgtcgtgact gggaaaaccc tggcgttacc 6960
caacttaatc gccttgcagc acatccccct ttcgccagct ggcgtaatag cgaagaggcc 7020
cgcaccgatc gcccttccca acagttgcgc agcctgaatg gcgaatggcg cgacgcgccc 7080
tgtagcggcg cattaagcgc ggcgggtgtg gtggttacgc gcagcgtgac cgctacactt 7140
gccagcgccc tagcgcccgc tcctttcgcg gcttcccttc ctttctcgcc acgttcgcc 7200
ggctttcccc gtcaagctct aaatcggggg ctccctttag ggttccgatt tagtgcttta 7260
cggcacctcg accccaaaaa acttgattag ggtgatggtt cacgtagtgg gccatcgccc 7320

tgatagacgg tttttcgccc tttgacgttg gagtccacgt tctttaatag tggactcttg 7380
ttccaaactg gaacaacact caaccctatc tcggtctatt cttttgattt ataagggatt 7440
ttgccgattt cggcctattg gttaaaaaat gagctgattt aacaaaaatt taacgcgaat 7500
tttaacaaaa tattaacgtt tacaatttcc 7530

<210> 10
<211> 8889
<212> DNA
<213> Artificial

<220>
<223> HIV-1 gag/pol (pLP1) vector

<400> 10
ttggcccatt gcatacgttg tatccatatc ataatatgta catttatatt ggctcatgtc 60
caacattacc gccatgttga cattgattat tgactagtta ttaatagtaa tcaattacgg 120
ggtcattagt tcatagccca tatatggagt tccgcgttac ataacttacg gtaaatggcc 180
cgcctggctg accgcccaac gacccccgcc cattgacgtc aataatgacg tatgttccca 240
tagtaacgcc aatagggact ttccattgac gtcaatgggt ggagtattta cggtaaactg 300
cccacttggc agtacatcaa gtgtatcata tgccaagtac gccccctatt gacgtcaatg 360
acggtaaatg gcccgcctgg cattatgccc agtacatgac cttatgggac tttcctactt 420
ggcagtacat ctacgtatta gtcatcgcta ttaccatggt gatgcggttt tggcagtaca 480
tcaatgggcg tggatagcgg tttgactcac ggggatttcc aagtctccac cccattgacg 540
tcaatgggag tttgttttgg caccaaaatc aacgggactt tccaaaatgt cgtaacaact 600
ccgccccatt gacgcaaatg ggcggtaggc gtgtacggtg ggaggtctat ataagcagag 660
ctcgtttagt gaaccgtcag atcgcctgga gacgccatcc acgctgtttt gacctccata 720
gaagacaccg ggaccgatcc agcctcccct cgaagcttac atgtggtacc gagctcggat 780
cctgagaact tcagggtgag tctatgggac ccttgatgtt ttctttcccc ttcttttcta 840
tggttaagtt catgtcatag gaaggggaga agtaacaggg tacacatatt gaccaaatca 900
gggtaatttt gcatttgtaa ttttaaaaaa tgctttcttc ttttaatata cttttttgtt 960
tatcttattt ctaatacttt ccctaatctc tttctttcag ggcaataatg atacaatgta 1020
tcatgcctct ttgcaccatt ctaaagaata acagtgataa tttctgggtt aaggcaatag 1080
caatatttct gcatataaat atttctgcat ataaattgta actgatgtaa gaggtttcat 1140
attgctaata gcagctacaa tccagctacc attctgcttt tattttatgg ttgggataag 1200
gctggattat tctgagtcca agctaggccc ttttgctaat catgttcata cctcttatct 1260
tcctcccaca gctcctgggc aacgtgctgg tctgtgtgct ggcccatcac tttggcaaag 1320
cacgtgagat ctgaattcga gatctgccgc cgccatgggt gcgagagcgt cagtattaag 1380
cgggggagaa ttagatcgat gggaaaaaat tcggttaagg ccagggggaa agaaaaaata 1440
taaattaaaa catatagtat gggcaagcag ggagctagaa cgattcgcag ttaatcctgg 1500

cctgttagaa acatcagaag gctgtagaca aatactggga cagctacaac catcccttca 1560
gacaggatca gaagaactta gatcattata taatacagta gcaaccctct attgtgtgca 1620
tcaaaggata gagataaaag acaccaagga agctttagac aagatagagg aagagcaaaa 1680
caaaagtaag aaaaaagcac agcaagcagc agctgacaca ggacacagca atcaggtcag 1740
ccaaaattac cctatagtgc agaacatcca ggggcaaatg gtacatcagg ccatatcacc 1800
tagaacttta aatgcatggg taaaagtagt agaagagaag gctttcagcc cagaagtgat 1860
acccatgttt tcagcattat cagaaggagc caccccacaa gatttaaaca ccatgctaaa 1920
cacagtgggg ggacatcaag cagccatgca aatgttaaaa gagaccatca atgaggaagc 1980
tgcagaatgg gatagagtgc atccagtgca tgcagggcct attgcaccag gccagatgag 2040
agaaccaagg ggaagtgaca tagcaggaac tactagtacc cttcaggaac aaataggatg 2100
gatgacacat aatccaccta tcccagtagg agaaatctat aaaagatgga taatcctggg 2160
attaaataaa atagtaagaa tgtatagccc taccagcatt ctggacataa gacaaggacc 2220
aaaggaaccc tttagagact atgtagaccg attctataaa actctaagag ccgagcaagc 2280
ttcacaagag gtaaaaaatt ggatgacaga aaccttgttg gtccaaaatg cgaacccaga 2340
ttgtaagact attttaaaag cattgggacc aggagcgaca ctagaagaaa tgatgacagc 2400
atgtcaggga gtggggggac ccggccataa agcaagagtt ttggctgaag caatgagcca 2460
agtaacaaat ccagctacca taatgataca gaaaggcaat tttaggaacc aaagaaagac 2520
tgttaagtgt ttcaattgtg gcaaagaagg gcacatagcc aaaaattgca gggcccctag 2580
gaaaaagggc tgttggaaat gtggaaagga aggacaccaa atgaaagatt gtactgagag 2640
acaggctaat tttttaggga agatctggcc ttcccacaag ggaaggccag ggaattttct 2700
tcagagcaga ccagagccaa cagccccacc agaagagagc ttcaggtttg gggaagagac 2760
aacaactccc tctcagaagc aggagccgat agacaaggaa ctgtatcctt tagcttccct 2820
cagatcactc tttggcagcg acccctcgtc acaataaaga taggggggca attaaaggaa 2880
gctctattag atacaggagc agatgataca gtattagaag aaatgaattt gccaggaaga 2940
tggaaaccaa aaatgatagg gggaattgga ggttttatca aagtaagaca gtatgatcag 3000
atactcatag aaatctgcgg acataaagct ataggtacag tattagtagg acctacacct 3060
gtcaacataa ttggaagaaa tctgttgact cagattggct gcactttaaa ttttcccatt 3120
agtcctattg agactgtacc agtaaaatta aagccaggaa tggatggccc aaaagttaaa 3180
caatggccat tgacagaaga aaaaataaaa gcattagtag aaatttgtac agaaatggaa 3240
aaggaaggaa aaatttcaaa aattgggcct gaaaatccat acaatactcc agtatttgcc 3300
ataaagaaaa aagacagtac taaatggaga aaattagtag atttcagaga acttaataag 3360
agaactcaag atttctggga agttcaatta ggaataccac atcctgcagg gttaaaacag 3420
aaaaaatcag taacagtact ggatgtgggc gatgcatatt tttcagttcc cttagataaa 3480
gacttcagga agtatactgc atttaccata cctagtataa acaatgagac accagggatt 3540

agatatcagt acaatgtgct tccacaggga tggaaaggat caccagcaat attccagtgt 3600
agcatgacaa aaatcttaga gccttttaga aaacaaaatc cagacatagt catctatcaa 3660
tacatggatg atttgtatgt aggatctgac ttagaaatag ggcagcatag aacaaaaata 3720
gaggaactga gacaacatct gttgaggtgg ggatttacca caccagacaa aaaacatcag 3780
aaagaacctc cattcctttg gatgggttat gaactccatc ctgataaatg gacagtacag 3840
cctatagtgc tgccagaaaa ggacagctgg actgtcaatg acatacagaa attagtggga 3900
aaattgaatt gggcaagtca gatttatgca gggattaaag taaggcaatt atgtaaactt 3960
cttaggggaa ccaaagcact aacagaagta gtaccactaa cagaagaagc agagctagaa 4020
ctggcagaaa acagggagat tctaaaagaa ccggtacatg gagtgtatta tgacccatca 4080
aaagacttaa tagcagaaat acagaagcag gggcaaggcc aatggacata tcaaatttat 4140
caagagccat ttaaaaatct gaaaacagga aagtatgcaa gaatgaaggg tgcccacact 4200
aatgatgtga aacaattaac agaggcagta caaaaaatag ccacagaaag catagtaata 4260
tggggaaaga ctcctaaatt taaattaccc atacaaaagg aaacatggga agcatggtgg 4320
acagagtatt ggcaagccac ctggattcct gagtgggagt ttgtcaatac ccctccctta 4380
gtgaagttat ggtaccagtt agagaaagaa cccataatag gagcagaaac tttctatgta 4440
gatggggcag ccaataggga aactaaatta ggaaaagcag gatatgtaac tgacagagga 4500
agacaaaaag ttgtccccct aacggacaca acaaatcaga agactgagtt acaagcaatt 4560
catctagctt tgcaggattc gggattagaa gtaaacatag tgacagactc acaatatgca 4620
ttgggaatca ttcaagcaca accagataag agtgaatcag agttagtcag tcaaataata 4680
gagcagttaa taaaaaagga aaaagtctac ctggcatggg taccagcaca caaaggaatt 4740
ggaggaaatg aacaagtaga taaattggtc agtgctggaa tcaggaaagt actattttta 4800
gatggaatag ataaggccca agaagaacat gagaaatatc acagtaattg gagagcaatg 4860
gctagtgatt ttaacctacc acctgtagta gcaaaagaaa tagtagccag ctgtgataaa 4920
tgtcagctaa aaggggaagc catgcatgga caagtagact gtagcccagg aatatggcag 4980
ctagattgta cacatttaga aggaaaagtt atcttggtag cagttcatgt agccagtgga 5040
tatatagaag cagaagtaat tccagcagag acagggcaag aaacagcata cttcctctta 5100
aaattagcag gaagatggcc agtaaaaaca gtacatacag acaatggcag caatttcacc 5160
agtactacag ttaaggccgc ctgttggtgg gcggggatca agcaggaatt tggcattccc 5220
tacaatcccc aaagtcaagg agtaatagaa tctatgaata aagaattaaa gaaaattata 5280
ggacaggtaa gagatcaggc tgaacatctt aagacagcag tacaaatggc agtattcatc 5340
cacaatttta aaagaaaagg ggggattggg gggtacagtg caggggaaag aatagtagac 5400
ataatagcaa cagacataca aactaaagaa ttacaaaaac aaattacaaa aattcaaaat 5460
tttcgggttt attacaggga cagcagagat ccagtttgga aaggaccagc aaagctcctc 5520
tggaaaggtg aaggggcagt agtaatacaa gataatagtg acataaaagt agtgccaaga 5580

agaaaagcaa agatcatcag ggattatgga aaacagatgg caggtgatga ttgtgtggca 5640
agtagacagg atgaggatta acacatggaa ttccggagcg gccgcaggag ctttgttcct 5700
tgggttcttg ggagcagcag gaagcactat gggcgcagcg tcaatgacgc tgacggtaca 5760
ggccagacaa ttattgtctg gtatagtgca gcagcagaac aatttgctga gggctattga 5820
ggcgcaacag catctgttgc aactcacagt ctggggcatc aagcagctcc aggcaagaat 5880
cctggctgtg gaaagatacc taaaggatca acagctcctg gggatttggg gttgctctgg 5940
aaaactcatt tgcaccactg ctgtgccttg gaatgctagt tggagtaata aatctctgga 6000
acagatttgg aatcacacga cctggatgga gtgggacaga gaaattaaca attacacaag 6060
cttccgcgga attcacccca ccagtgcagg ctgcctatca gaaagtggtg gctggtgtgg 6120
ctaatgccct ggcccacaag tatcactaag ctcgctttct tgctgtccaa tttctattaa 6180
aggttccttt gttccctaag tccaactact aaactggggg atattatgaa gggccttgag 6240
catctggatt ctgcctaata aaaaacattt attttcattg caatgatgta tttaaattat 6300
ttctgaatat tttactaaaa agggaatgtg ggaggtcagt gcatttaaaa cataaagaaa 6360
tgaagagcta gttcaaacct tgggaaaata cactatatct taaactccat gaaagaaggt 6420
gaggctgcaa acagctaatg cacattggca acagcccctg atgcctatgc cttattcatc 6480
cctcagaaaa ggattcaagt agaggcttga tttggaggtt aaagttttgc tatgctgtat 6540
tttacattac ttattgtttt agctgtcctc atgaatgtct tttcactacc catttgctta 6600
tcctgcatct ctcagccttg actccactca gttctcttgc ttagagatac cacctttccc 6660
ctgaagtgtt ccttccatgt tttacggcga gatggtttct cctcgcctgg ccactcagcc 6720
ttagttgtct ctgttgtctt atagaggtct acttgaagaa ggaaaaacag ggggcatggt 6780
ttgactgtcc tgtgagccct tcttccctgc ctcccccact cacagtgacc cggaatccct 6840
cgacatggca gtctagcact agtgcggccg cagatctgct tcctcgctca ctgactcgct 6900
gcgctcggtc gttcggctgc ggcgagcggt atcagctcac tcaaaggcgg taatacggtt 6960
atccacagaa tcaggggata acgcaggaaa gaacatgtga gcaaaaggcc agcaaaaggc 7020
caggaaccgt aaaaaggccg cgttgctggc gtttttccat aggctccgcc cccctgacga 7080
gcatcacaaa aatcgacgct caagtcagag gtggcgaaac ccgacaggac tataaagata 7140
ccaggcgttt ccccctggaa gctccctcgt gcgctctcct gttccgaccc tgccgcttac 7200
cggatacctg tccgcctttc tcccttcggg aagcgtggcg ctttctcata gctcacgctg 7260
taggtatctc agttcggtgt aggtcgttcg ctccaagctg ggctgtgtgc acgaaccccc 7320
cgttcagccc gaccgctgcg ccttatccgg taactatcgt cttgagtcca acccggtaag 7380
acacgactta tcgccactgg cagcagccac tggtaacagg attagcagag cgaggtatgt 7440
aggcggtgct acagagttct tgaagtggtg gcctaactac ggctacacta gaagaacagt 7500
atttggtatc tgcgctctgc tgaagccagt taccttcgga aaaagagttg gtagctcttg 7560
atccggcaaa caaaccaccg ctggtagcgg tggttttttt gtttgcaagc agcagattac 7620

gcgcagaaaa aaaggatctc aagaagatcc tttgatcttt tctacggggt ctgacgctca 7680

gtggaacgaa aactcacgtt aagggatttt ggtcatgaga ttatcaaaaa ggatcttcac 7740

ctagatcctt ttaaattaaa aatgaagttt taaatcaatc taaagtatat atgagtaaac 7800

ttggtctgac agttaccaat gcttaatcag tgaggcacct atctcagcga tctgtctatt 7860

tcgttcatcc atagttgcct gactccccgt cgtgtagata actacgatac gggagggctt 7920

accatctggc cccagtgctg caatgatacc gcgagaccca cgctcaccgg ctccagattt 7980

atcagcaata aaccagccag ccggaagggc cgagcgcaga agtggtcctg caactttatc 8040

cgcctccatc cagtctatta attgttgccg ggaagctaga gtaagtagtt cgccagttaa 8100

tagtttgcgc aacgttgttg ccattgctac aggcatcgtg gtgtcacgct cgtcgtttgg 8160

tatggcttca ttcagctccg gttcccaacg atcaaggcga gttacatgat cccccatgtt 8220

gtgcaaaaaa gcggttagct ccttcggtcc tccgatcgtt gtcagaagta agttggccgc 8280

agtgttatca ctcatggtta tggcagcact gcataattct cttactgtca tgccatccgt 8340

aagatgcttt tctgtgactg gtgagtactc aaccaagtca ttctgagaat agtgtatgcg 8400

gcgaccgagt tgctcttgcc cggcgtcaat acgggataat accgcgccac atagcagaac 8460

tttaaaagtg ctcatcattg gaaaacgttc ttcggggcga aaactctcaa ggatcttacc 8520

gctgttgaga tccagttcga tgtaacccac tcgtgcaccc aactgatctt cagcatcttt 8580

tactttcacc agcgtttctg ggtgagcaaa aacaggaagg caaaatgccg caaaaaaggg 8640

aataagggcg acacggaaat gttgaatact catactcttc ctttttcaat attattgaag 8700

catttatcag ggttattgtc tcatgagcgg atacatattt gaatgtattt agaaaaataa 8760

acaaataggg gttccgcgca catttccccg aaaagtgcca cctgacggga tcccctgagg 8820

gggcccccat gggctagagg atccggcctc ggcctctgca taaataaaaa aaattagtca 8880

gccatgagc 8889

<210> 11
<211> 5821
<212> DNA
<213> Artificial

<220>
<223> VSV-G envelope (pLP/VSVG) vector

<400> 11
ttggcccatt gcatacgttg tatccatatc ataatatgta catttatatt ggctcatgtc 60

caacattacc gccatgttga cattgattat tgactagtta ttaatagtaa tcaattacgg 120

ggtcattagt tcatagccca tatatggagt tccgcgttac ataacttacg gtaaatggcc 180

cgcctggctg accgcccaac gacccccgcc cattgacgtc aataatgacg tatgttccca 240

tagtaacgcc aatagggact ttccattgac gtcaatgggt ggagtattta cggtaaactg 300

cccacttggc agtacatcaa gtgtatcata tgccaagtac gccccctatt gacgtcaatg 360

acggtaaatg gcccgcctgg cattatgccc agtacatgac cttatgggac tttcctactt 420

ggcagtacat ctacgtatta gtcatcgcta ttaccatggt gatgcggttt tggcagtaca 480
tcaatgggcg tggatagcgg tttgactcac ggggatttcc aagtctccac cccattgacg 540
tcaatgggag tttgttttgg caccaaaatc aacgggactt tccaaaatgt cgtaacaact 600
ccgccccatt gacgcaaatg ggcggtaggc gtgtacggtg ggaggtctat ataagcagag 660
ctcgtttagt gaaccgtcag atcgcctgga gacgccatcc acgctgtttt gacctccata 720
gaagacaccg ggaccgatcc agcctcccct cgaagcttac atgtggtacc gagctcggat 780
cctgagaact tcagggtgag tctatgggac ccttgatgtt ttctttcccc ttcttttcta 840
tggttaagtt catgtcatag gaaggggaga agtaacaggg tacacatatt gaccaaatca 900
gggtaatttt gcatttgtaa ttttaaaaaa tgctttcttc ttttaatata cttttttgtt 960
tatcttattt ctaatacttt ccctaatctc tttctttcag ggcaataatg atacaatgta 1020
tcatgcctct ttgcaccatt ctaaagaata acagtgataa tttctgggtt aaggcaatag 1080
caatatttct gcatataaat atttctgcat ataaattgta actgatgtaa gaggtttcat 1140
attgctaata gcagctacaa tccagctacc attctgcttt tattttatgg ttgggataag 1200
gctggattat tctgagtcca agctaggccc ttttgctaat catgttcata cctcttatct 1260
tcctcccaca gctcctgggc aacgtgctgg tctgtgtgct ggcccatcac tttggcaaag 1320
cacgtgagat ctgaattctg acactatgaa gtgccttttg tacttagcct ttttattcat 1380
tggggtgaat tgcaagttca ccatagtttt tccacacaac caaaaaggaa actggaaaaa 1440
tgttccttct aattaccatt attgcccgtc aagctcagat ttaaattggc ataatgactt 1500
aataggcaca gccttacaag tcaaaatgcc caagagtcac aaggctattc aagcagacgg 1560
ttggatgtgt catgcttcca aatgggtcac tacttgtgat ttccgctggt atggaccgaa 1620
gtatataaca cattccatcc gatccttcac tccatctgta gaacaatgca aggaaagcat 1680
tgaacaaacg aaacaaggaa cttggctgaa tccaggcttc cctcctcaaa gttgtggata 1740
tgcaactgtg acggatgccg aagcagtgat tgtccaggtg actcctcacc atgtgctggt 1800
tgatgaatac acaggagaat gggttgattc acagttcatc aacggaaaat gcagcaatta 1860
catatgcccc actgtccata actctacaac ctggcattct gactataagg tcaaagggct 1920
atgtgattct aacctcattt ccatggacat caccttcttc tcagaggacg gagagctatc 1980
atccctggga aaggagggca cagggttcag aagtaactac tttgcttatg aaactggagg 2040
caaggcctgc aaaatgcaat actgcaagca ttggggagtc agactcccat caggtgtctg 2100
gttcgagatg gctgataagg atctctttgc tgcagccaga ttccctgaat gcccagaagg 2160
gtcaagtatc tctgctccat ctcagacctc agtggatgta agtctaattc aggacgttga 2220
gaggatcttg gattattccc tctgccaaga aacctggagc aaaatcagag cgggtcttcc 2280
aatctctcca gtggatctca gctatcttgc tcctaaaaac ccaggaaccg gtcctgcttt 2340
caccataatc aatggtaccc taaaatactt tgagaccaga tacatcagag tcgatattgc 2400
tgctccaatc ctctcaagaa tggtcggaat gatcagtgga actaccacag aaagggaact 2460

gtgggatgac tgggcaccat atgaagacgt ggaaattgga cccaatggag ttctgaggac 2520
cagttcagga tataagtttc ctttatacat gattggacat ggtatgttgg actccgatct 2580
tcatcttagc tcaaaggctc aggtgttcga acatcctcac attcaagacg ctgcttcgca 2640
acttcctgat gatgagagtt tattttttgg tgatactggg ctatccaaaa atccaatcga 2700
gcttgtagaa ggttggttca gtagttggaa aagctctatt gcctcttttt tctttatcat 2760
agggttaatc attggactat tcttggttct ccgagttggt atccatcttt gcattaaatt 2820
aaagcacacc aagaaaagac agatttatac agacatagag atgaaccgac ttggaaagta 2880
actcaaatcc tgcacaacag attcttcatg tttggaccaa atcaacttgt gataccatgc 2940
tcaaagaggc ctcaattata tttgagtttt taatttttat gaaaaaaaaa aaaaaaaacg 3000
gaattcaccc caccagtgca ggctgcctat cagaaagtgg tggctggtgt ggctaatgcc 3060
ctggcccaca agtatcacta agctcgcttt cttgctgtcc aatttctatt aaaggttcct 3120
ttgttcccta agtccaacta ctaaactggg ggatattatg aagggccttg agcatctgga 3180
ttctgcctaa taaaaaacat ttattttcat tgcaatgatg tatttaaatt atttctgaat 3240
attttactaa aaagggaatg tgggaggtca gtgcatttaa aacataaaga aatgaagagc 3300
tagttcaaac cttgggaaaa tacactatat cttaaactcc atgaaagaag gtgaggctgc 3360
aaacagctaa tgcacattgg caacagcccc tgatgcctat gccttattca tccctcagaa 3420
aaggattcaa gtagaggctt gatttggagg ttaaagtttt gctatgctgt attttacatt 3480
acttattgtt ttagctgtcc tcatgaatgt cttttcacta cccatttgct tatcctgcat 3540
ctctcagcct tgactccact cagttctctt gcttagagat accacctttc ccctgaagtg 3600
ttccttccat gttttacggc gagatggttt ctcctcgcct ggccactcag ccttagttgt 3660
ctctgttgtc ttatagaggt ctacttgaag aaggaaaaac agggggcatg gtttgactgt 3720
cctgtgagcc cttcttccct gcctccccca ctcacagtga cccggaatcc ctcgacatgg 3780
cagtctagca ctagtgcggc cgcagatctg cttcctcgct cactgactcg ctgcgctcgg 3840
tcgttcggct gcggcgagcg gtatcagctc actcaaaggc ggtaatacgg ttatccacag 3900
aatcagggga taacgcagga aagaacatgt gagcaaaagg ccagcaaaag gccaggaacc 3960
gtaaaaaggc cgcgttgctg gcgtttttcc ataggctccg cccccctgac gagcatcaca 4020
aaaatcgacg ctcaagtcag aggtggcgaa acccgacagg actataaaga taccaggcgt 4080
ttccccctgg aagctccctc gtgcgctctc ctgttccgac cctgccgctt accggatacc 4140
tgtccgcctt tctcccttcg ggaagcgtgg cgctttctca tagctcacgc tgtaggtatc 4200
tcagttcggt gtaggtcgtt cgctccaagc tgggctgtgt gcacgaaccc cccgttcagc 4260
ccgaccgctg cgccttatcc ggtaactatc gtcttgagtc caacccggta agacacgact 4320
tatcgccact ggcagcagcc actggtaaca ggattagcag agcgaggtat gtaggcggtg 4380
ctacagagtt cttgaagtgg tggcctaact acggctacac tagaagaaca gtatttggta 4440
tctgcgctct gctgaagcca gttaccttcg gaaaaagagt tggtagctct tgatccggca 4500

aacaaaccac cgctggtagc ggtggttttt ttgtttgcaa gcagcagatt acgcgcagaa 4560

aaaaaggatc tcaagaagat cctttgatct tttctacggg gtctgacgct cagtggaacg 4620

aaaactcacg ttaagggatt ttggtcatga gattatcaaa aaggatcttc acctagatcc 4680

ttttaaatta aaaatgaagt tttaaatcaa tctaaagtat atatgagtaa acttggtctg 4740

acagttacca atgcttaatc agtgaggcac ctatctcagc gatctgtcta tttcgttcat 4800

ccatagttgc ctgactcccc gtcgtgtaga taactacgat acgggagggc ttaccatctg 4860

gccccagtgc tgcaatgata ccgcgagacc cacgctcacc ggctccagat ttatcagcaa 4920

taaaccagcc agccggaagg gccgagcgca gaagtggtcc tgcaacttta tccgcctcca 4980

tccagtctat taattgttgc cgggaagcta gagtaagtag ttcgccagtt aatagtttgc 5040

gcaacgttgt tgccattgct acaggcatcg tggtgtcacg ctcgtcgttt ggtatggctt 5100

cattcagctc cggttcccaa cgatcaaggc gagttacatg atcccccatg ttgtgcaaaa 5160

aagcggttag ctccttcggt cctccgatcg ttgtcagaag taagttggcc gcagtgttat 5220

cactcatggt tatggcagca ctgcataatt ctcttactgt catgccatcc gtaagatgct 5280

tttctgtgac tggtgagtac tcaaccaagt cattctgaga atagtgtatg cggcgaccga 5340

gttgctcttg cccggcgtca atacgggata ataccgcgcc acatagcaga actttaaaag 5400

tgctcatcat tggaaaacgt tcttcggggc gaaaactctc aaggatctta ccgctgttga 5460

gatccagttc gatgtaaccc actcgtgcac ccaactgatc ttcagcatct tttactttca 5520

ccagcgtttc tgggtgagca aaaacaggaa ggcaaaatgc cgcaaaaaag ggaataaggg 5580

cgacacggaa atgttgaata ctcatactct tcctttttca atattattga agcatttatc 5640

agggttattg tctcatgagc ggatacatat ttgaatgtat ttagaaaaat aaacaaatag 5700

gggttccgcg cacatttccc cgaaaagtgc cacctgacgg gatcccctga gggggccccc 5760

atgggctaga ggatccggcc tcggcctctg cataaataaa aaaaattagt cagccatgag 5820

c 5821

<210> 12
<211> 4180
<212> DNA
<213> Artificial

<220>
<223> HIV-1 Rev protein (pLP2) vector

<400> 12
aatgtagtct tatgcaatac tcttgtagtc ttgcaacatg gtaacgatga gttagcaaca 60

tgccttacaa ggagagaaaa agcaccgtgc atgccgattg gtggaagtaa ggtggtacga 120

tcgtgcctta ttaggaaggc aacagacggg tctgacatgg attggacgaa ccactgaatt 180

ccgcattgca gagatattgt atttaagtgc ctagctcgat acaataaacg ccatttgacc 240

attcaccaca ttggtgtgca cctccaagct cgagctcgtt tagtgaaccg tcagatcgcc 300

tggagacgcc atccacgctg ttttgacctc catagaagac accgggaccg atccagcctc 360

ccctcgaagc tagtcgatta ggcatctcct atggcaggaa gaagcggaga cagcgacgaa 420
gacctcctca aggcagtcag actcatcaag tttctctatc aaagcaaccc acctcccaat 480
cccgagggga cccgacaggc ccgaaggaat agaagaagaa ggtggagaga gagacagaga 540
cagatccatt cgattagtga acggatcctt agcacttatc tgggacgatc tgcggagcct 600
gtgcctcttc agctaccacc gcttgagaga cttactcttg attgtaacga ggattgtgga 660
acttctggga cgcagggggt gggaagccct caaatattgg tggaatctcc tacaatattg 720
gagtcaggag ctaaagaata gtgctgttag cttgctcaat gccacagcta tagcagtagc 780
tgaggggaca gatagggtta tagaagtagt acaagaagct tggcactggc cgtcgtttta 840
caacgtcgtg atctgagcct gggagatctc tggctaacta gggaacccac tgcttaagcc 900
tcaataaagc ttgccttgag tgcttcaagt agtgtgtgcc cgtctgttgt gtgactctgg 960
taactagaga tcaggaaaac cctggcgtta cccaacttaa tcgccttgca gcacatcccc 1020
ctttcgccag ctggcgtaat agcgaagagg cccgcaccga tcgcccttcc caacagttgc 1080
gcagcctgaa tggcgaatgg cgcctgatgc ggtattttct ccttacgcat ctgtgcggta 1140
tttcacaccg catacgtcaa agcaaccata gtacgcgccc tgtagcggcg cattaagcgc 1200
ggcgggtgtg gtggttacgc gcagcgtgac cgctacactt gccagcgccc tagcgcccgc 1260
tcctttcgct ttcttccctt cctttctcgc cacgttcgcc ggctttcccc gtcaagctct 1320
aaatcggggg ctccctttag ggttccgatt tagtgcttta cggcacctcg accccaaaaa 1380
acttgatttg ggtgatggtt cacgtagtgg gccatcgccc tgatagacgg tttttcgccc 1440
tttgacgttg gagtccacgt tctttaatag tggactcttg ttccaaactg gaacaacact 1500
caaccctatc tcgggctatt cttttgattt ataagggatt ttgccgattt cggcctattg 1560
gttaaaaaat gagctgattt aacaaaaatt taacgcgaat tttaacaaaa tattaacgtt 1620
tacaatttta tggtgcactc tcagtacaat ctgctctgat gccgcatagt taagccagcc 1680
ccgacacccg ccaacacccg ctgacgcgcc ctgacgggct tgtctgctcc cggcatccgc 1740
ttacagacaa gctgtgaccg tctccgggag ctgcatgtgt cagaggtttt caccgtcatc 1800
accgaaacgc gcgagacgaa agggcctcgt gatacgccta tttttatagg ttaatgtcat 1860
gataataatg gtttcttaga cgtcaggtgg cacttttcgg ggaaatgtgc gcggaacccc 1920
tatttgttta tttttctaaa tacattcaaa tatgtatccg ctcatgagac aataaccctg 1980
ataaatgctt caataatatt gaaaaaggaa gagtatgagt attcaacatt tccgtgtcgc 2040
ccttattccc ttttttgcgg cattttgcct tcctgttttt gctcacccag aaacgctggt 2100
gaaagtaaaa gatgctgaag atcagttggg tgcacgagtg ggttacatcg aactggatct 2160
caacagcggt aagatccttg agagttttcg ccccgaagaa cgttttccaa tgatgagcac 2220
ttttaaagtt ctgctatgtg gcgcggtatt atcccgtatt gacgccgggc aagagcaact 2280
cggtcgccgc atacactatt ctcagaatga cttggttgag tactcaccag tcacagaaaa 2340
gcatcttacg gatggcatga cagtaagaga attatgcagt gctgccataa ccatgagtga 2400

taacactgcg gccaacttac ttctgacaac gatcggagga ccgaaggagc taaccgcttt 2460
tttgcacaac atgggggatc atgtaactcg ccttgatcgt tgggaaccgg agctgaatga 2520
agccatacca aacgacgagc gtgacaccac gatgcctgta gcaatggcaa caacgttgcg 2580
caaactatta actggcgaac tacttactct agcttcccgg caacaattaa tagactggat 2640
ggaggcggat aaagttgcag gaccacttct gcgctcggcc cttccggctg gctggtttat 2700
tgctgataaa tctggagccg gtgagcgtgg gtctcgcggt atcattgcag cactggggcc 2760
agatggtaag ccctcccgta tcgtagttat ctacacgacg gggagtcagg caactatgga 2820
tgaacgaaat agacagatcg ctgagatagg tgcctcactg attaagcatt ggtaactgtc 2880
agaccaagtt tactcatata tactttagat tgatttaaaa cttcattttt aatttaaaag 2940
gatctaggtg aagatccttt ttgataatct catgaccaaa atcccttaac gtgagttttc 3000
gttccactga gcgtcagacc ccgtagaaaa gatcaaagga tcttcttgag atcctttttt 3060
tctgcgcgta atctgctgct tgcaaacaaa aaaaccaccg ctaccagcgg tggtttgttt 3120
gccggatcaa gagctaccaa ctctttttcc gaaggtaact ggcttcagca gagcgcagat 3180
accaaatact gttcttctag tgtagccgta gttaggccac cacttcaaga actctgtagc 3240
accgcctaca tacctcgctc tgctaatcct gttaccagtg gctgctgcca gtggcgataa 3300
gtcgtgtctt accgggttgg actcaagacg atagttaccg gataaggcgc agcggtcggg 3360
ctgaacgggg ggttcgtgca cacagcccag cttggagcga acgacctaca ccgaactgag 3420
atacctacag cgtgagctat gagaaagcgc cacgcttccc gaagggagaa aggcggacag 3480
gtatccggta agcggcaggg tcggaacagg agagcgcacg agggagcttc cagggggaaa 3540
cgcctggtat ctttatagtc ctgtcgggtt tcgccacctc tgacttgagc gtcgattttt 3600
gtgatgctcg tcaggggggc ggagcctatg gaaaaacgcc agcaacgcgg cctttttacg 3660
gttcctggcc ttttgctggc cttttgctca catgttcttt cctgcgttat cccctgattc 3720
tgtggataac cgtattaccg cctttgagtg agctgatacc gctcgccgca gccgaacgac 3780
cgagcgcagc gagtcagtga gcgaggaagc ggaagagcgc ccaatacgca aaccgcctct 3840
ccccgcgcgt tggccgattc attaatgcag ctggcacgac aggtttcccg actggaaagc 3900
gggcagtgag cgcaacgcaa ttaatgtgag ttagctcact cattaggcac cccaggcttt 3960
acactttatg cttccggctc gtatgttgtg tggaattgtg agcggataac aatttcacac 4020
aggaaacagc tatgacatga ttacgaattc gatgtacggg ccagatatac gcgtatctga 4080
ggggactagg gtgtgtttag gcgaaaagcg gggcttcggt tgtacgcggt taggagtccc 4140
ctcaggatat agtagtttcg cttttgcata gggaggggga 4180

<210> 13
<211> 7384
<212> DNA
<213> Artificial

<220>
<223> pRRL-eGFP vector

<400> 13
caggtggcac ttttcgggga aatgtgcgcg gaacccctat ttgtttattt ttctaaatac 60
attcaaatat gtatccgctc atgagacaat aaccctgata aatgcttcaa taatattgaa 120
aaaggaagag tatgagtatt caacatttcc gtgtcgccct tattcccttt tttgcggcat 180
tttgccttcc tgtttttgct cacccagaaa cgctggtgaa agtaaaagat gctgaagatc 240
agttgggtgc acgagtgggt tacatcgaac tggatctcaa cagcggtaag atccttgaga 300
gttttcgccc cgaagaacgt tttccaatga tgagcacttt taaagttctg ctatgtggcg 360
cggtattatc ccgtattgac gccgggcaag agcaactcgg tcgccgcata cactattctc 420
agaatgactt ggttgagtac tcaccagtca cagaaaagca tcttacggat ggcatgacag 480
taagagaatt atgcagtgct gccataacca tgagtgataa cactgcggcc aacttacttc 540
tgacaacgat cggaggaccg aaggagctaa ccgctttttt gcacaacatg ggggatcatg 600
taactcgcct tgatcgttgg gaaccggagc tgaatgaagc cataccaaac gacgagcgtg 660
acaccacgat gcctgtagca atggcaacaa cgttgcgcaa actattaact ggcgaactac 720
ttactctagc ttcccggcaa caattaatag actggatgga ggcggataaa gttgcaggac 780
cacttctgcg ctcggccctt ccggctggct ggtttattgc tgataaatct ggagccggtg 840
agcgtgggtc tcgcggtatc attgcagcac tggggccaga tggtaagccc tcccgtatcg 900
tagttatcta cacgacgggg agtcaggcaa ctatggatga acgaaataga cagatcgctg 960
agataggtgc ctcactgatt aagcattggt aactgtcaga ccaagtttac tcatatatac 1020
tttagattga tttaaaactt catttttaat ttaaaaggat ctaggtgaag atcctttttg 1080
ataatctcat gaccaaaatc ccttaacgtg agttttcgtt ccactgagcg tcagaccccg 1140
tagaaaagat caaaggatct tcttgagatc ctttttttct gcgcgtaatc tgctgcttgc 1200
aaacaaaaaa accaccgcta ccagcggtgg tttgtttgcc ggatcaagag ctaccaactc 1260
tttttccgaa ggtaactggc ttcagcagag cgcagatacc aaatactgtc cttctagtgt 1320
agccgtagtt aggccaccac ttcaagaact ctgtagcacc gcctacatac ctcgctctgc 1380
taatcctgtt accagtggct gctgccagtg gcgataagtc gtgtcttacc gggttggact 1440
caagacgata gttaccggat aaggcgcagc ggtcgggctg aacggggggt tcgtgcacac 1500
agcccagctt ggagcgaacg acctacaccg aactgagata cctacagcgt gagctatgag 1560
aaagcgccac gcttcccgaa gggagaaagg cggacaggta tccggtaagc ggcagggtcg 1620
gaacaggaga gcgcacgagg gagcttccag ggggaaacgc ctggtatctt tatagtcctg 1680
tcgggtttcg ccacctctga cttgagcgtc gatttttgtg atgctcgtca ggggggcgga 1740
gcctatggaa aaacgccagc aacgcggcct ttttacggtt cctggccttt tgctggcctt 1800
ttgctcacat gttctttcct gcgttatccc ctgattctgt ggataaccgt attaccgcct 1860
ttgagtgagc tgataccgct cgccgcagcc gaacgaccga gcgcagcgag tcagtgagcg 1920
aggaagcgga agagcgccca atacgcaaac cgcctctccc cgcgcgttgg ccgattcatt 1980

aatgcagctg gcacgacagg tttcccgact ggaaagcggg cagtgagcgc aacgcaatta 2040
atgtgagtta gctcactcat taggcacccc aggctttaca ctttatgctt ccggctcgta 2100
tgttgtgtgg aattgtgagc ggataacaat ttcacacagg aaacagctat gaccatgatt 2160
acgccaagcg cgcaattaac cctcactaaa gggaacaaaa gctggagctg caagcttaat 2220
gtagtcttat gcaatactct tgtagtcttg caacatggta acgatgagtt agcaacatgc 2280
cttacaagga gagaaaaagc accgtgcatg ccgattggtg gaagtaaggt ggtacgatcg 2340
tgccttatta ggaaggcaac agacgggtct gacatggatt ggacgaacca ctgaattgcc 2400
gcattgcaga gatattgtat ttaagtgcct agctcgatac aataaacggg tctctctggt 2460
tagaccagat ctgagcctgg gagctctctg gctaactagg gaacccactg cttaagcctc 2520
aataaagctt gccttgagtg cttcaagtag tgtgtgcccg tctgttgtgt gactctggta 2580
actagagatc cctcagaccc ttttagtcag tgtggaaaat ctctagcagt ggcgcccgaa 2640
cagggacctg aaagcgaaag ggaaaccaga gctctctcga cgcaggactc ggcttgctga 2700
agcgcgcacg gcaagaggcg aggggcggcg actggtgagt acgccaaaaa ttttgactag 2760
cggaggctag aaggagagag atgggtgcga gagcgtcagt attaagcggg ggagaattag 2820
atcgcgatgg gaaaaaattc ggttaaggcc agggggaaag aaaaaatata aattaaaaca 2880
tatagtatgg gcaagcaggg agctagaacg attcgcagtt aatcctggcc tgttagaaac 2940
atcagaaggc tgtagacaaa tactgggaca gctacaacca tcccttcaga caggatcaga 3000
agaacttaga tcattatata atacagtagc aaccctctat tgtgtgcatc aaaggataga 3060
gataaaagac accaaggaag ctttagacaa gatagaggaa gagcaaaaca aaagtaagac 3120
caccgcacag caagcggccg ctgatcttca gacctggagg aggagatatg agggacaatt 3180
ggagaagtga attatataaa tataaagtag taaaaattga accattagga gtagcaccca 3240
ccaaggcaaa gagaagagtg gtgcagagag aaaaaagagc agtgggaata ggagctttgt 3300
tccttgggtt cttgggagca gcaggaagca ctatgggcgc agcctcaatg acgctgacgg 3360
tacaggccag acaattattg tctggtatag tgcagcagca gaacaatttg ctgagggcta 3420
ttgaggcgca acagcatctg ttgcaactca cagtctgggg catcaagcag ctccaggcaa 3480
gaatcctggc tgtggaaaga tacctaaagg atcaacagct cctggggatt tggggttgct 3540
ctggaaaact catttccacc actgctgtgc cttggaatgc tagttggagt aataaatctc 3600
tggaacagat ttggaatcac acgacctgga tggagtggga cagagaaatt aacaattaca 3660
caagcttaat acactcctta attgaagaat cgcaaaacca gcaagaaaag aatgaacaag 3720
aattattgga attagataaa tgggcaagtt tgtggaattg gtttaacata acaaattggc 3780
tgtggtatat aaaattattc ataatgatag taggaggctt ggtaggttta agaatagttt 3840
ttgctgtact ttctatagtg aatagagtta ggcagggata ttcaccatta tcgtttcaga 3900
cccacctccc aaccccgagg ggacccgaca ggcccgaagg aatagaagaa gaaggtggag 3960
agagagacag agacagatcc attcgattag tgaacggatc tcgacggtat cggttaactt 4020

ttaaaagaaa aggggggatt ggggggtaca gtgcagggga aagaatagta gacataatag 4080
caacagacat acaaactaaa gaattacaaa aacaaattac aaaaattcaa aattttatcg 4140
atcacgagac tagcctcgag aagcttgata tcgaattccc acggggttgg ggttgcgcct 4200
tttccaaggc agccctgggt ttgcgcaggg acgcggctgc tctgggcgtg gttccgggaa 4260
acgcagcggc gccgaccctg ggtctcgcac attcttcacg tccgttcgca gcgtcacccg 4320
gatcttcgcc gctacccttg tgggcccccc ggcgacgctt cctgctccgc ccctaagtcg 4380
ggaaggttcc ttgcggttcg cggcgtgccg gacgttacaa acggaagccg cacgtctcac 4440
tagtaccctc gcagacggac agcgccaggg agcaatggca gcgcgccgac cgcgatgggc 4500
tgtggccaat agcggctgct cagcggggcg cgccgagagc agcggccggg aaggggcggt 4560
gcgggaggcg gggtgtgggg cggtagtgtg ggccctgttc ctgcccgcgc ggtgttccgc 4620
attctgcaag cctccggagc gcacgtcggc agtcggctcc ctcgttgacc gaatcaccga 4680
cctctctccc cagggggatc caccggtcgc caccatggtg agcaagggcg aggagctgtt 4740
caccggggtg gtgcccatcc tggtcgagct ggacggcgac gtaaacggcc acaagttcag 4800
cgtgtccggc gagggcgagg gcgatgccac ctacggcaag ctgaccctga agttcatctg 4860
caccaccggc aagctgcccg tgccctggcc caccctcgtg accaccctga cctacggcgt 4920
gcagtgcttc agccgctacc ccgaccacat gaagcagcac gacttcttca agtccgccat 4980
gcccgaaggc tacgtccagg agcgcaccat cttcttcaag gacgacggca actacaagac 5040
ccgcgccgag gtgaagttcg agggcgacac cctggtgaac cgcatcgagc tgaagggcat 5100
cgacttcaag gaggacggca acatcctggg gcacaagctg gagtacaact acaacagcca 5160
caacgtctat atcatggccg acaagcagaa gaacggcatc aaggtgaact tcaagatccg 5220
ccacaacatc gaggacggca gcgtgcagct cgccgaccac taccagcaga acacccccat 5280
cggcgacggc cccgtgctgc tgcccgacaa ccactacctg agcacccagt ccgccctgag 5340
caaagacccc aacgagaagc gcgatcacat ggtcctgctg gagttcgtga ccgccgccgg 5400
gatcactctc ggcatggacg agctgtacaa gataagcggc cgcgtcgaca atcaacctct 5460
ggattacaaa atttgtgaaa gattgactgg tattcttaac tatgtggctc cttttacgct 5520
atgtggatac gctgctttaa tgcctttgta tcatgctatt gcttcccgta tggctttcat 5580
tttctcctcc ttgtataaat cctggttgct gtctctttat gaggagttgt ggcccgttgt 5640
caggcaacgt ggcgtggtgt gcactgtgtt tgctgacgca acccccactg gttggggcat 5700
tgccaccacc tgtcagctcc tttccgggac tttcgctttc cccctcccta ttgccacggc 5760
ggaactcatc gccgcctgcc ttgcccgctg ctggacaggg gctcggctgt tgggcactga 5820
caattccgtg gtgttgtcgg ggaagctgac ctcctttcca tggctgctcg cctgtgttgc 5880
cacctggatt ctgcgcggga cgtccttctg ctacgtccct tcggccctca atccagcgga 5940
ccttccttcc cgcggcctgc tgccggctct gcggcctctt ccgcgtcttc gccttcgccc 6000
tcagacgagt cggatctccc tttgggccgc ctccccgcct ggaattcgag ctcggtacct 6060

ttaagaccaa tgacttacaa ggcagctgta gatcttagcc actttttaaa agaaaagggg 6120
ggactggaag ggctaattca ctcccaacga agacaagatc tgctttttgc ttgtactggg 6180
tctctctggt tagaccagat ctgagcctgg gagctctctg gctaactagg gaacccactg 6240
cttaagcctc aagaaagctt gccttgagtg cttcaagtag tgtgtgcccg tctgttgtgt 6300
gactctggta actagagatc cctcagaccc ttttagtcag tgtggaaaat ctctagcagt 6360
agtagttcat gtcatcttat tattcagtat ttataacttg caaagaaatg aatatcagag 6420
agtgagagga acttgtttat tgcagcttat aatggttaca aataaagcaa tagcatcaca 6480
aatttcacaa ataaagcatt tttttcactg cattctagtt gtggtttgtc caaactcatc 6540
aatgtatctt atcatgtctg gctctagcta tcccgcccct aactccgccc agttccgccc 6600
attctccgcc ccatggctga ctaatttttt ttatttatgc agaggccgag gccgcctcgg 6660
cctctgagct attccagaag tagtgaggag gcttttttgg aggcctaggc ttttgcgtcg 6720
agacgtaccc aattcgccct atagtgagtc gtattacgcg cgctcactgg ccgtcgtttt 6780
acaacgtcgt gactgggaaa accctggcgt tacccaactt aatcgccttg cagcacatcc 6840
ccctttcgcc agctggcgta atagcgaaga ggcccgcacc gatcgccctt cccaacagtt 6900
gcgcagcctg aatggcgaat ggcgcgacgc gccctgtagc ggcgcattaa gcgcggcggg 6960
tgtggtggtt acgcgcagcg tgaccgctac acttgccagc gccctagcgc ccgctccttt 7020
cgcgggcttc ccttcctttc tcgccacgtt cgccggcttt ccccgtcaag ctctaaatcg 7080
ggggctccct ttagggttcc gatttagtgc tttacggcac ctcgacccca aaaaacttga 7140
ttagggtgat ggttcacgta gtgggccatc gccctgatag acggtttttc gccctttgac 7200
gttggagtcc acgttcttta atagtggact cttgttccaa actggaacaa cactcaaccc 7260
tatctcggtc tattcttttg atttataagg gattttgccg atttcggcct attggttaaa 7320
aaatgagctg atttaacaaa aatttaacgc gaattttaac aaaatattaa cgtttacaat 7380
ttcc 7384

<210> 14
<211> 324
<212> PRT
<213> Artificial

<220>
<223> GPI-ScFv peptide sequence

<400> 14

Met Glu Thr Lys His Leu Trp Phe Phe Leu Leu Leu Val Ala Ala Pro
1               5                   10                  15

Arg Trp Val Leu Ser Gln Val Gln Leu Gln Gln Trp Gly Ala Gly Leu
            20                  25                  30

Leu Lys Pro Ser Glu Thr Leu Ser Leu Thr Cys Ala Val Tyr Gly Asp
        35                  40                  45

Ser Phe Gly Gly His His Trp Ser Trp Ile Arg Gln Pro Pro Gly Lys
    50                  55                  60

Gly Leu Glu Trp Ile Gly Glu Ile Asn Phe Leu Gly Asn Thr Asn Tyr
65                  70                  75                  80

Asn Pro Ser Leu Lys Ser Arg Val Ala Ile Ser Lys Asp Thr Ser Lys
                85                  90                  95

Asn Gln Phe Ser Leu Thr Leu Asn Ser Ala Thr Ala Ala Asp Thr Ala
            100                 105                 110

Val Tyr Phe Cys Ala Arg Arg Glu Thr Met Glu Thr Ala Ser Arg Lys
        115                 120                 125

Arg Ala Phe Asp Ile Trp Gly Gln Gly Thr Met Glu Thr Val Thr Val
    130                 135                 140

Ser Ala Gly Gly Gly Gly Ser Gly Gly Gly Gly Ser Gly Gly Gly Gly
145                 150                 155                 160

Ser Ala Glu Leu Met Glu Thr Thr Gln Ser Pro Pro Thr Leu Ser Ala
                165                 170                 175

Ser Val Gly Asp Arg Val Thr Ile Thr Cys Arg Ala Ser Gln Ser Leu
            180                 185                 190

Ser Gly Trp Val Ala Trp Tyr Gln Gln Lys Pro Gly Lys Ala Pro Lys
        195                 200                 205

Leu Leu Ile Tyr Lys Ala Ser Thr Leu Glu Ser Gly Val Pro Ser Arg
    210                 215                 220

Phe Ser Gly Ser Gly Ser Gly Thr Val Phe Ala Leu Thr Ile Ser Ser
225                 230                 235                 240

Leu Gln Pro Asp Asp Ser Ala Thr Tyr Tyr Cys Gln Gln His Ser Ala
                245                 250                 255

Phe Ser Ser Thr Phe Gly Gln Gly Thr Lys Leu Glu Ile Lys Arg Thr
            260                 265                 270

Pro Leu Gly Asp Thr Thr His Thr Ser Gly His His His His His His
        275                 280                 285

Gly Ser Gly Thr Thr Ser Gly Thr Thr Arg Leu Leu Ser Gly His Thr
    290                 295                 300

Cys Phe Thr Leu Thr Gly Leu Leu Gly Thr Leu Val Thr Met Glu Thr
305                 310                 315                 320

Gly Leu Leu Thr

<210> 15
<211> 6
<212> PRT
<213> Artificial

<220>
<223> His Peptide tag

<400> 15

His His His His His His
1               5

<210> 16
<211> 10
<212> PRT
<213> Artificial

<220>
<223> c-MYC peptide tag

<400> 16

Glu Gln Lys Leu Ile Ser Glu Glu Asp Leu
1               5                   10

<210> 17
<211> 9
<212> PRT
<213> Artificial

<220>
<223> HA peptide tag

<400> 17

Tyr Pro Tyr Asp Val Pro Asp Tyr Ala
1               5

<210> 18
<211> 11
<212> PRT
<213> Artificial

<220>
<223> VSV-G peptide tag

<400> 18

Tyr Thr Asp Ile Glu Met Asn Arg Leu Gly Lys
1               5                   10

<210> 19
<211> 11
<212> PRT
<213> Artificial

<220>
<223> HSV peptide tag

<400>  19

Gln Pro Glu Leu Ala Pro Glu Asp Pro Glu Asp
1               5                   10


<210>  20
<211>  14
<212>  PRT
<213>  Artificial

<220>
<223>  V5 peptide tag

<400>  20

Gly Lys Pro Ile Pro Asn Pro Leu Leu Gly Leu Asp Ser Thr
1               5                   10


<210>  21
<211>  8
<212>  PRT
<213>  Artificial

<220>
<223>  FLAG peptide tag

<400>  21

Asp Tyr Lys Asp Asp Asp Asp Lys
1               5

## Description

The current invention relates to a novel type of anti-viral peptide which comprises an anti-viral antibody or an active fragment thereof and a lipid attachment signal. Specifically the invention relates to anti-viral peptides comprising single chain variable fragments (scFv) of anti-viral antibodies operatively connected to a lipid attachment signal.

Many enveloped viruses, such as Human immunodeficiency virus (HIV), avian flu, Severe Acute Respiratory Syndrome (SARS) and Ebola are the cause of serious and ongoing epidemiological problems throughout the world. Many examples of these enveloped viruses have also proven to be extremely difficult targets for known systems of creating prophylactic or acute treatments in the form of vaccines or medicaments. In particular after several decades of intensive research an effective vaccine or cure for HIV infection remains at the present time undiscovered.

HIV is a retrovirus that can lead to acquired immunodeficiency syndrome (AIDS), a condition in humans in which the immune system begins to fail, leading to life-threatening opportunistic infections. During cellular infection by Human immunodeficiency virus type 1 (HIV-1), HIV-1 spike which is a trimeric complex of gp 120 and gp41 heterodimers is important for host cell entry. By so doing it facilitates delivery of the viral genetic material into the host cell.

gp120 is a surface attachment protein, interacts with host cell surface receptors and co-receptors and determines viral tropism. The term viral tropism refers to which cell types HIV infects, HIV can infect a variety of immune cells such as CD4⁺ T cells, macrophages, and microglial cells: HIV-1 entry to macrophages and CD4⁺ T cells is mediated through interaction of the virion envelope glycoproteins (gp120) with the CD4 molecule on the target cell and also with chemokine coreceptors.

gp41, a membrane-spinning protein, mediates the fusion process between the virus membrane of HIV and the target cell membrane. By so doing, it facilitates the formation of fusion pores in the target cell membrane.

gp120 and gp41 are initially produced as a single glycoprotein precursor gp160, which is then cleaved by a cellular protease. On the virion surface these glycoproteins are non-covalently linked. Both gp120 and gp41 go through conformational changes following virus attachment leading to the formation of a fusion pore (1).

In addition to the involvement of gp41 in membrane fusion, it has also been found to contain a set of hydrophobic heptad sequence repeats for oligomerisation, to have calcium binding capability, to interact with HIV-1 p17 (matrix protein) through its cytoplasmic tail during the virus-budding process and to be involved in the pathogenesis of AIDS dementia by elevating immunogenic nitric oxide synthase.

gp 120 and gp41 are targets for antibody responses during natural HIV infection and have also been targets for attempts at developing vaccines to prevent HIV infection.

Neutralizing antibodies block viral entry by, amongst other mechanisms, recognizing epitopes on the envelope spike critical for its attachment, interaction with cellular receptors and co-receptors or the virion/cell fusion process. However, antibodies that can neutralize a broad range of primary isolates of HIV-1 have been extremely difficult to generate, due to the well documented very high levels of genetic diversity of HIV, heavy glycosylation of envelope proteins as well as inducible nature of some of epitopes.

Despite almost two decades of effort, only four such antibodies have been identified (2-9). They include antibodies 2F5 and 4E10 that are directed against the membrane proximate region of gp41 (2-5); antibody 2G12 that recognizes cluster *alpha*1 to 2 linked mannose residues on the distal ends of oligomannose sugar located on the carbohydrate-covered silent face of gp120 (6-8); and antibody b12 that interacts with the epitope that overlaps with CD4 binding site of gp120 (9). Unfortunately, antibodies with such broadly neutralizing activity occur rarely in patients and attempts to induce such broadly neutralizing antibody responses by immunization have so far been unsuccessful (10, 11).

Another class of anti-viral antibodies are called non-neutralizing antibodies. These are antibodies which recognize specific epitopes of the virus but are unable to prevent cellular infection due to a number of reasons. In particular several non-neutralizing antibodies recognize epitopes which are only transiently expressed by the virion during specific stages in its life cycle limiting the window of opportunity in which the antibody can recognize and adhere to the virion.

In a series of previous experiments, the Inventors genetically linked a single-chain variable fragment (scFv) of a human non-neutralizing anti-gp41 monoclonal antibody, termed TG15, (13) to the human IgG3 hinge region and the transmembrane domain of type I interferon receptor subunit 1. The fusion gene was transduced into HIV-1-susceptible cells by way of a MMLV vector. It was found that expressing the scFv of this non-neutralizing antibody on the cell surface markedly inhibited the replication of HIV-1 from various subtypes, as well as inhibiting HIV-1 envelope-mediated cell-cell fusion and HIV-1 transmission from dendritic cells (DCs) to CD4⁺ T cells (14, 15). Thus, the Inventors demonstrated for the first time that a non-neutralizing antibody, when expressed as a soluble protein, does not block HIV entry. However, when expressed on the surface of HIV-1 susceptible cells, it can act as a broad, potent neutralizing antibody. Thus they named it a membrane-bound antibody (14).

Several problems exist with this prior art method of presenting antibody molecules or fragments thereof upon a cell membrane using the transmembrane domain of type I interferon receptor subunit 1; firstly the creation of a functional hybrid comprising an active scFv and a transmembrane domain is difficult, in particular the transmembrane domain comprises several hydrophobic residues which most normally form hydrophobic alpha-helices which are ultimately embedded within the lipid bilayer. Such a collection of hydrophobic residues can also make the effective expression and intracellular transportation of a product difficult. Secondly it is also very difficult to purify such transmembrane domain/scFv peptides from the cellular membranes as disruption of the membrane will invariably lead to the conformational alteration of the transmembrane domain making it impossible to reintroduce such purified peptides into the cell membrane of another target cell.

The Inventors seeing these disadvantages with the prior art set out to improve existing materials and techniques to present antibody molecules upon the exterior of a cellular membrane.

Therefore the current invention relates to an anti-viral peptide comprising an anti-enveloped virus antibody or an active fragment thereof and a lipid attachment signal, wherein said lipid attachment signal and said antibody are arranged in a C' terminal to N' terminal orientation in this anti-viral peptide.

In the current application, when the antibody (or a fragment thereof) and lipid attachment signal components of the claimed anti-viral peptide are described as being in a C' terminal to N' terminal orientation, this means that the lipid attachment signal is located in a portion of the anti-viral peptide closer to its C' terminus than the antibody (or a fragment thereof). Conversely the antibody (or a fragment thereof) is located in a portion of the anti-viral peptide closer to its N' terminus than the lipid attachment signal.

The anti-viral peptide can comprise just the lipid attachment signal and antibody (or a fragment thereof), in which case they will form the C' terminus and N' terminus respectively. Or alternatively, the anti-viral peptide can comprise additional components, as specified below, which may be present at the C' terminus and/or N' terminus and hence the lipid attachment signal and antibody (or a fragment thereof) may be comprised within the peptide but maintaining there respective C' / N' terminus orientation.

In the current application, enveloped virus refers to any virus which comprises a lipid membrane covering of the whole or a part of its protein capsid. Examples of enveloped viruses include HIV, Ebola, SARS, avian flu, Influenza.

In the current application, an anti-enveloped virus antibody or an active fragment thereof refers to a monoclonal immunoglobulin molecule or a natural or artificial fragment thereof which recognizes and binds to at least one epitope presented by an enveloped virus. The antibody can have been artificially generated *in vivo or in vitro* or alternatively can have been isolated from a natural source such as a patient currently or previously infected with the envelop virus.

Preferably the antibody is from a human source or alternatively it has been humanized. Humanized antibodies can be produced by merging the DNA that encodes the binding portion of a monoclonal non-human antibody with human antibody-producing DNA. Such humanized antibodies do not elicit an immune reaction in humans.

The Inventors have unexpectedly found that a new type of anti-viral peptide comprising a suitable anti-enveloped virus antibody linked to a lipid attachment signal is very efficiently presented upon the cell membrane of a cell expressing such a new type of anti-viral peptide or when cells are exposed to such this new type of anti-viral peptide in a purified form and that such cells are resistant to the virus against which the antibody was raised.

The Inventors have also unexpectedly found that such a new type of anti-viral peptide is expressed an order of magnitude more readily than prior methods to present anti-viral antibodies allow and further that such expressed peptides can be purified from a first population of cells expressing these and transplanted or painted onto a second population of cells thereby conferring anti virus immunity to this second population of cells.

As explained above, previous workers in this field, including the inventors (14), believed that to place an antibody (or a fragment thereof) in a cell membrane, such that it would interact and bind with its target efficiently, that it would be necessary to use one or more transmembrane domains fused to the antibody. This thinkning was because of the inherent complexity and dynamic nature of the cell membrane as well as the fact that the antibody must retain its correct structure in order to function properly.

The finding by the inventors that a new class of simpler membrane bound anti-viral proteins which comprise only an anti-enveloped virus antibody or an active fragment thereof and a lipid attachment signal, can be expressed an order of magnitude more easily than those which have previously been generated and most importantly which can be used as an agent to confer immunity to an initial cell population which expresses these anti-viral peptides and also to other cells by way of an inter-cellular transplantation method, was unexpected.

This result was unexpected because of the differences in function and composition of a peptide comprising a transmembrane domain versus lipidated peptides. A transmembrane domain is a three-dimensional protein structure which is thermodynamically stable in a membrane. The transmembrane domain may comprise a single alpha helix or a complex of several transmembrane alpha helices, a transmembrane beta barrel, a beta-helix of gramicidin A or many other structures. Transmembrane domains are usually about 20 amino acids in length. Transmembrane domains based upon their "transmembrane topology" (that is which parts of it protrude into the cell, what parts protrude out, and how many times the protein chain crosses the membrane) allow a worker to predict accurately the positioning of a chimeric portion which has been fused with the transmembrane domain. This predictability is the reason why when molecules with complex tertiary structures such as proteins are required to be positioned upon the outer surface of a cellular membrane, they are normally fused with a transmembrane domain.

Conversely, a lipid attachment signal such as the GPI anchor replacement signal of decay accelerating factor (DAF), is merely an intracellular signal which causes a specific lipid to be attached to one or more of the residues in the lipid attachment signal. The resulting lipidated peptide is not specifically located in the cell membrane or structurally held in a particular position or orientation.

The inventors have shown through their work with several different antibodies and specifically scFv of these antibodies, that this new class of anti-viral peptides, comprising a lipid attachment signal and an anti membrane virus antibody, are positioned in the cell membrane in a functional way and can protect a cell comprising such anti-viral peptides from infection by the membrane virus which the antibody recognizes.

In particular the antibody is neutralizing.

Alternatively the antibody is non-neutralizing.

In particular the antibody recognizes an epitope in the prehairpin structure of said enveloped virus; preferably the antibodies recognize epitopes located in the second heptad repeat of HIV-1 g41.

In particular the antibody is selected from the group consisting of:
an intact Immunoglobulin molecule;
a monovalent antibody fragment, such as a fab fragment or scFv;
a multivalent antibody fragment, such as a Fab₂, a Fab₃, a Bis-scFv, a minibody, a diabody, a triabody, a tetrabody or an isolated V_{H} domain.

Intact antibodies (IgG, IgM, IgA, IgE) are highly specific targeting reagents and provide humans with a key defense mechanism against pathogenic organisms and toxins. IgG, the main serum antibody and the intact format almost exclusively used in therapeutic antibodies, is a Y-shaped, multidomain protein with antigen-binding sites located on the two Fab tips and recruitment of effector functions mediated by the stem Fc domain.

IgG antibodies are bivalent and the ability to bind to two antigens greatly increases their functional affinity and confers high retention times (also called avidity) on many cell-surface receptors and polyvalent antigens. The Fc domain recruits cytotoxic effector functions through complement and/or through interactions with *gamma*-Fc receptors (Fc receptors for gamma globulins) and can provide long serum half-lives (>10 days) through interaction with the neonatal Fc receptor (FcRn), which acts as a salvage receptor (binding and transporting IgGs in intact form both within and across cells and rescuing them from a default degradative pathway).

There is a range of applications, however, in which the Fc-mediated effects are not required and are even undesirable. For example, a long serum half-life results in poor contrast in imaging applications, and inappropriate activation of Fc receptor-expressing cells can lead to massive cytokine release and associated toxic effects. To remove the Fc domain (and associated effects), IgGs have been dissected into constituent domains, initially through proteolysis (with such enzymes as papain and pepsin) and later genetically engineered into either monovalent (Fab, scFv, single variable V_{H} and V_{L} domains) or bivalent fragments (Fab'₂, diabodies, minibodies, etc).

Single-chain variable fragments (also known as Single-chain FVs or scFV) are a format in which the V_{H} and V_{L} domains are joined with a flexible polypeptide linker preventing dissociation. Antibody Fab and scFv fragments, comprising both V_{H} and V_{L} domains, usually retain the specific, monovalent, antigen-binding affinity of the parent IgG, while showing improved pharmacokinetics for tissue penetration.

Fab and scFv fragments have also been engineered into dimeric, trimeric or tetrameric conjugates using either chemical or genetic cross-links. For example, Fab fragments have been chemically cross-linked into di- and trivalent multimers, which have improved retention and internalization properties as compared with the parent IgG. Of the various strategies to genetically encode multimeric scFv, the most successful design has been the reduction of scFv linker length to between zero and five residues, which directs self-assembly into either bivalent dimers (diabodies, 55 kDa), trivalent trimers (triabodies) (80 kDa), or tetravalent tetrabodies (110 kDa).

With all the above antibody molecules or fragments, the Inventors consider humanized forms of these to be most preferable when implementing the current invention. One problem in medical application of antibodies is that the standard procedure of producing monoclonal antibodies yields non-human, normally murine, antibodies. Although murine antibodies are very similar to human ones there are differences. The human immune system hence recognizes mouse antibodies as foreign, rapidly removing them from circulation and causing systemic inflammatory effects.

A solution to this problem would be to generate human antibodies directly from humans. However, this is not generally done due to ethical considerations.

Various approaches using recombinant DNA technology to overcome this problem have been tried since the late 1980s. In one approach, the DNA that encodes the binding portion of for example a monoclonal mouse antibody is merged with human antibody encoding DNA sequence. Using a mammalian cell culture transformed so as to express this DNA fusion construct and produce these half-mouse and half-human antibodies. Such antibodies being called humanized antibodies. Another approach involves mice genetically engineered to produce more human-like antibodies.

Native antibodies are also suitable for the current invention, these being non-humanized versions of murine or another animal antibody.

In a preferred embodiment of this new type of anti-viral peptide, the antibody and the lipid attachment signal are separated by a hinge region from an immunoglobulin.

By linking the antibody and lipid attachment signal portions of the peptide via a hinge region, this allows the antibody portion of the peptide a greater freedom of movement so increasing the chances of it recognizing its epitope.

In particular, the hinge region is selected from the group: IgG1, IgG2, IgG3, IgG4.

The hinge region of IgG1 encompasses 15 amino acid residues and since it is freely flexible. IgG2 has a shorter hinge than IgG1, with 12 amino acid residues and four disulfide bridges. IgG3 differs from the other subclasses by its unique extended hinge region (about four times as long as the IgG1 hinge), containing 62 amino acids (including 21 prolines and 11 cysteines), forming an inflexible poly-proline double helix. In IgG3 the Fab fragments are relatively far away from the Fc fragment, giving the molecule a greater flexibility. The elongated hinge in IgG3 is also responsible for its higher molecular weight compared to the other subclasses. The hinge region of IgG4 is shorter than that of IgG1 and its flexibility is intermediate between that of IgG 1 and IgG2.

In particular, the hinge region comprises SEQ ID NO: 1.

SEQ ID NO: 1 corresponds to a fragment of the IgG3 hinge which has been found to be suitable for use in the current invention.

In particular the lipid attachment signal is selected from the group consisting of a peptide lipid attachment signal and a chemical attachment moiety.

In the present invention a peptide lipid attachment signal is a peptide sequence which *in vivo* or *ex vivo* can be attached to a lipid either spontaneously, via the action of an enzyme or through a chemical reaction.

In the present invention a chemical attachment moiety is a chemical group or groups, which are either naturally present on a peptide or specifically upon an amino acid residue comprised in said peptide or which has been attached thereto or has been created by means of a chemical reaction thereon.

According to a preferred embodiment the peptide lipid attachment signal is configured to link said peptide to glycosyl-phosphatidylinositol (GPI).

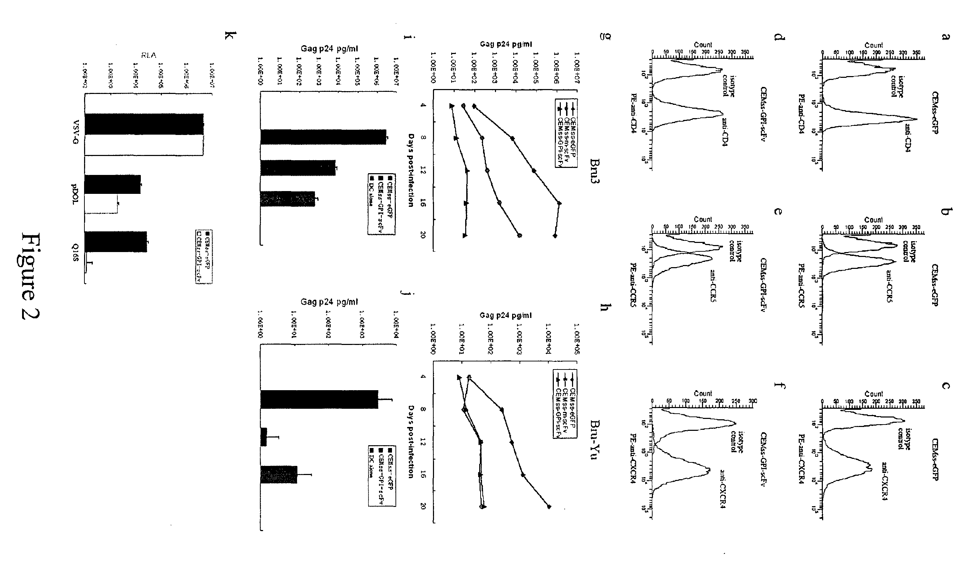

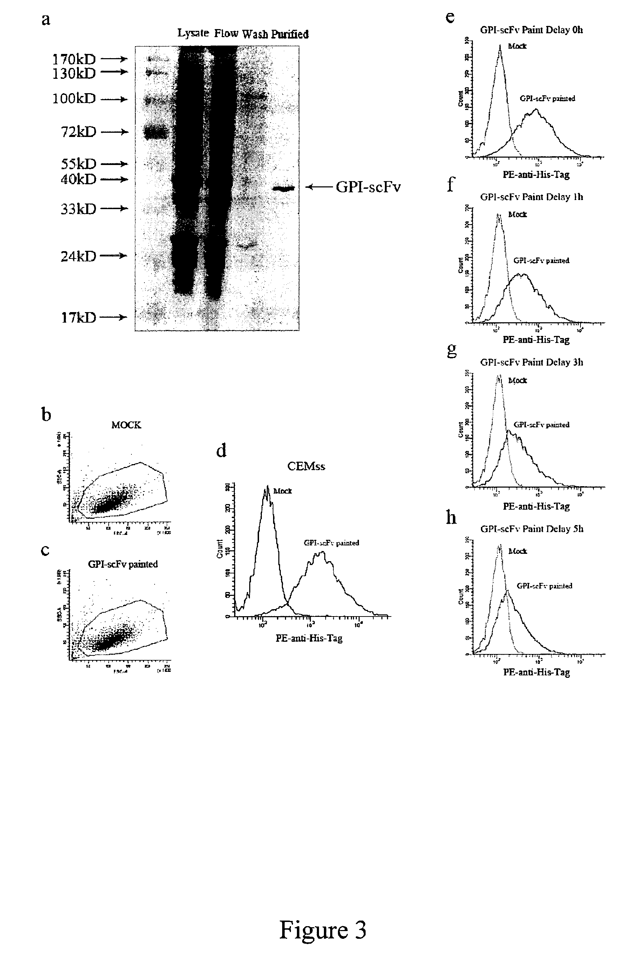

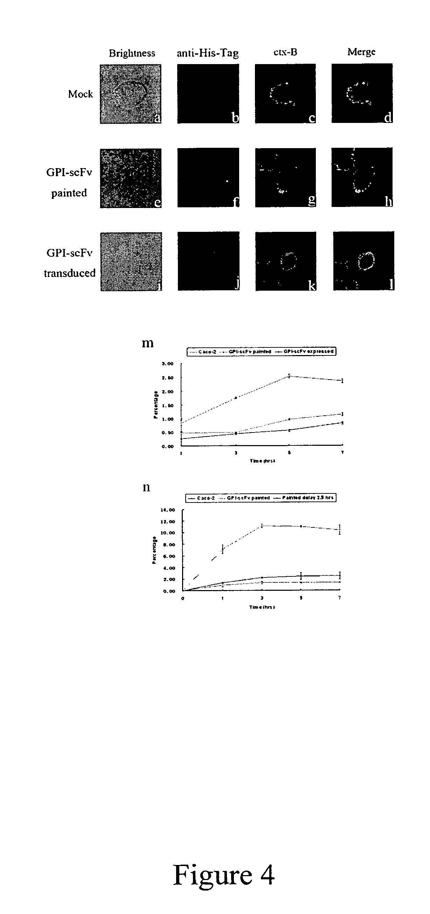

The Inventors have surprisingly found that by linking an anti-viral antibody or an active fragment thereof to a lipid attachment signal such as that corresponding to the GPI attachment signal, and so causing the anchoring of such a new type of anti-viral protein upon the exterior of a cell member; that this renders such cells resistant to both cell-free, DC-captured and transferred HIV-1 (Fig. 2g-j). Moreover, they have for the first time demonstrated that this new type of anti-viral peptide can be purified from transduced cells and "painted" onto new cell targets (Fig. 3). Finally, they have demonstrated that transduced and "painted" anti-viral peptides are anchored onto the lipid raft of apical membrane of an epithelial monolayer and block transcytosis of HIV-1 (Fig. 4). Thus, anchoring an anti-HIV antibody onto the lipid raft of plasma membrane significantly blocks all three modes of HIV-1 transmission: cell-free, DC-captured and transferred, and transcytosis.

In particular the lipid attachment signal comprises SEQ ID NO: 2.

SEQ ID NO: 2 corresponding to the GPI anchor replacement signal of decay accelerating factor (DAF).

According to another preferred embodiment the chemical attachment moiety is a thioester group.

Thioesters are compounds resulting from the bonding of sulfur with an acyl group with the general formula R1-S-CO-R2, wherein R1 and R2 are any chemical group.

In particular said thioester group is present upon the C' terminal residue of said anti-virus peptide.

In particular the peptide may further comprise a detectable tag.

Several short peptide tags are known in the art, these short peptide tags work by providing a specific antigenic determinant (epitope) in the tagged peptide that can be recognized by specific antibodies to the tag. Examples of tags encompassed by the present invention include HIS (peptide sequence HHHHHH (SEQ ID NO: 15)), c-MYC (Peptide Sequence EQKLISEEDL (SEQ ID NO: 16)), HA (Peptide Sequence YPYDVPDYA (SEQ ID NO: 17)), VSV-G (Peptide Sequence YTDIEMNRLGK (SEQ ID NO: 18)), HSV (Peptide Sequence QPELAPEDPED (SEQ ID NO: 19)), V5 (Peptide Sequence GKPIPNPLLGLDST (SEQ ID NO: 20)), FLAG (Peptide Sequence DYKDDDDK (SEQ ID NO: 21)).

In particular the detectable tag may comprise a multiple hisitidine residue sequence, such as a six Histidine tag (SEQ ID NO: 15).

The use of a histidine tag allows the easy visualization of a labeled peptide *in vivo or in vitro* using commonly available anti-his antibodies and other reagents. Such a tag can also be used to purify tagged peptides using techniques such as affinity purification.

The current invention also relates to a nucleic acid molecule encoding an open reading frame for any one of the anti-viral peptides according to the current invention.

The current invention also relates to a vector comprising a nucleic acid molecule according to the current invention.

In particular the vector is a retrovirus.

In particular the vector is a lentiviral vector.

The current invention also relates to a lipidated anti-viral peptide comprising an anti-enveloped virus antibody or an active fragment thereof and a lipid attachment signal and a lipid attached thereto, wherein said lipid attachment signal and said antibody are arranged in a C' terminal to N' terminal orientation in said peptide.

In particular the lipidated anti-viral peptide may comprise a lipid in the range C₁₂ to C₂₄,preferably in the range C₁₂ to C₁₈.

Chemically, lipids can be described as long-chain monocarboxylic acids the saturated examples of which have a general structure of CH₃(CH₂)ₙCOOH. The length of the chain usually ranges from C₁₂ to C₂₄, always with an even number of carbon atoms.

In particular the lipid may be saturated or unsaturated.

When the carbon chain contains no double bonds, it is a saturated chain. If it contains one or more such bonds, it is unsaturated. Furthermore, unsaturated lipids can occur either in cis or trans geometric isomers. In naturally occurring lipids, the double bonds are in the cis-configuration.

In particular the lipidated anti-viral peptide is purified from one or more cells expressing said lipidated anti-viral peptide.

In particular the lipid is glycosyl-phosphatidylinositol.

In particular the anti-viral peptide component of the lipidated peptide may comprise any of the anti-viral peptides according to the current invention.

The current invention also relates to a host cell, characterized in that it comprises an anti-viral peptide or a lipidated anti-viral peptide according to the current invention.

The current invention also relates to a host cell, characterized in that it is modified by the polynucleotide or the vector according to the current invention.

The current invention also relates to a composition for the modulation or treatment of a viral infection comprising a lipidated anti-viral peptide according to the current invention in an administrable form with one or more pharmaceutically acceptable carriers or excipients.

The current invention also relates to the use of an effective amount of a composition comprising a lipodated anti-viral polypeptide according to the current invention and one or more pharmaceutically acceptable carriers or excipients, for the preparation of a medicament for modulating or treating a viral infection in a subject in need thereof.

The current invention also relates to a method of purifying a lipidated anti-viral peptide, comprising the steps:
introducing at least one coding sequence for said lipidated anti-viral peptide into at least one cell;
expressing said at least one coding sequence in said at least one cell;
inducing lysis of said at least one cell;
centrifuging the resulting lysate;
passing the resulting supernatant over an affinity purification column;
eluting said column and collecting protein containing fractions.

The current invention also relates to a method of treating at least one cell with a lipidated anti-viral peptide, comprising the steps:
the at least one cell is washed in buffer comprising detergent;
the at least one cell is then suspended in buffer comprising detergent and said lipidated anti-viral peptide and allowed to incubate;
the at least one cell is washed in buffer comprising detergent.

For a better understanding of the invention and to show how the same may be carried into effect, there will now be shown by way of example only, specific embodiments, methods and processes according to the present invention with reference to the accompanying drawings in which:
Figure 1:
   *a*. Schematic diagram of the lentiviral vectors pRRL-scFv/hinge/histag/DAF of the current invention and pRRL-m-scFv (14).
   *b*. FACS analysis of cell surface expression of m-scFv in m-scFv-transduced CEMss cells vs. parental CEMss control with or without PI-PLC treatment.
   *c*. FACS analysis of cell surface expression of scFv/hinge/histag/DAF in scFv/hinge/histag/DAF-transduced CEMss cells vs. parental CEMss control with or without PI-PLC treatment.
Figure 2. *a-f.* Cell surface expression of CD4, CXCR4 and CCR5 of a representative CEMss-eGFP cell line (*a-c*) and of a representative CEMss-GPI-scFv cell line (*d*-*f*). p24 activity in pooled CEMss-eGFP, CEMss-m-scFv, and CEMss-GPI-scFv cell lines infected with HIV-1 strain Bru-3
   (*g*) and Bru-Yu
   (*h*). p24 activity detected in supernatants of CEMss-eGFP or CEMss-GPI-scFv co-cultured with human DC-captured HIV-1 NL4-3
   (*i*) or primary isolate #A
   (*j*). Relative luciferase activity (RLA) in CEMss-eGFP or CEMss-GPI-scFv cells transduced with lentiviral vectors expressing HIV-1 envelope pDOL or Q168 or VSV-G envelope (ik).
Figure 3.
   *a*. Silver stain of 12% SDS/PAGE of cell lysate, flow through, wash, and elution (after the samples were combined, dialyzed and filtered).
   *b*. Forward and side scatters of mock "painted" CEMss stained with anti-his-tag antibody.
   *c*. Forward and side scatters of GPI-scFv-"painted" CEMss stained with anti-his-tag antibody.
   *d*. Detection of reintegrated GPI-scFv on the cell surface between mock and GPI-scFv"painted" CEMss cells with anti-his-tag antibody.
   *e*-*h*. Detection of turnover of reintegrated GPI-scFv on the cell surface at 0 (*e*), 1 (*f*), 3 (*g*), and 5 (*h*) hours post "paint".
Figure 4. *a*-*l*. Confocal analysis of "painted" or transduced GPI-scFv and lipid raft marker GM1 on parental Caco2 cells (*a-d*), GPI-scFv-"painted" Caco2 cells (*e-h*) and GPI-scFv-transduced Caco2 cells (*i-l*) stained with mouse anti-his-tag Ab followed by Cy3-conjugated goat anti-mouse IgG Ab and further incubated with biotinylated cholera toxin B subunit (Ctx B) followed by FITC-conjugated streptavidin.
   *m*. Time course of HIV-1 transcytosis across the human epithelial monolayer with or without "painted" or transduced with GPI-scFv.
   *n*. Time course of HIV-1 transcytosis across the human epithelial monolayer with or without "painted" with GPI-scFv followed by viral challenge right away or delayed for 2.5 hours (the estimated half-life of reintegrated GPI-scFv on the cell surface).
Figure 5. shows the susceptibility of GPI-scFv-transduced Maji-CCR5/CXCR4 cells to various HIV-1 envelope pseudotypes and VSV-G control measured by relative luciferase activity.
Figure 6. shows the effect of GPI-scFvs on infection of wild type HIV-1 (Bru-3 subtype B X4; Bru-Yu subtype B R5; and MJ4 subtype C R5) measured by relative luciferase assay (a) and β-gal staining (b). Compared to mock and negative GPI-scFv control (AB65).
Figure 7. shows a western blot performed to determine the expression levels of soluble version of scFv/intein/CBD fusion proteins according to the present Patent Application.

There will now be described by way of example a specific mode contemplated by the Inventors. In the following description numerous specific details are set forth in order to provide a thorough understanding. It will be apparent however, to one skilled in the art, that the present invention may be practiced without limitation to these specific details. In other instances, well known methods and structures have not been described so as not to unnecessarily obscure the description.

### Example 1 - Materials and Methods

### Viruses and cell lines

The R5-tropic primary HIV-1 isolate #A was isolated from subject A in a cohort of individuals who were recruited for a study of HIV-1 fitness and who were not receiving HAART therapy. Informed consent was obtained from the participants in accordance with the Institutional Review Boards of University of Texas Health Science Center at Houston, Texas and the Baylor College of Medicine. The cell tropism of primary isolate #A was determined using a panel of GHOST co-receptor cell lines as described before (43, 44). HIV-1 molecular clones Bru-3 (X4 tropic, subtype B), Bru-Yu (R5 tropic, subtype B), NL4-3 (X4 tropic, subtype B) were produced by transfecting proviral plasmids pBru-3, pBru-Yu or pNL4-3 into 293T cells using a calcium phosphate precipitation or a Fugene-6 reagent method (Roche Diagnostics, Mannheim, Germany).

The packaging cell line 293T and human CD4⁺ T cell line CEMss were maintained complete DMEM medium [i.e. high glucose DMEM supplemented with 10% FBS, 2 mM L-glutamine, 1 mM sodium pyruvate, penicillin (100 U/ml),) and streptomycin (100 µg/ml); Invitrogen Life Technologies]. A human intestinal epithelial cell line Caco2 was purchased from American Tissue Culture Cooperation (ATCC) and maintained in complete DMEM.

Chronically HIV-1 infected CEMss cells were generated by infecting CEMss cells with HIV-1 Bru-Yu at the multiple of infection (MOI) of 0.01. After the infection, cells were maintained in the complete DMEM. Periodically, the amount of p24 in the supernatants was measured by ELISA.

### Lentiviral vector and recombinant viruses

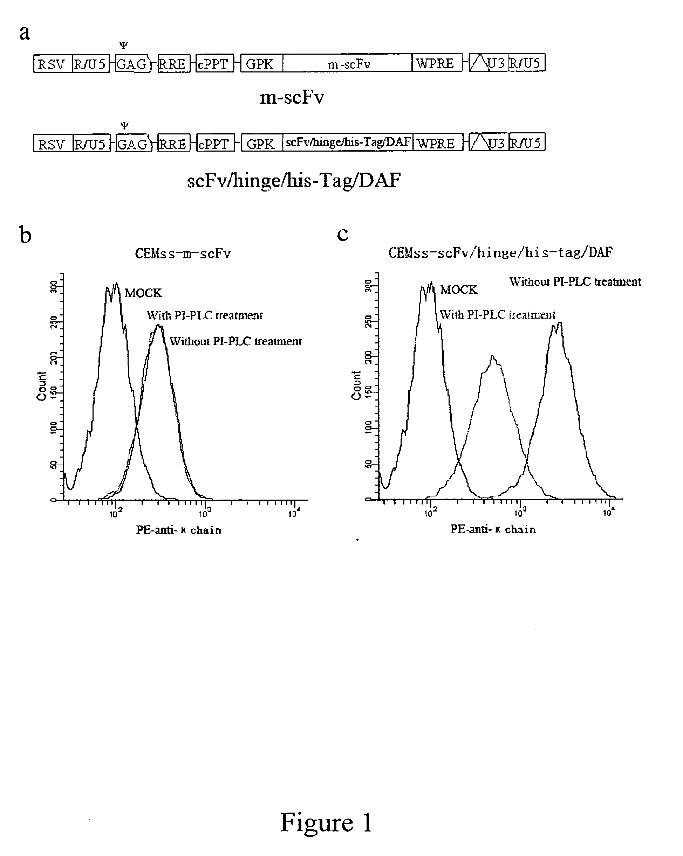

To construct the scFv/hinge/his-tag/DAF, a 143-bp DNA fragment (SEQ ID NO: 3) containing an 8-bp sequence of 3'end of human IgG3 hinge region and a second 123-bp DNA fragment (SEQ ID NO:4) scFv/hinge/his-tag/DAF encoding a 6 x his tag, the C-terminal 34 amino acid residues of DAF and a stop codon, and a 12-bp sequence of a *Sal* I site and additional 6 bp was generated by a recursive PCR (45). Another DNA fragment containing a 12-bp sequence of a *Blg* II site and additional 6 bp, a sequence encoding the signal peptide, anti-HIV-1 gp41 scFv and 11 residues of human IgG3 hinge region, and an 8-bp sequence of 5'end of his tag was amplified by PCR using previously generated pLNCX-TCR-m-scFv as a template (14). These two fragments were designed so that there are 16 nt overlapping between the 3'end of scFv/hinge (SEQ ID NO: 3) and 5'end of his tag/DAF fragments (SEQ ID NO: 4). The whole fragment encoding the entire scFv/hinge/his-tag/DAF (SEQ ID NO: 5) was then generated by an overlapping PCR. The amplified cDNA was then ligated in a TA vector system for sequence analysis (Invitrogen Life Technologies). cDNA containing the correct scFv/hinge/his-tag/DAF was cloned into the Bam H1 and Sal I sites of lentiviral transfer vector pRRLsin-18.PPT.hPGK.Wpre (46, obtained from Dr. L. Naldini, University of Torino Medical School, Torino, Italy). The resulting lentiviral transfer construct (SEQ ID NO: 6) was designated as pRRL- scFv/hinge/his-tag/DAF (see Fig. 1a).

To construct m-scFv into the same lentiviral vector, m-scFv was also amplified by PCR using previously generated pLNCX-TCR-m-scFv (SEQ ID NO: 7) as a template (14). cDNA containing the correct m-scFv was cloned into the Bam H1 and Sal I sites of lentiviral transfer vector pRRLsin-18.PPT.hPGK.Wpre (SEQ ID NO: 8). The resulting lentiviral transfer construct was designated as pRRL-m-scFv (SEQ ID NO: 9) (see Fig. 1a)

Recombinant lentiviral vectors were prepared as described by Follenzi *et al.* (46). Briefly, 293 T cells were transfected with the transfer construct pRRL-scFv/hinge/his-tag/DAF (SEQ ID NO: 6) or pRRL-m-scFv(SEQ ID NO: 9), a packaging construct encoding HIV-1 gag/pol (pLP1) (SEQ ID NO: 10) and two other plasmids encoding VSV-G envelope (pLP/VSVG) (SEQ ID NO: 11) and HIV-1 Rev protein (pLP2) (SEQ ID NO: 12) (Invitrogen Life Technologies). As control, the transfer construct pRRL-eGFP (SEQ ID NO: 13) (14) was also used to transfect 293 T cells. Twenty four hours later, the supernatant was harvested and ultracentrifuged. The vector pellets were resuspended in a small volume of DMEM. Vector titer was determined as described before (14). The amount of HIV-1 gag p24 in concentrated vector stocks was determined by ELISA.

### Stable transduced CEMss cell line

To transduce CEMss cells, 1 x 10⁵ CEMss cells and 2 x 10⁶ TU of lentiviral vector containing scFv/hinge/his-tag/DAF (SEQ ID NO: 6), eGFP or m-scFv (SEQ ID NO: 9) were added onto 24-well tissue culture plate in the presence of 8 µg/ml of polybrene (Sigma). The next day cells were extensively washed and cultured for another 3 days. Transduction efficiency was measured by FACS analysis of expression of eGFP, m-scFv or scFv/hinge/his-tag/DAF (see below). The inventors usually found that after a single round transduction, over 95% cells were eGFP-, m-scFv- or scFv/hinge/his-tag/DAF- positive (data not shown). Stable transduced cell lines were then generated by limiting dilution assay as described before (14).

### FACS analysis

To study cell surface expression of m-scFv (SEQ ID NO: 9) and scFv/hinge/his-tag/DAF (SEQ ID NO: 6), 1 x 10⁶ parental and m-scFv and scFv/hinge/his-tag/DAF-transduced CEMss cells were incubated with a rabbit anti-human *kappa*-chain antibody (Boehringer Mannheim, Indianapolis, IN) for 45 min on ice. Cells then were washed twice with FACS buffer (PBS containing 1% BSA and 0.02% NaN₃) and stained with PE-conjugated goat anti-rabbit IgG Ab (Sigma-Aldrich, St. Louis, MO) for another 45 min on ice. Cells then were washed twice with FACS buffer and fixed with 1% formaldehyde in 0.5 ml of FACS buffer. FACS analysis was performed on a FACScan (Becton Dickinson, Mountain View, CA).

To determine whether the expression of scFv/hinge/his-tag/DAF (SEQ ID NO: 6) is truly through a GPI anchor, 2 x 10⁵ parental and m-scFv (SEQ ID NO: 9) and scFv/hinge/his-tag/DAF-transduced CEMss cells were first incubated with or without 0.04 µg PI-PLC (Prozyme Products, Niles, IL) in a buffer containing 10mM Tris-HCl, 140mM NaCl pH 7.5, 1% FBS at 37°C for 15 min. After the incubation, cells were washed twice to remove remaining PI-PLC and then stained with a rabbit anti-human *kappa*-chain antibody as described above.

### HIV-1 infection and p24 assay

Pooled EGFP-, m-scFv-, and GPI-scFv-transduced CEMss cell lines (1 X 10⁶) were incubated with HIV-1 strains Bru-3 and Bru-Yu (60,000 c.p.m. reverse transcriptase activity) in a final volume of 0.5 ml for 5 hours. Cells were then washed three times with HBSS and resuspended in 6 ml of the complete DMEM medium and incubated at 37°C for 20 days. Every 4 days, 4.5 ml of cell suspensions were harvested and replaced with the fresh medium. The supernatants were then collected. HIV-1 p24 in the supernatants were measured by ELISA (Beckman Coulter) according to the manufacturer's instruction.

### Generation of pseudotypes of HIV-1 vector and a single-cycle infectivity assay

To generate pseudotypes of HIV-1 vector, 4.5 X 10⁶ 293T packaging cells were co-transfected with 20 µg of HIV-1-luciferase transfer vector and 20 µg of DNA plasmid encoding either HIV-1 envelope pDOL or Q168 or VSV-G control using a calcium phosphate precipitation method. After overnight incubation, cells were washed once with HBSS and cultured in 10 ml of complete DMEM supplemented with 100 µM sodium butyrate. 6 hrs later, cells were washed once with HBSS and cultured in 10 ml of complete DMEM. The pseudotype-containing supernatants were harvested in 16 to 20 hrs and the amount of HIV-1 p24 in the supernatants was measured by ELISA.

In a single-cycle assay to measure the infectivity of pseudotypes, 1 x 10⁵ pooled EGFP- and GPI-scFv-transduced CEMss cell lines were transduced with various amounts of pseudotype-containing supernatants overnight. Cells were then washed twice with HBSS and cultured in complete DMEM medium for 2 days. Cells were then harvested and washed once with HBSS (without phenol red) and resuspended in 200 µl of HBSS (without phenol red). Luciferase activity in 50 µl of cell suspensions was measured by a BrightGlo Luciferase assay according to the manufacturer's instruction (Promega).

### Ex vivo generation of human DCs

Peripheral blood was obtained from healthy donors at the Gulf Coast Regional Blood Center, Houston, Texas. Peripheral blood mononuclear cells (PBMCs) were isolated using Ficoll density gradient centrifugation as described before (47). To isolate CD14⁺ monocytes, the anti-CD14 microbeads and miniMACS system were used according to manufacturer's instructions (Miltenyi Biotech, Auburn, CA). Briefly, 10⁸ PBMCs were resuspended in 800 µl of binding buffer (PBS containing 0.5% BSA and 2 mM EDTA). 200 µl of MACS CD14 microbeads were added, mixed and incubated at 4°C for 15 minutes. The cell-bead mixture was then washed once with binding buffer and resuspended in 1 ml of fresh binding buffer. CD14⁺ cells were positively selected using an MS+/RS+ column tip. The isolated cells were washed once with complete RPMI and cultured in the complete RPMI supplemented with GM-CSF (1000 U/ml) and IL-4 (1000 U/ml) (R & D System, Minneapolis, MN) for 7 days to generate immature DCs as described before (48). The expression of DC-SIGN on the cell surface of immature DCs was measured by anti-DC-SIGN antibody staining followed by FACS analysis as described above.

### Capture and transfer assay with human DCs

DCs (10⁵ per well) were seeded in triplicates in a 96 well U-bottom plate and incubated with 25 µl of NL4-3, or 100 TCID₅₀ of primary isolate #A in a total volume of 200 µl at 37°C and 5% CO₂ for 4 hours. Cells were then washed three times with PBS to remove free viruses and resuspended in 2 ml of complete RPMI into wells of 24-well plate with or without 1.5 X 10⁵ CEMss-EGFP and CEMss-GPI-scFv cells added. Cells were continued to be cultured for 30 days. Every 3 days, medium was removed from each well and replaced with fresh complete medium. HIV-1 p24 in the supernatants was measured by ELISA as described above.

### Protein extraction and purification

To purify GPI-scFv (SEQ ID NO: 14) from GPI-scFv-transduced CEMss cells, the Ni-NTA purification system (Invitrogen) was used. Briefly, 3 to 5 x 10⁸ cells were harvested, washed once with PBS and then lysed with 6 to 10 ml of lysis buffer (50mM Tris-HCl pH8.0, 1% NP-40, 0.1% saponin and protease inhibitor cocktail) at 4°C for 1 hour. The lysate was centrifuged at 14,000 rpm for 10 min at 4°C. The supernatants were collected, mixed with 1 to 2 ml of loading buffer-equilibrated Ni-NTA agarose beads at 4°C for 3 hours and then loaded onto the column. The column was then washed with 30 ml of washing buffer and eluted with elution buffer containing a step-wise increase of concentration of imidazole from 40 to 250µM. The amount of protein in each fraction was estimated by a BCA assay (Pierce). The fractions containing protein peak were combined and dialyzed against PBS overnight and filtered through a 0.45 µm filter. The amount of protein was again determined by the BCA assay. To determine the purity, purified protein was loaded and separated in 12% SDS-PAGE and followed by silver staining. From 1 to 5 x 10⁸ cells, the inventors usually obtained 100 to 800 µg of protein with over 95% purity.

### "Paint" cells with a GPI anchored protein

To "paint" CEMss cells with a GPI-scFv (SEQ ID NO: 14), CEMss cells with viabilities greater than 97% were washed 3 times with PBS containing 1% FBS. Cells (2x10⁵) were suspended in 100 µl PBS containing 1% FBS and 100 ng purified GPI-scFv. Cells and GPI-scFv mixtures were incubated at 37°C and 5% CO₂ for 1 hour. After the incubation, cells were extensively washed with PBS containing 1% FBS. Incorporation of GPI-scFv (SEQ ID NO: 14) onto cells was determined by anti-his tag antibody staining followed by FACS analysis as described above.

To determine how stable the incorporated GPI-scFv (SEQ ID NO: 14) on the surface of cells, CEMss cells were "painted" as described above. The "painted" cells were then cultured in the complete medium for 0, 1, 3, 5 hours before being stained with anti-his tag antibody.

To "paint" Caco2 cells, Caco2 cells were seeded (5,000 cells per well) onto the Lab-Tek chamber slide (Nalge Nunc International, Rochester, NY) and incubated at 37°C 5% CO₂ for 2 days. Cells were then washed 3 times with PBS containing 1% FBS and incubated with 100 µl PBS containing 1% FBS and 100 ng purified GPI-scFv at 37°C and 5% CO₂ for 1 hour. After the incubation, cells were extensively washed with PBS containing 1% FBS. Incorporation of GPI-scFv onto cells and co-localization of GPI-scFv with a lipid raft marker GM1 were determined by immunofluorescent staining and confocal analysis (see below).

To "paint" GPI-scFv (SEQ ID NO: 14) onto the apical membrane of Caco2 cell monolayer, 1.5 x 10⁴ Caco2 cells were seeded per transwell (12 mm diameter, 0.4 µm pore size, Coming, NY) in complete medium. Medium was changed every day until cell monolayer was formed. The integrity of monolayer was monitored by the TEER and by dextran-FITC transportation until no random leak was found. The monolayer of Caco2 in the upper chamber of transwell was washed 5 times with PBS and incubated with 0.5 ml PBS containing 1% FBS and 1 µg purified GPI-scFv at 37°C and 5% CO₂ for 1 hour. After the incubation, the upper chamber of transwell was extensively washed with PBS containing 1% FBS before being used in transcytosis studies (see below).

### Immunofluorescent staining and confocal analysis

Parental and GPI-scFv-transduced Caco2 cells were seeded (5,000 cells per well) onto the Lab-Tek chamber slide (Nalge Nunc International, Rochester, NY) and incubated at 37°C 5% CO₂ for 2 days. Parental Caco2 cells were then "painted" with purified GPI-scFv as described above. GPI-scFv-"unpainted" and "painted" as well as GPI-scFv-transduced Caco2 cells were then washed twice with 500 µl PBS and blocked with 5% goat serum for 1 hour. Cells were stained with mouse anti-his-tag Ab (Sigma) at 4°C for 1 hour, washed 3 times with PBS, and stained with Cy3-conjugated goat anti-mouse IgG Ab (Jackson Lab.) at 4°C. Cells were washed 3 times with PBS and then incubated with biotinylated B-Ctx (Sigma) for 30 min at 4°C. Cells were washed 3 times with PBS and incubated with FITC-conjugated streptavidin (Sigma) for 1 hour at 4°C. After cells were washed 3 times with PBS, the slides were mounted before being analyzed under confocal fluorescent microscope (Zeiss Model LSM 510).

### Transcytosis

To investigate if purified GPI-scFv (SEQ ID NO: 14) could anchor onto the apical membrane of intestinal epithelial monolayer to block HIV transcytosis, 1.5 x 10⁴ untransduced and GPI-scFv-transduced Caco2 cells were seeded per transwell (12 mm diameter, 0.4 µm pore size, Coming, NY) in complete DMEM. Medium was changed every day until cell monolayer was formed. The integrity of monolayer was monitored until TEER reached between 500 and 600 Ohm x cm² and dextran-FITC transportation was less than 1000 as described before (49). The monolayer of Caco2 was then washed 5 times with PBS and "painted" in triplicate with purified GPI-scFv protein (SEQ ID NO: 14) (see above) at 37°C for 1 hour. During the time of "painting", chronically HIV-1 Bru-Yu infected CEMss cells were harvested, washed 8 times with PBS to remove all free viruses, counted and then added onto the apical membrane of monolayer of parental, GPI-scFv-"painted" and transduced Caco2 cells in the upper chamber of the transwell (1 million cells per transwell). At 0, 1, 3, 5 and 7 hours post cell-associated HIV-1 challenge, 100 µl of supernatant from both the upper and the lower chambers were harvested and stored at -80°C. The amount of HIV-1 gag p24 was measured as described above.

In the delayed challenge experiments, parental Caco2 cells were seeded and "painted" with purified GPI-scFv protein the same as described above. After the "paint", cells were cultured in the complete medium for another 2.5 hours (the estimated half-life of "painted" GPI-scFv on the cell surface) before chronically HIV-1 Bru-Yu infected CEMss cells were added onto the apical membrane of monolayer in the upper chamber of the transwell. Cell culture, supernatant collection and measurement of HIV-1 gag p24 were the same as described above.

### Example 2 - Expression of anti-HIV-1 gp41 scFv on cell surface through a GPI anchor

The Inventors have genetically linked the sequence encoding the anti-HIV-1 gp41 scFv/IgG3 hinge region (SEQ ID NO: 1) with the sequence encoding a GPI anchor replacement signal (SEQ ID NO: 2) of DAF (decay accelerating factor) (29). The fusion gene scFv/IgG3 hinge/DAF was inserted into a transfer construct of the third generation lentiviral vector. The recombinant viruses were then used to transduce human CD4⁺ T cells CEMss and human intestinal epithelial cells Caco2.

The experiments were initiated to determine 1) whether anti-HIV-1 gp41 scFv can be expressed on cell surface through a GPI anchor; 2) if so, whether GPI-anchored scFv renders cells resistant to both cell-free and DC-captured and transferred HIV-1; 3) whether GPI-anchored scFv can be purified from transduced cells and reintegrated into new cell targets and what the stability of reintegrated GPI-anchored scFv is; and 4) whether GPI-anchored scFv integrated into lipid raft of apical membrane of epithelial monolayer blocks transcytosis of HIV-1.

With reference to figure 1, this shows expression of scFv/hinge/histag/DAF and m-scFv in stably transduced CEMss cell line. *a*. Schematic diagram of the lentiviral vectors pRRL-scFv/hinge/histag/DAF and pRRL-m-scFv (14). scFv: anti-HIV-1 gp41 single-chain Fv derived from a human monoclonal antibody TG15; hinge: a human IgG3 hinge region; histag: a 6 histidine residue tag; DAF: the C-terminal 34 amino acid residues of decay accelerating factor. b. FACS analysis of cell surface expression of m-scFv in m-scFv-transduced CEMss cells vs. parental CEMss control with or without PI-PLC treatment. c. FACS analysis of cell surface expression of scFv/hinge/histag/DAF in scFv/hinge/histag/DAF-transduced CEMss cells vs. parental CEMss control with or without PI-PLC treatment.

Fig. 1a shows the lentiviral vectors containing scFv/hinge/his-tag/DAF (SEQ ID NO: 6) of the current invention and previously generated m-scFv (SEQ ID NO: 9) (14). The m-scFv contains the sequence encoding the anti-HIV-1 gp41 scFv, human IgG3 hinge region and the transmembrane domain of the subunit 1 of the type I IFN receptor (30); while the scFv/hinge/his-tag/DAF contains the same scFv and hinge sequence, a 6 X his residue tag, and a GPI anchor replacement signal of DAF (29). Fig. 1b and 1c show representative FACS analysis of surface expression of m-scFv and scFv/hinge/his-tag/DAF in transduced CEMss cells with or without treatment of PI-PLC (phosphatidylinositol-specific phospholipase C). Although both scFv/hinge/his-tag/DAF (SEQ ID NO: 6) and m-scFv (SEQ ID NO: 9) are expressed, the level of scFv/hinge/his-tag/DAF (SEQ ID NO: 6) expression is about 10-fold higher than that of m-scFv (SEQ ID NO: 9).

Moreover, when treated with PI-PLC, substantial reduction of cell surface expression of scFv/hinge/his-tag/DAF was observed (Fig. lc); while no reduction in m-scFv (Fig. 1b), indicating that the expression of scFv/hinge/his-tag/DAF on the cell surface is indeed through a GPI anchor. The scFv/hinge/his-tag/DAF will be referred as GPI-scFv for simplicity.

In conclusion therefore the Inventors have shown that GPI-scFv is expressed significantly better than m-scFv using otherwise comparable expression systems.

### Example 3 - Effects of GPI-scFv expression on C, CXCR4 and CCR5 expression

The Inventors next examined the effect of the GPI-scFv on the surface expression of CD4 (Cluster of Differentiation 4 is a glycoprotein expressed on the surface of T helper cells, regulatory T cells, monocytes, macrophages, and dendritic cells), CXCR4 (also called fusin, is an alpha-chemokine receptor specific for stromal-derived-factor-1 and CCR5 (chemokine (C-C motif) receptor 5). CEMss-GPI-scFv and CEMss-eGFP were stained with anti-CD4, CCR5 and CXCR4 antibodies and isotype matched controls. No significant difference in CD4, CXCR4 and CCR5 between CEMss-GPI-scFv and CEMss-eGFP (Fig. 2a-f). Thus, like m-scFv (SEQ ID NO: 9) (14), the surface expression of GPI-scFv (SEQ ID NO: 6) does not alter HIV-1 receptor and co-receptor expression.

### Example 4 - GPI-scFv expressed on the cell surface renders cells resistant to cell-free and DC-captured and transferred HIV-1

With reference to figure 2, shows the cell surface expression of CD4, CXCR4 and CCR5 of a representative CEMss-eGFP cell line (*a-c*) and of a representative CEMss-GPI-scFv cell line (*d*-*f*). p24 activity in pooled CEMss-eGFP, CEMss-m-scFv, and CEMss-GPI-scFv cell lines infected with HIV-1 strain Bru-3 (g) and Bru-Yu (h). p24 activity detected in supernatants of CEMss-eGFP or CEMss-GPI-scFv co-cultured with human DC-captured HIV-1 NL4-3 (i) or primary isolate #A (*j*). Relative luciferase activity (RLA) in CEMss-eGFP or CEMss-GPI-scFv cells transduced with lentiviral vectors expressing HIV-1 envelope pDOL or Q168 or VSV-G envelope (*k*).

To examine the effect of the GPI-scFv (SEQ ID NO: 6) on HIV-1 infection, four CEMss-GPI-scFv, four CEMss-m-scFv and four CEMss-eGFP cell lines were pooled and infected with HIV-1 Bru-3 (X4 virus) and Bru-Yu (R5 virus). For cells infected with Bru-3, inhibition was apparent at day 4 and continued throughout an experiment of 20 days duration. Supernatants of CEMss-GPI-scFv showed 99% reduction on day 8, over 3 log reduction on day 12, and more than 4 log reduction on day 16 and 20; while supernatants of CEMss-m-scFv showed only 90% reduction on day 8, 99% reduction on day 12, less than 3 log reduction on day 16, and less than 2 log reduction on day 20 (Fig. 2g). For cells infected with Bru-Yu, inhibition was apparent at day 8 and continued throughout an experiment of 20 days duration. Supernatants collected on day 12, 16 and 20 showed 90-99.5% reduction in both CEMss-m-scFv and CEMss-GPI-scFv (Fig. 2h). The experiment was repeated two additional times with similar results. The Inventors consider it possible that the better inhibition of Bru-3 replication seen in CEMss-GPI-scFv could be due to the 10-fold higher surface expression of GPI-scFv than m-scFv (see Fig. 1b and c).

The Inventors next examined the effect of GPI-scFv (SEQ ID NO: 6) on the infection of HIV-1 captured and transferred by human DCs. Fig. 2i and j show that DCs alone infected with NL4-3 or primary isolate #A resulted in a lower amount HIV-1 replication, which is consistent with the recent report by Nobile *et al.* (31). However, 7 days after coculturing CEMss-eGFP with NL4-3-infected DCs the amount of p24 in the supernatant increased over 3 logs as compared to NL4-3-infected DCs alone.

In contrast, 7 days after coculturing CEMss-GPI-scFv with NL4-3-infected DCs the amount of p24 in the supernatant increased less than 1 log (Fig.2 i). Coculturing CEMss-eGFP and CEMss-GPI-scFv with DC-captured primary isolate #A resulted in much slower HIV-1 replication. Only after 14 days or later could measurable HIV-1 gag p24 be detected (data not shown). 21 days after coculturing CEMss-eGFP with primary isolate #A-infected DCs the amount of p24 in supernatant increased about 2 log as compared to primary isolate #A infected DCs alone.

21 days after coculturing CEMss-GPI-scFv with primary isolate #A-infected DCs the amount of p24 in the supernatant was even lower than primary isolate #A-infected DCs alone (Fig. 2j). The experiment has been repeated once with similar results. These results demonstrated that GPI-scFv blocks HIV-1 captured and transferred by human DCs.

To examine whether inhibition by GPI-scFv is HIV-1 envelope specific, the Inventors transduced CEMss-eGFP and CEMss-GPI-scFv with recombinant HIV-1 vectors expressing HIV-1 envelope pDOL (subtype B) (32) and Q168 (subtype A) (33) or VSV-G envelope. As VSV-G envelope interacts directly with the lipid moiety in lipid bilayer of the plasma membrane, it bypasses the requirement of the interaction between HIV-1 envelope and its receptor and coreceptor. Fig. 2k shows that for CEMss-GPI-scFv transduced with pDOL pseudotypes an 8-fold reduction in relative luciferase activity (RLA) was observed as compared to CEMss-eGFP and for CEMss-GPI-scFv transduced with Q168 pseudotypes a 180-fold reduction was observed. In contrast, similar transduction efficiency was observed with VSV-G pseudotypes. The Inventors therefore conclude that the GPI-scFv specifically targets the HIV-1 envelope.

### Example 5 - GPI-scFv can be purified from stable transduced cells and "painted" onto new target cells

To purify GPI-scFv (SEQ ID NO: 14), GPI-scFv-transduced CEMss cells were lysed. GPI-scFv was purified through a Ni-NTA agarose column.

With reference to figure 3, this shows the purification of GPI-scFv and reintegration of purified GPI-scFv, in which. *a*. Silver stain of 12% SDS/PAGE of cell lysate, flow through, wash, and elution (after the samples were combined, dialyzed and filtered). b. Forward and side scatters of mock "painted" CEMss stained with anti-his-tag antibody. c. Forward and side scatters of GPI-scFv-"painted" CEMss stained with anti-his-tag antibody. d. Detection of reintegrated GPI-scFv on the cell surface between mock and GPI-scFv"painted" CEMss cells with anti-his-tag antibody. e-h. Detection of turnover of reintegrated GPI-scFv on the cell surface at 0 (e), 1 (*f*), 3 (g), and 5 (h) hours post "paint".

The amount of proteins in each eluted fraction was measured by BCA assay. The positive fractions were combined and dialyzed against PBS. After the dialysis, the samples were filtered and aliquots were stored at - 20 °C until use.

With reference to figure 4, this shows "Painted" or transduced GPI-scFv onto plasma membrane of human intestinal cell line Caco2 cells colocalizes with the raft marker GM1. *a-l.* Confocal analysis of "painted" or transduced GPI-scFv and lipid raft marker GM1 on parental Caco2 cells (*a-d*), GPI-scFv-"painted" Caco2 cells *(e-h)* and GPI-scFv-transduced Caco2 cells (*i*-*l*) stained with mouse anti-his-tag Ab followed by Cy3-conjugated goat anti-mouse IgG Ab and further incubated with biotinylated cholera toxin B subunit (Ctx B) followed by FITC-conjugated streptavidin. Inhibition of transcytosis of cell-associated HIV-1 through an epithelial monolayer by "painted" or transduced GPI-scFv. m. Time course of HIV-1 transcytosis across the human epithelial monolayer with or without "painted" or transduced with GPI-scFv. Percentage (%) was determined by the mean value of the amount of p24 detected in the lower chamber versus in the upper chamber of the transwell. The mean value and standard deviation were derived from triplicate wells. n. Time course of HIV-1 transcytosis across the human epithelial monolayer with or without "painted" with GPI-scFv followed by viral challenge right away or delayed for 2.5 hours (the estimated half-life of reintegrated GPI-scFv on the cell surface). Percentage (%) was determined by the mean value of the amount of p24 detected in the lower chamber versus in the upper chamber of the transwell. The mean value and standard deviation were derived from triplicate wells.

Fig. 4a shows silver staining of protein profile in cell lysates, flow through, wash, and elution (after the samples were combined, dialyzed and filtered). Clearly, only one dominant 35-37 kD band corresponding to GPI-scFv was detected in the elution. Thus, highly purified GPI-scFv was obtained through affinity purification.

To "paint" GPI-scFv (SEQ ID NO: 14) onto cell surface, CEMss cells were incubated with 100 ng purified GPI-scFv (SEQ ID NO: 14) for 1 hour. Cells were then extensively washed and stained with an anti-his-tag specific antibody. Fig. 4d shows that almost all cells were "painted" with GPI-scFv (SEQ ID NO: 14). Importantly, no difference in side and forward scatters between mock and "painted" cells was found (Fig. 4b and c).

To determine the stability of reintegrated GPI-scFv on cell surface, CEMss cells were "painted" with purified GPI-scFv as described above. After the "paint", cells were extensively washed and cultured in complete DMEM at 37°C. At 0, 1, 3, and 5 hours post "paint", cells were harvested and stained with an anti-his-tag specific antibody. Fig. 4e-h show that the amount of reintegrated GPI-scFv on the cell surface gradually decreased. The estimated half-life is about 2.5 hours.

### Example 6 - GPI-scFv is anchored into lipid raft of plasma membrane and blocks transcytosis of HIV-1 through an epithelial monolayer

The Inventors next examined whether GPI-scFv (SEQ ID NO: 14) is integrated into lipid raft of plasma membrane, Caco2-GPI-scFv (transduced) and Caco2 were seeded onto a chamber slide. Caco2 was then "painted" with or without 100 ng purified GPI-scFv. After the "paint", cells were co-stained with an anti-his-tag specific antibody and cholera toxin B subunit (Ctx B) (see the Materials and Methods for the details). Ctx B interacts with GM1 ganglioside receptor (GM1) molecules located in lipid raft of the plasma membrane (34). After the co-staining, cells were observed and analyzed under a confocal fluorescent microscope. Both transduced and "painted" GPI-scFv were well detected on the surface of Caco2 cells (Fig. 4f and 4j) and co-localized with GM1 (Fig. 4h and 4l), indicating that GPI anchors scFv onto the lipid raft of plasma membrane.

The Inventors next examined whether GPI-scFv can be "painted" onto apical membrane of epithelial monolayer to block transcytosis of HIV-1. Caco-2 and Caco-2-GPI-scFv (transduced) were seeded onto the upper chamber of a 24-well transwell plate and cultured for 15 days or longer until the formation of an epithelial monolayer. Cell growth and integrity of the monolayer were monitored by the transepithelial electrical resistance (TEER) and dextran-FITC transportation. After intact monolayer was formed, 1 µg purified GPI-scFv was added onto the upper chamber of the transwell in triplicate and incubated at 37°C for 1 hour. After the "paint", the upper chambers were carefully washed and incubated with CEMss cells chronically infected with HIV-1 Bru-Yu (see Materials and Methods for details). At 0, 1, 3, 5, and 7 hours, aliquots of supernatants in both the upper and the lower chamber were harvested. The amount of HIV-1 gag p24 was measured. While no significant difference in the amount of p24 was detected in supernatants from the upper chamber among "unpainted", GPI-scFv-"painted" or transduced monolayer, the amount of p24 in supernatants from the lower chamber of 'unpainted" control was significantly higher than of GPI-scFv-"painted" or transduced monolayer. The percentage of the amount of p24 in the lower chamber versus the upper chamber of transwell, therefore, was also significantly higher in "unpainted" control than of GPI-scFv-"painted" or transduced monolayer (Fig. 4m), indicating that both "painted" and transduced GPI-scFv effectively blocks transcytosis of cell-associated HIV-1.

To further examine the effect of "painted" GPI-scFv on HIV-1 transcytosis, a delayed challenge experiment was carried out (see the Materials and Methods for details). In the delayed experiment, after the "paint", epithelial monolayer was either incubated right away or cultured for 2.5 hours (the estimated half life of reintegrated GPI-scFv on the cell surface) before being incubated with chronically HIV-1 Bru-Yu-infected CEMss. Fig. 4n shows that the percentage of the amount of p24 in the lower chamber versus the upper chamber was significantly higher in "unpainted" control than of GPI-scFv-"painted" monolayer challenged non-delayed or delayed for 2.5 hours, indicating that epithelial monolayer with half an amount of initial "painted" GPI-scFv is still significantly resistant to transcytosis of HIV-1.

### Example 7 - scfv Antibodies comprising a glycosylphosphatidylinositol (GPI) anchor

Following completion of the proof-of-principle study, see examples 2, 3 and 4 above, which demonstrated that GPI can be used to anchor a non-neutralizing anti-HIV-1 gp41 scFv (TG15) onto the lipid raft of the plasma membrane and that this significantly blocks all three forms of virus transmission: cell-free virus, DC-captured and transferred virus and transcytosis through a mucosal epithelial monolayer. The inventors have constructed several new GPI-anchored human scFvs (AB31, AB32, AB5 and AB48) against different epitopes of the HIV-1 envelope as well as a negative scFv control (AB65). This new set of anti-viral proteins were constructed using essentially the same method as the scFv/hinge/his-tag/DAF construct (SEQ ID NO: 5), see example 1 above.

The resulting recombinant lentiviral vectors expressing these new GPI-anchored human scFvs were generated and transduced into several HIV-1-susceptible cell lines, using the same method described in example 1 above. The expression of transgenes in transduced cell lines was studied with or without PI-PLC treatment and their susceptibility to various HIV-1 envelope pseudotypes (Q168 subtype A, pDOL and AD8 subtype B, MJ4 subtype C) and VSV-G control were also tested.

The inventors have demonstrated that all these scFv fusion genes are expressed on the cell surface through GPI anchor.

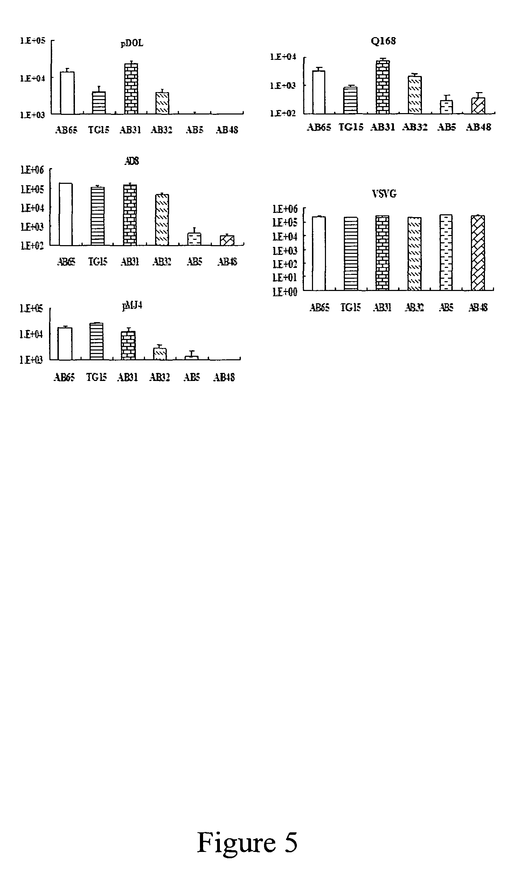

Compared to the negative GPI-scFv control (AB65), GPI-scFv (AB31) does not have any inhibitory effect on transduction efficiency of any of the tested HIV-1 envelope pseudotypes. GPI-scFv (TG15) has a moderate inhibitory effect on HIV-1 envelope pseudotypes Q168 and pDOL, but not AD8 and MJ4. GPI-scFv (AB32) has a moderate inhibitory effect on HIV-1 envelope pseudotypes pDOL and MJ4, but not Q168 and AD8. In contrast, GPI-scFvs (AB5 and AB48) both have strong inhibitory effects on all HIV-1 pseudotypes tested (see Figure 5). Thus, the inventors have demonstrated that the effectiveness of inhibition by GPI-scFvs is dependent upon which epitope(s) of the HIV-1 envelope that the scFvs interacts with.

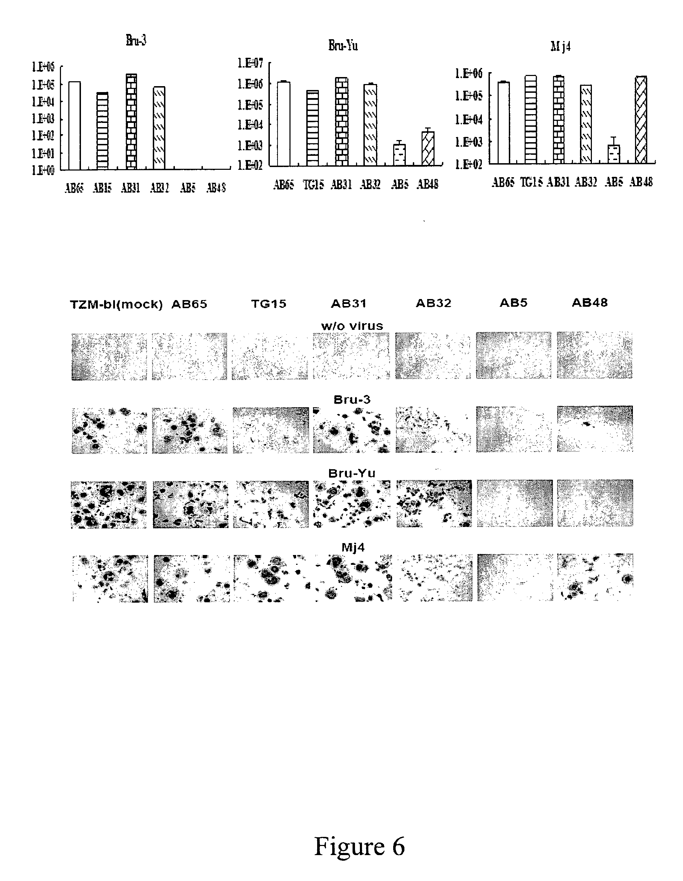

Figure 6 shows the effect of GPI-scFvs on infection of wild type HIV-1 (Bru-3 subtype B X4; Bru-Yu subtype B R5; and MJ4 subtype C R5) measured by relative luciferase assay (a) and β-gal staining (b). Compared to mock and negative GPI-scFv control (AB65), GPI-scFv (AB31) does not have any inhibitory effect on all these viruses. In the present Patent Application the mock control cell population does not comprise any GPI-scFv anti-viral peptides.

GPI-scFv (TG15) has moderate inhibitory effect on Bru-3 and Bru-Yu, but not on MJ4, as measured by β-gal staining. GPI-scFv (AB32) has moderate inhibitory effect on Bru-3 and MJ4, but not on Bru-Yu, as measured by β-gal staining. GPI-scFv (AB48) has strong inhibitory effect on Bru-3 and Bru-Yu, but not MJ4, as measured by RLA assay. As measured by β-gal staining, GPI-scFv (AB48) has strong inhibitory effect on Bru-3 and Bru-Yu, but moderate effect on MJ4. In contrast, GPI-scFv (AB5) has strong inhibitory effect on all three viruses as measured by both assays.

### Example 8 - scFv Antibodies comprising chemically synthesized lipid anchors

To show the utility of the semi-chemical synthesis approach, the inventors have chemically synthesized 12 lipid moieties, including 2 GPI analogs. At the same time the inventors have constructed and produced green fluorescent protein (GFP) with a thioester group at its C-terminus. The inventors have covalently linked these GFP-thioester proteins with each of the lipid moieties by native chemical ligation (NCL).

Native chemical ligation is a widely used form of chemical ligation and is normally used to construct a larger polypeptide from two or more unprotected peptides. In the present invention native chemical ligation is used to link a peptide containing a C-terminal thioester group, with a reactive lipid molecule in the presence of a thiol catalyst.

After the ligation, the lipidated GFPs along with non-lipidated GFP were incubated with human CD4⁺ T cell line CEMss as per the experiments in example 5 above. Table 1 summarises the mean and the standard deviation of incorporation of various lipidated GFP into human CD4 T cell lines CEMss detected by FACS analysis.

**Table 1. The efficiency of incorporation of various lipidated GFPs into CEMss.**

| Cat.# | Mean Fluorescence Intensity | Standard Deviation Fluorescence Intensity |
|---|---|---|
| Mock (assay control) | 72.0 | 0.0 |
| GFP alone | 77.3 | 0.6 |
| GPI analog-2 GFP | 288.0 | 14.4 |
| GPI analog-3 GFP | 521.7 | 29.7 |
| 74 GFP | 1,093.0 | 30.1 |
| 76 GFP | 3,150.0 | 204.9 |
| 78 GFP | 1,611.0 | 64.6 |
| 82 GFP | 79.3 | 1.2 |
| 84 GFP | 317.0 | 15.1 |
| 86 GFP | 2,718.0 | 123.5 |
| 88 GFP | 187.0 | 16.0 |
| 90 GFP | 233.3 | 14.3 |
| 91 GFP | 152.3 | 5.5 |
| 94 GFP | 1,318.0 | 47.5 |
| CEMss-GFP (assay control) | 6,113.3 | 292.6 |

The incubation of the various lipidated GFPs with CEMss cells do not alter the cell viability measured by side and forward scatters. While the incubation of GFP alone does not lead to the incorporation of GFP into CEMss cells, the incubation of the various lipidated GFPs does lead to the efficiently incorporation into CEMss cell membranes.

In particular, several lipid moieties (namely #74, #76, #78, #86 and #94) incorporate GFP into the cells much more efficiently than GPI analogs, which make them very attractive candidates to be used to covalently link with suitable antibody molecules (see below). Thus, the inventors have demonstrated that GFP can be covalently linked to lipid moieties by NCL and that this lipidated GFP, but not GFP alone, can efficiently be integrated into cellular plasma membranes with much better efficiency than GFP with a GPI analog, suggesting that anti-viral petides lipidated using NCL with defined lipid moieties is feasible and could lead to anti-viral peptides which can be more efficiently incorporated into target cell membranes and hence more efficiently protect such cells from the virus against which the antibody component of the peptide has activity.

These synthesized chemical anchors can therefore be used to anchor soluble scFvs which comprise thioester group at their C-terminus into a plasma membrane and to have the required refractory effect upon the target of the scFv.

In particular the inventors consider lipid moiety ^{#}76, which is one of the lipid moieties that incorporate GFP best to the plasma membrane in the inventors preliminary test (see Table 1 above), to be a very good candidate for use with such soluble scFvs.

### Example 9 - scFv/intein/CBD fusion

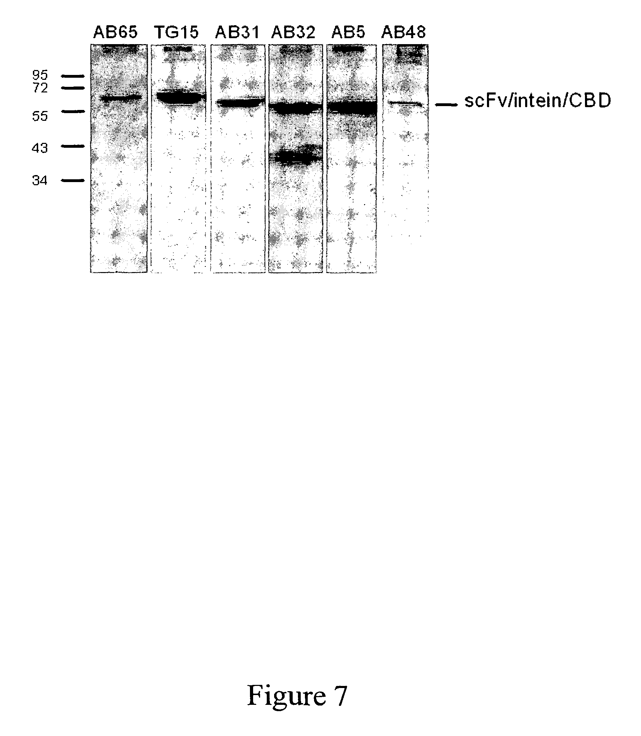

In addition, the inventors have constructed several scFv/intein/CBD fusion genes in an inducible S2 vector and transfected these into S2 cells. After a series of selection and limiting dilution steps, the inventors obtained several individual clones expressing high levels of soluble scFv/intein/CBD fusion proteins (AB65, TG15, AB31, AB32, AB5 and AB48) (Figure 7). These scFv/intein/CBD fusions genes were generated using essentially the same method as described above in example 1 for the scFv/hinge/his-tag/DAF construct (SEQ ID NO: 5).

### Example 10 - Conclusions

The Inventors have demonstrated that by linking an anti-enveloped virus antibody, such as anti-HIV-1 gp41 scFv (TG15) to a lipid attachment signal, either genetically to for example a GPI replacement signal or via a chemical linkage such as via thioester group on the anti-enveloped virus antibody (or a peptide sequence attached thereto); that the scFv is highly expressed on the cell surface through for instance a GPI anchor (Fig. 1c) and/or can be painted onto a cell surface either as an isolated molecule population from a first cell population expressing the scFv-GPI anti-viral peptide or as a de novo molecular population comprising an scFV linked to a suitable peptide.

Anchoring GPI-scFv on the cell surface renders cells resistant to both cell-free and DC-captured and transferred HIV-1 (Fig. 2g-j). Moreover, they have for the first time demonstrated that the GPI-scFv can be purified from transduced cells and "painted" onto new cell targets (Fig. 3). Finally, the Inventors have demonstrated that transduced and "painted" GPI-scFv are anchored onto the lipid raft of apical membrane of an epithelial monolayer and block transcytosis of HIV-1 (Fig. 4). Thus, anchoring even a non-neutralizing anti-HIV-1 gp41 antibody onto the lipid raft of plasma membrane significantly blocks all three modes of HIV-1 transmission: cell-free, DC-captured and transferred, and transcytosis.

These findings have several important implications. First, although the mechanism of blockage by the GPI-scFv is still unknown, it likely has something to do with the epitope it recognizes. The epitope locates in the second heptad repeat (aa residues 644 to 663 of HIV-1 gp41). HIV-1 gp41-mediated fusion is triggered by interaction between the second and the first heptad repeats, which converts a prehairpin gp41 trimer into a fusogenic three-hairpin bundle (1). A peptide T20 derived from this second heptad repeat acts as a fusion inhibitor (35). The epitope is buried within the envelope spikes of the virion. As a result of this, the soluble scFv does not neutralize viral entry. However, the interaction of gp120 to its receptor and co-receptors causes drastic conformational changes (1). During this process it is proposed that this epitope is exposed. When the GPI-scFv happens to be nearby on the cell surface, it is proposed that the interaction of the GPI-scFv with this exposed epitope blocks the subsequent fusion process.

Since many enveloped viruses, such as avian flu, SARS, Paramyxovirus, such as mumps, parainfluenza, measles, RSV; and Filoviruses, such as Marburg and Ebola also go through similar conformational changes during their fusion process, anchoring scFvs against epitopes located in similar prehairpin structure onto the plasma membrane may also block these viruses.

According to the model initially proposed by Weissenhorn et al. (50), the hairpin structure may represent the stable and final conformation adopted by the ectodomain of gp41 or other retroviral TMs during the fusion process. This model envisions the existence of another gp41 conformation, termed the prehairpin, in which the HR1 and HR2 helices are not packed together but rather are dissociated so that the fusion peptide is projected toward the target cell membrane. Transition from this metastable conformation to the stable hairpin structure would provide the force needed to drive the merging of the two membranes. This model and the existence of the prehairpin structure are supported by three lines of arguments: (i) structural analogies between retroviral TMs and the influenza virus HA2, for which transition from inactive to fusion-active conformation could be analyzed by X-ray crystallography; (ii) unmasking of epitopes in the core of the gp41 ectodomain after contact of gp120 with receptors; and (iii) a transdominant-negative effect on gp41-mediated fusion and HIV-1 entry of peptides corresponding to the C-terminal helix (HR2) of HIV-1 gp41, in particular T20 (also known as DP 178).

Second, conventional soluble neutralizing antibodies effectively block cell-free HIV-1. But whether they block cell-associated HIV-1 is questionable (36, 37). For example, neutralizing activity by DC-captured and transferred HIV-1 was demonstrated only when antibody and virus were preincubated before being added to DCs, but not after virus was captured by DCs (36, 37). This could be due to the fact that the transmission of HIV-1 from DCs to CD4⁺ T cells occurs within an infectious synapse (38). The latter could be too tight to be accessible to conventional soluble antibodies. However, since GPI-scFv blocks both cell-free and DC-captured and transferred HIV-1, likely GPI-scFv is present in the infectious synapse. Thus, GPI-scFv blocks HIV-1 transmission under conditions where conventional soluble neutralizing antibody fails to do so.

Third, most HIV infection occurs via mucosal surface such as the vagina, intestine and tonsil. Cellular targets in these sites include M cells (microfold cells) which are specialized epithelial cells in the intestine and tonsil and intraepithelial DCs in the vaginal mucosa. M cells transcytose HIV-1 from the apical to the basolateral surface and intraepithelial DCs capture HIV-1 from apical surface and transfer it to the susceptible cells underneath tissues. Once HIV-1 crosses the mucosa it can directly infect CD4 T cells and DCs. Infected DCs then migrate to the draining lymph node and transfer virus to CD4 T cells there. Thus, the mucosal transmission of HIV-1 takes advantage of normal trafficking CD4 T cells and DCs facilitated by special cells, such as M cells and intraepithelial DCs (39). Thus, for any agent to be effective to prevent mucosal transmission of HIV-1, it should block all these modes of transmission. Since GPI-scFv blocks all three modes of HIV-1 transmission: cell-free, DC-captured and transferred and transcytosis, these results strongly suggest that GPI-scFv may be developed a novel form of microbicide to prevent mucosal transmission of HIV-1. Experiments are currently under the way to investigate whether "painting" GPI-scFv onto cells in human mucosal tissue explant can block HIV-1 transmission.

Finally, the Inventors have shown that when HIV-1 challenge was delayed for 2.5 hours (the estimated half-life of "painted" GPI-scFv on the cell surface) epithelial monolayer with "painted" GPI-scFv is still significantly resistant to transcytosis of HIV-1 (Fig. 6n). Currently what determines the turnover of "painted" GPI-anchored scFv on the cell surface is still not known. Several potential mechanisms, such as the recycling of GPI-scFv between plasma membrane and endosome (40) and the presence of GPI-anchored protein releasing factors, PI-PLD (41) and angiotensin-converting enzyme (42), are currently being investigated in an attempt to find a way to extend the half-life of "painted" GPI-scFv.

### References

1. Wyatt, R., and Sodroski, J. The HIV-1 envelope glycoproteins: fusogens, antigens and immunogens. Science 280, 1884-1888 (1998).
2. Muster, T. et al. A conserved neutralizing epitope on gp41 of human immunodeficiency virus type 1. J. Virol. 67, 6642-6647 (1993).
3. Muster, T. et al. Cross-neutralizing activity against divergent human immunodeficiency virus type 1 isolates induced by the gp41 sequence ELDKWAS. J Virol. 68, 4031-4034 (1994).
4. Stiegler, G. et al. A potent cross-clade neutralizing human monoclonal antibody against a novel epitope on gp41 of human immunodeficiency virus type 1. AIDS Res. And Hum. Retroviruses. 17, 1757-1765 (2001).
5. Zwick, M.B. et al. Broadly neutralizing antibodies targeted to the membrane-proximal external region of human immunodeficiency virus type 1 glycoprotein gp4 1. J. Virol. 75, 10892-10905 (2001).
6. Trkola, A. et al. 1995. Cross-clade neutralization of primary isolates of human immunodeficiency virus type 1 by human monoclonal antibodies and tetrameric CD4-IgG. J. Virol. 69, 6609-6617 (1995).
7. Trkola, A. et al. 1996. Human monoclonal antibody 2G12 defines a distinctive neutralization epitope on the gp 120 glycoprotein of human immunodeficiency virus type 1. J. Virol. 70, 1100-1108 (1996).
8. Moore, J. et al. Primary isolates of human immunodeficiency virus type 1 are relatively resistant to neutralization by monoclonal antibodies to gp 120, and their neutralization is not predicted by studies with monomeric gp120. J. Virol. 69, 101-109 (1995).
9. Burton, D.R. et al. Efficient neutralization of primary isolates of HIV-1 by a recombinant human monoclonal antibody. Science 266, 1024-1027 (1994).
10. Li, Y et al. Broad HIV-1 neutralization mediated by CD4-binding site antibodies. Nat. Med. 13:1032-1034 (2007).
11. Burton, D. R. et al. HIV vaccine design and the neutralizing antibody problem. Nat, Immunol. 5 :233-236 (2004).
12. Takeda, S.N., Dorfman, N.A., Robert-Guroff, M., Notkins, A.L., and Rando, R.F. Two-phase approach for the expression of high-affinity human anti-human immunodeficiency virus immunoglobulin Fab domain in Escherichia coli. Hybridoma 14: 9-18 (1995).
13. Zhou, P., Goldstein, S., Devadas, K., Tewari, D., and Notkins, A.L. Cells transfected with a non-neutralizing antibody gene are resistant to HIV infection: targeting the endoplasmic reticulum and trans-golgi network. J. Immunol. 160: 1489-1496 (1998).
14. Lee, S.J., Gauza, L., Yao, J., Notkins, A.L., and Zhou, P. A non-neutralizing anti-HIV-1 antibody turns into a neutralizing antibody by expressing it on the surface of HIV-1-susceptible cells - a new way to fight HIV-1. J. Immunol. 173:4618-4626 (2004).
15. Lee, S.J. et al. A non-neutralizing anti-HIV-1 antibody turns into a broad neutralizing antibody when expressed on the surface of HIV-1-susceptible cells (II): inhibition of HIV-1 captured and transferred by DC-SIGN, AIDS Res. And Hum. Retroviruses 22:874-883 (2006).
16. Kinoshita, T., Ohishi, K. and Takeda, J. GPI-anchor synthesis in mammalian cells: genes, their products, and a deficiency. J. Biochem. 122:251-257 (1997).
17. Ikezawa, M. et al. Glycosylphosphatidylinositol (GPI)-anchored proteins. Biol. Pharm. Bull. 25:409-417 (2002).
18. Medof, M. E., and Tykocinski, M. L. The cytoplasmic extension as a determinant for glycoinositolphospholipid anchor substitution. In: Welply JK, Jaworski E, editors. Glcobiology. New York: Wiley-Liss, Inc. p. 17-22 (1990).
19. Gerber, L. D., Kodukula, K., and Udenfriend, S. Phosphatidylinositol glycan (PI-G) anchored membrane proteins: amino acid requirements adjacent to the site of cleavage, and PI-G attachment in the COOH-terminal signal peptide. J Biol. Chem. 267:12168-12173 (1992).
20. Sharom, F. J., and Lehto, M. T. Glycosylphosphatidylinositol-anchored proteins: structure, function, and cleavage by phosphatidylinositol-specific phospholipase C. Biochem. Cell Biol. 80:535-549 (2002).
21. Medof, M. E., Kinoshita, T., and Nussenzweig, V. Inhibtion of complement activation on the surface of cells after incorporation of decay-accelerating factor (DAF) into their membranes. J. Exp. Med. 160:1558-1578 (1984).
22. Dedof ME, Nagarajan S, and Tykocinski ML. Cell-surface engineering with GPI-anchored proteins. FASEB J. 10:574-586 (1996).
23. Nagarajan S, Anderson M, Ahned SN, Sell KW, Selvaraj. Purification and optimization of functional reconstitution on the surface of leukemic cell lines of GPI-anchored Fc gamma receptor III. J. Immunol. Meth. 184:241 (1995).
24. Premkumar, D. R. D. et al. Properties of exogenously added GPI-anchored proteins following their incorporation into cells. J. Cell. Biochem. 82:234-245 (2001).
25. Brown, D. A., and Rose, J. K. Sorting of GPI-anchored proteins to glycolipid-enriched membrane subdomains during transport to the apical cell surface. Cell 68:533-544 (1992).
26. Arreaza, G, Melkonian, K. A., LaFevre-Brent, M., and Brown, D. A. Triton X-100-resistant membrane complexes from cultured kidney epithelial cells contain the Src family protein tyrosine kinase p62yes. J. Biol. Chem. 269:19123-19127 (1994).
27. Campbell, S. M., Crowe, S. M., and Mak, J. Lipid rafts and HIV-1: from viral entry to assembly of progeny virions. J. Clin. Virol. 22:217-227 (2001).
28. Lisanti, M. P., Caras, I. W., Davitz, M. A., and Rodriguez-Boulan, E. A glycophospholipid membrane anchor acts as an apical targeting signal in polarized epithelial cells. J. Cell Biol. 109:2145-2156 (1989).
29. Medof, M. E. et al. Cloning and characterization of cDNAs encoding the complete sequence of decay-accelerating factor of human complement. Proc. Natl. Acad. Sci. USA 84:2007-2011 (1987).
30. Uze, G., Lutfalla, G., and Gresser, I. Genetic transfer of a functional human interferon α receptor into mouse cells: cloning and expression of its cDNA. Cell 60:225-234 (1990).
31. Nobile, C. et al. Covert human immunodeficiency virus replication in dendritic cells and in DC-SIGN-expressing cells promotes long term transmission to lymphocytes. J. Virol. 79:5386-5399 (2005).
32. Freed, E. O., D. J. Myers, R. Risser. Mutational analysis of the cleavage sequence of the human immunodeficiency virus type 1 envelope protein precursor gp160. J. Virol. 63:4670-4675 (1989).
33. Overbaugh, J., R. J. Anderson, J. O. Ndinya-Achola, J. K. Kreiss. Distinct but related human immunodeficiency virus type 1 variant populations in genital secretions and blood. AIDS Res. Hum. Retroviruses. 12:107-115 (1996).
34. Hullin-Matsuda, F., and Kobayashi, T. Monitoring the distribution and dynamics of signaling microdomains in living cells with lipid-specific probes. Cell Mol. Life Sci. ahead of print (2007)
35. Baldwin, C.E., Sanders, R.W., and Berkhout, B. Inhibiting HIV-1 entry with fusion inhibitors. Curr. Med. Chem. 10:1633-1642 (2003).
36. Frankel, S. S. et al. Neutralizing monoclonal antibodies block human immunodeficiency virus type 1 infection of dendritic cells and transmission to T cells. J. Virol. 72:9788-9794 (1998).
37. Ganesh, L. et al. Infection of specific dendritic cells by CCR5-tropic human immunodeficiency virus type 1 promotes cell-mediated transmission of virus resistant to broadly neutralizing antibodies. J. virol. 78:11980-11987 (2004).
38. McDonald, D. et al. Recruitment of HIV and its receptors to dendritic cell-T cell junctions. Science 300:1295-1297 (2003).
39. Lackner, A. A., and Veazey, R. S. Current concept in AIDS pathogenesis: insights from the SIV/macaque model. Annu. Rev. Med. 58:461-476 (2007).
40. Mayor, S., Sabharanjak, S., and Maxfield, F. R. Cholesterol-dependent retention of GPI-anchored proteins in endosomes. EMBO J. 275:4626-4638 (1998).
41. Scallon, B. J., et al. Primary structure and functional activity of a phosphatidylinositol-glycan-specific phospholipase D. Science 252:446-448 (1991).
42. Kondoh, G., et al. Angiotensin-converting enzyme is a GPI-anchored protein releasing factor crucial for fertilization. Nat. Med. 11:160-166 (2005).
43. Hollinger, F. B. et al. Standardization of sensitive human immunodeificiency virus coculture procedures and establishment of a multicenter quality assurance program for the AIDS Clinical Trials Group. J. Clin. Microbiol. 30:1787-1794 (1992).
44. Mornor, A. et al. Primary human immunodeficiency virus type 2 (HIV-2) isolates, like HIV-1 isolates, frequently use CCR5 but show promiscuity in coreceptor usage. J. Virol. 73:2343-2349 (1999).
45. Prodromou, C., and Pearl, L. H. Recursive PCR: a novel technique for total gene synthesis. Protein Eng. 5:827-830 (1992).
46. Follenzi, A., Ailes, L. E., Bakovic, S., Ceuna, M., and Naldini, L. Gene transfer by lentiviral vectors is limited by nuclear translocation and rescued by HIV-1 pol sequences. Nat. Genet. 25:217-220 (2000).
47. Zhou, P., Lee, J. H., Moore, P., and Brasky, K. M. High-efficiency gene transfer into rhesus macaque primary T lymphocytes by combining 32°C centrifugation and CH-296-coated plates: effect of gene transfer protocol on T cell homing receptor expression. Hum. Gene Ther. 12:1843-1855 (2001).
48. Yu Kimata, M. T. et al. Capture and transfer of simian immunodeficiency virus by macaque dendritic cells is enhanced by DC-SIGN. J. Virol. 76:11827-11836 (2002).
49. Bomsel, M. Transcytosis of infectious human immunodeficiency virus across a tight human epithelial cell line barrier. Nat. Med. 3:42-47 (1997).
50. Weissenhorn, W., A. Dessen, S. C. Harrison, J. J. Skehel, and D. C. Wiley. 1997. Atomic structure of the ectodomain from HIV-1 gp41. Nature 387:426-430

## Claims

1. An anti-viral peptide comprising an anti-enveloped virus antibody or an active fragment thereof and a lipid attachment signal, wherein said lipid attachment signal and said antibody are arranged in a C' terminal to N' terminal orientation in said peptide.

2. An anti-viral peptide according to claim 1, wherein said antibody is selected from the group consisting of a neutralizing antibody and a non-neutralizing antibody.

3. An anti-viral peptide according to any one of claims 1 or 2, wherein said antibody recognizes an epitope in the prehairpin structure of said enveloped virus, preferably in the second heptad repeat of HIV-1 gp41.

4. An anti-viral peptide according to any preceding claim, wherein said antibody is selected from the group consisting of: an intact Immunoglobulin molecule; a monovalent antibody fragment, preferably a monovalent antibody fragment selected from the group consisting of a fab fragment and a scFv; a multivalent antibody fragment, preferably a multivalent antibody fragment selected from the group consisting of: a Fab₂, a Fab₃, a Bis-scFv, a minibody, a diabody, a triabody, and a tetrabody; an isolated V_{H} domain.

5. An anti-viral peptide according to any one of claims 1 to 4, wherein said antibody and said lipid attachment signal are separated by an immunoglobulin hinge region, said immunoglobulin hinge region being preferably selected from the group: human IgG1, human IgG2, human IgG3, human IgG4.

6. An anti-viral peptide according to claim 5, wherein said hinge region comprises SEQ ID NO: 1.

7. An anti-viral peptide according to any preceding claim, wherein said lipid attachment signal is selected from the group consisting of a peptide lipid attachment signal and a chemical attachment moiety.

8. An anti-viral peptide according to claim 7, wherein said peptide lipid attachment signal is configured to link said peptide to glycosyl-phosphatidylinositol (GPI).

9. An anti-viral peptide according to claim 8, wherein said lipid attachment signal comprises SEQ ID NO: 2.

10. An anti-viral peptide according to claim 7, wherein said chemical attachment moiety consists of a thioester group.

11. An anti-viral peptide according to any preceding claim, wherein said peptide further comprises a detectable tag, said detectable tag comprising preferably a multiple histidine residue sequence.

12. An anti-viral peptide according to any one of claims 1 to 11, wherein said peptide consists of the sequence SEQ ID NO: 14.

13. A nucleic acid molecule encoding an open reading frame for any one of the peptides listed in claims 1 to 12.

14. A vector comprising a nucleic acid molecule according to claim 13, said vector being preferably selected in the group consisting of a retrovirus and a lentiviral vector.

15. A lipodated anti-viral peptide comprising an anti-enveloped virus antibody or an active fragment thereof and a lipid attachment signal and a lipid attached thereto, wherein said lipid attachment signal and said antibody are arranged in a C' terminal to N' terminal orientation in said peptide.

16. A lipodated anti-viral peptide according to claim 15, comprising a lipid in the range C₁₂ to C₂₄, said lipid being saturated or unsaturated, said lipid being preferably glycosyl-phosphatidylinositol.

17. A lipodated anti-viral peptide according to any one of claims 15 and 16, wherein said lipodated anti-viral peptide is purified from one or more cells expressing said lipodated anti-viral peptide.

18. A host cell, **characterized in that** it comprises an anti-viral peptide according to any one of claims 1 to 12 or a lipodated anti-viral peptide according to any one of claims 15 to 17 or it is modified by the polynucleotide according to claim 13 or the vector according to claim 14.

19. A composition for the modulation or treatment of a viral infection comprising a lipodated anti-viral peptide according to any one of claims 15 to 17 in an administrable form with one or more pharmaceutically acceptable carriers or excipients.

20. Use of an effective amount of a composition comprising a lipodated anti-viral polypeptide according to any one of Claims 15 to 17 and one or more pharmaceutically acceptable carriers or excipients, for the preparation of a medicament for modulating or treating a viral infection in a subject in need thereof.

21. A method of purifying a lipodated anti-viral peptide, comprising the steps:
introducing at least one coding sequence for said lipodated anti-viral peptide into at least one cell;
expressing said at least one coding sequence in said at least one cell;
inducing lysis of said at least one cell;
centrifuging the resulting lysate;
passing the resulting supernatant over an affinity purification column;
eluting said column and collecting protein containing fractions.

22. A method of treating at least one cell with a lipodated anti-viral peptide, comprising the steps:
said at least one cell is washed in buffer comprising detergent;
said at least one cell is then suspended in buffer comprising detergent and said lipodated anti-viral peptide and allowed to incubate;
said at least one cell is washed in buffer comprising detergent.
